# EUROPEAN PATENT APPLICATION

(11) **EP 4 621 409 A1**
(43) Date of publication of application: **24.09.2025**
(21) Application number: 23892006.0
(22) Date of filing: 15.11.2023
(51) Int. Cl.: G01N 33/68, G01N 33/50, C07K 7/08, G16B 20/00, A61P 31/14, A61K 38/00, G16B 50/00, G16B 30/00

(54) **NOVEL ANTIVIRAL PEPTIDE**

(30) Priority: 16.11.2022 KR 20220153695; 28.02.2023 KR 20230026405
(71) Applicant: Daehannupharm Co., Ltd., Hwaseong-si, Gyeonggi-do 18622 (KR)
(72) Inventor: KWON, Youngdo, Incheon 21064 (KR); JEON, Hyunjin, Suwon-si, Gyeonggi-do 16507 (KR); LEE, Minhyeong, Seongnam-si, Gyeonggi-do 13449 (KR)
(74) Representative: dompatent von Kreisler Selting Werner - Partnerschaft von Patent- und Rechtsanwälten mbB
(86) International application number: PCT/KR2023/018361
(87) International publication number: WO 2024/106940

(57) **Abstract**

The present disclosure relates to an antiviral peptide and a use thereof. More specifically, the present disclosure relates to a composition and therapeutic method for the treatment of viral diseases using an antiviral peptide.

## Description

### [Technical Field]

The present application relates to an antiviral peptide. The antiviral agents provided by some embodiments of the present application have therapeutic uses against viral diseases.

### [Background Art]

Common mechanisms of action of antiviral peptides are known to include suppressing viral membrane protein function, suppressing viral RNA amplification in the nucleus of infected cells, suppressing infection through viral envelope damage, and the like. In the case of enveloped viruses, the envelope has a lipid bilayer structure, so inactivation of the envelope means loss of viral infectivity.

### [Disclosure]

### [Technical Problem]

The present application provides an antiviral peptide that is not derived from known antiviral peptides.

### [Technical Solution]

The present application provides novel antiviral peptide. In this case, the amino acid sequence of the antiviral peptide is an amino acid sequence derived from the Swiss-Prot sequence database.

### [Advantageous Effects]

An object of the present application is to provide an antiviral peptide.

Another object of the present application is to provide a use of an antiviral peptide for preventing or treating viral diseases.

Another object of the present application is to provide a pharmaceutical composition including an antiviral peptide for the prevention or treatment of viral diseases.

Another object of the present application is to provide a method for treating viral diseases using an antiviral peptide.

Another object of the present application is to provide a method for screening a group of antiviral peptide candidates.

Another object of the present application is to provide a method for selecting an antiviral peptide.

### [Description of Drawings]

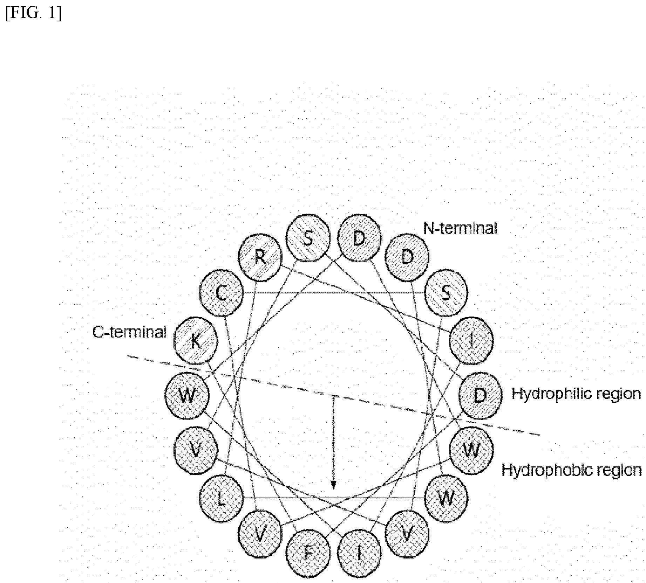

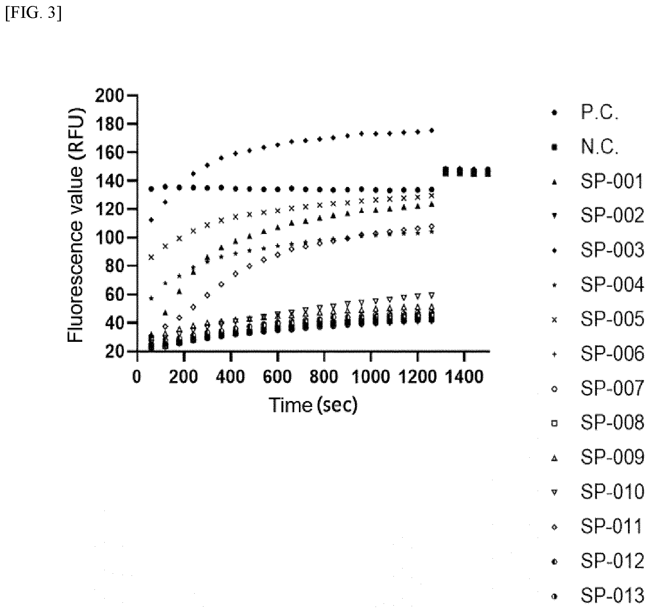

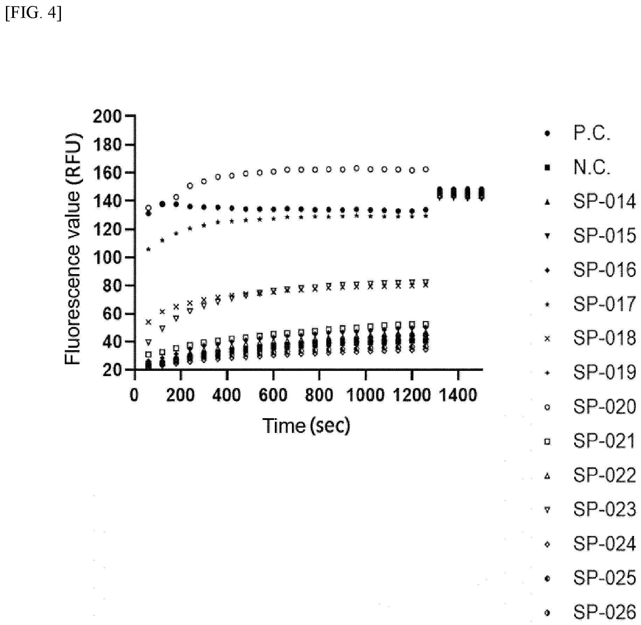

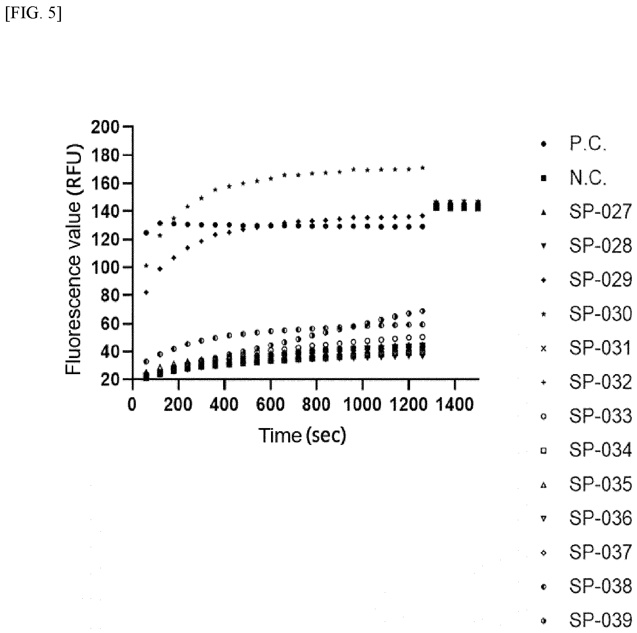

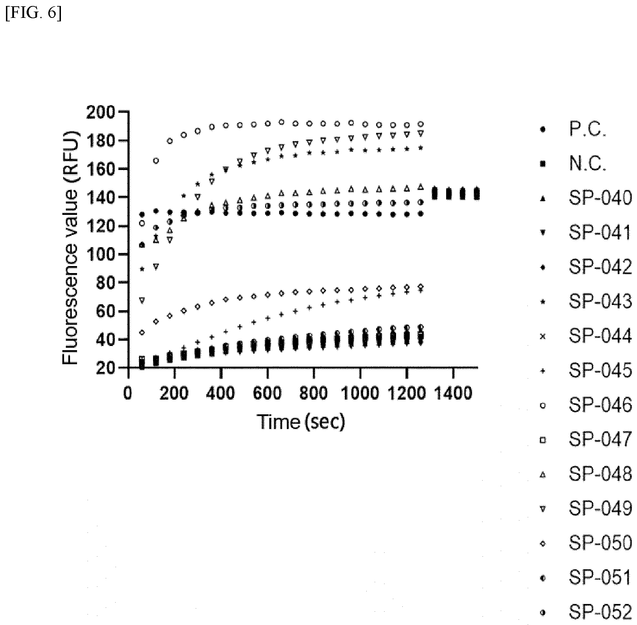

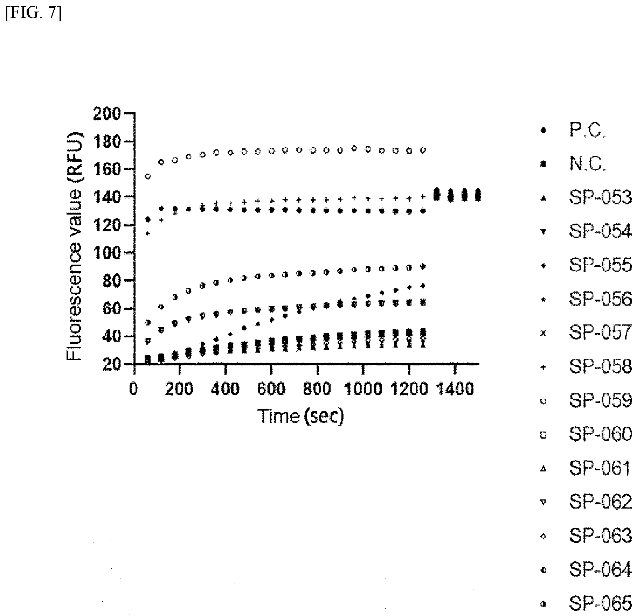

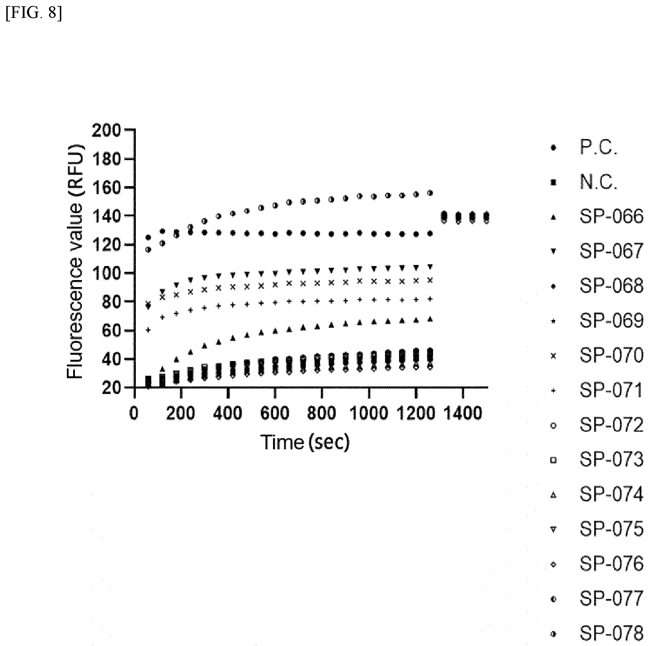

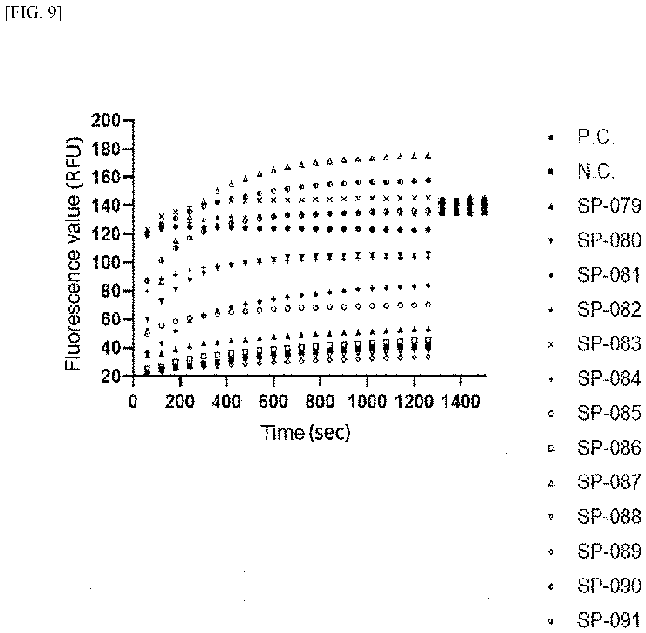

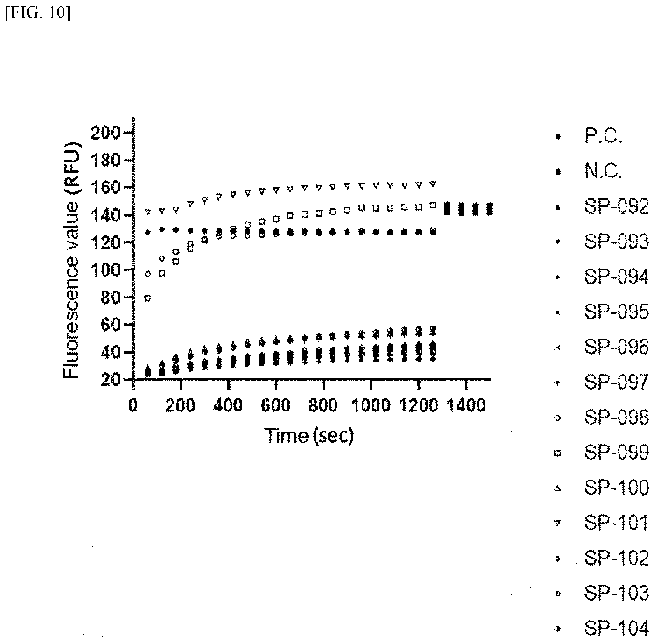

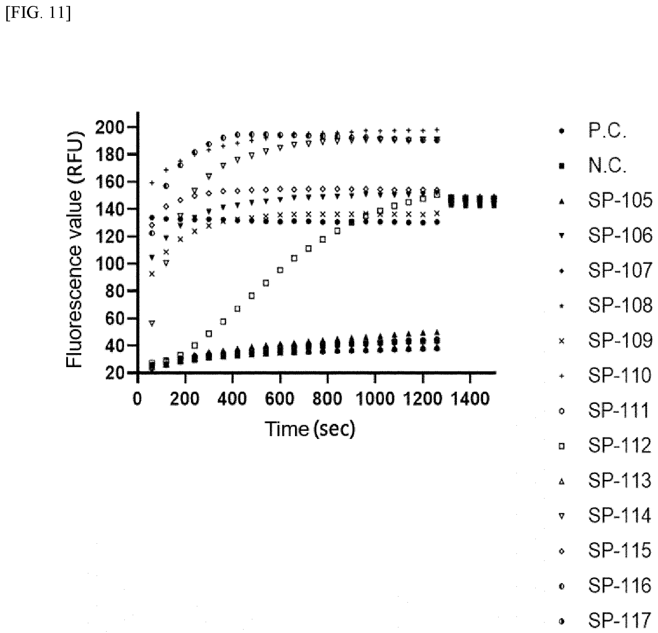

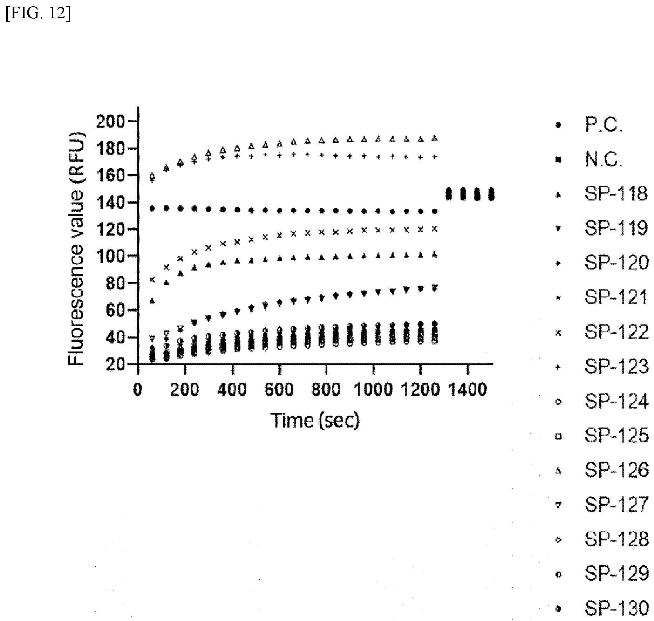

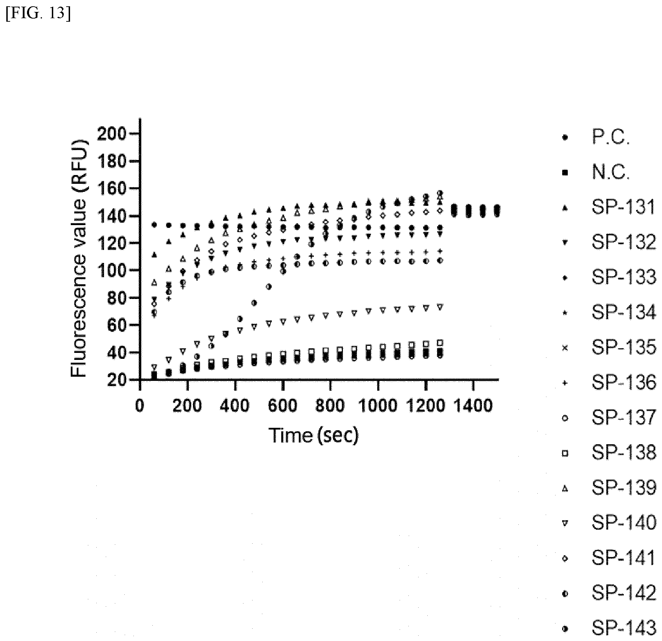

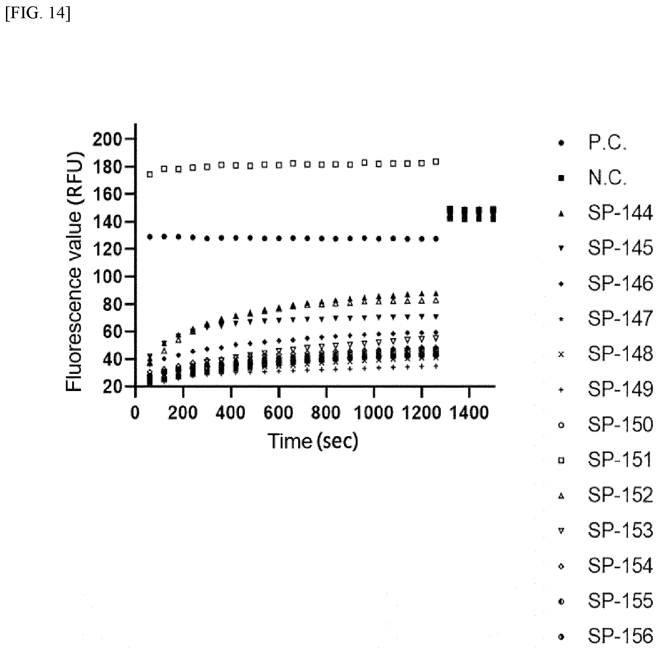

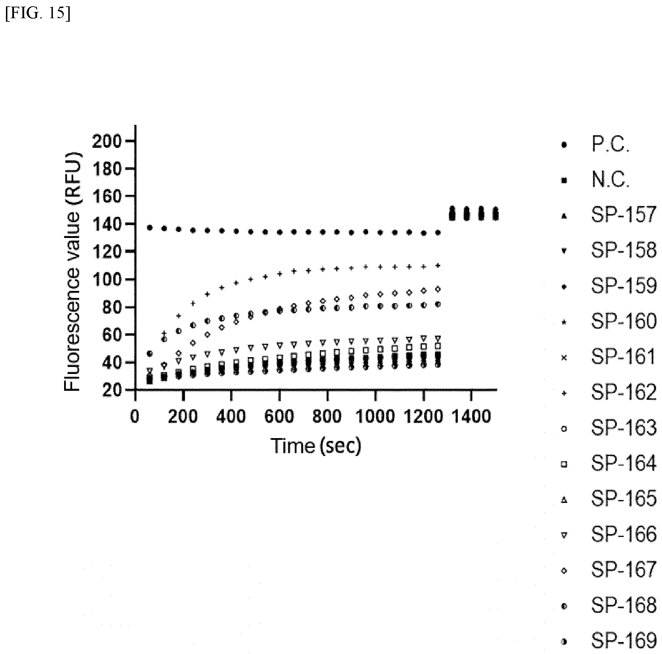

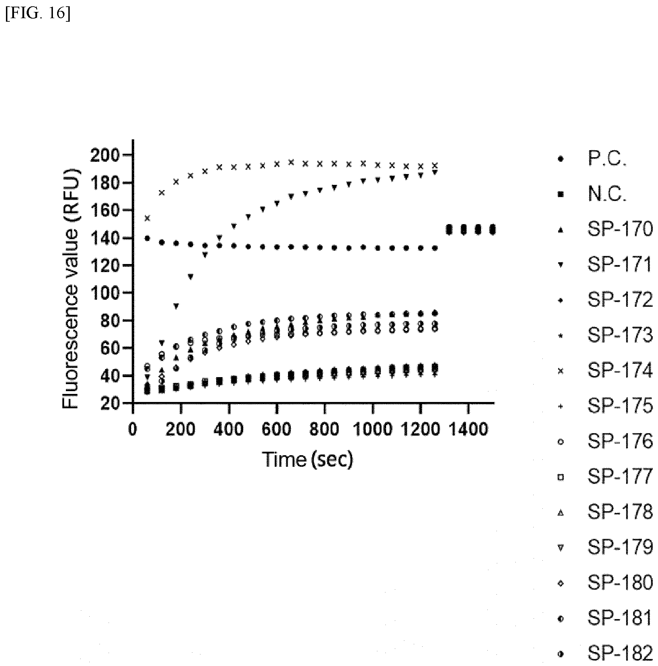

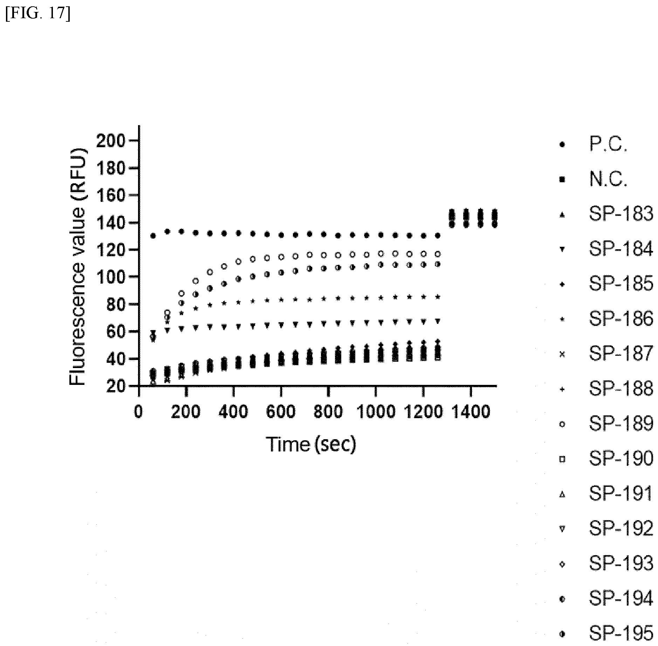

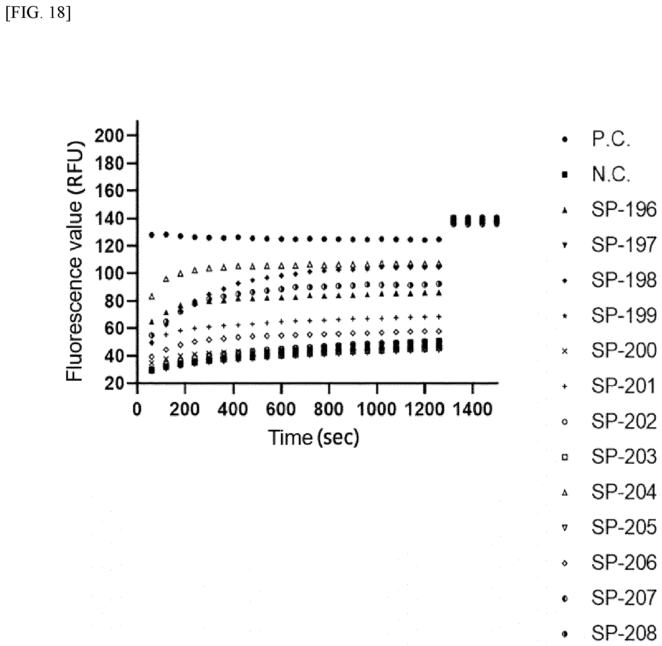

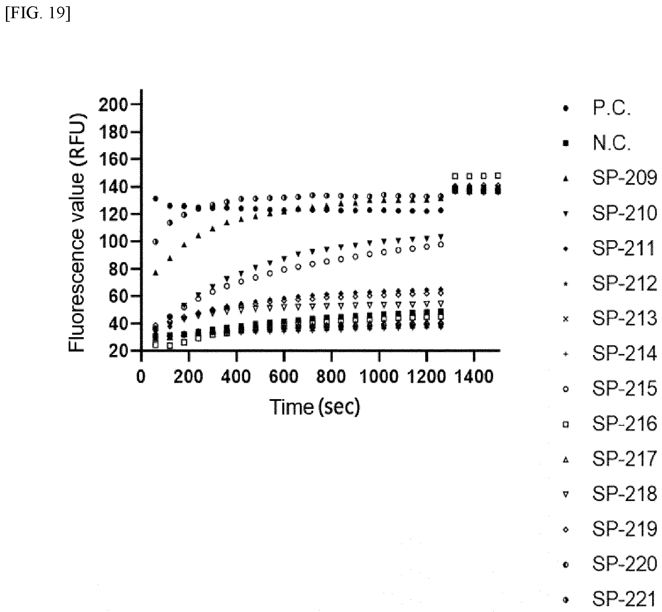

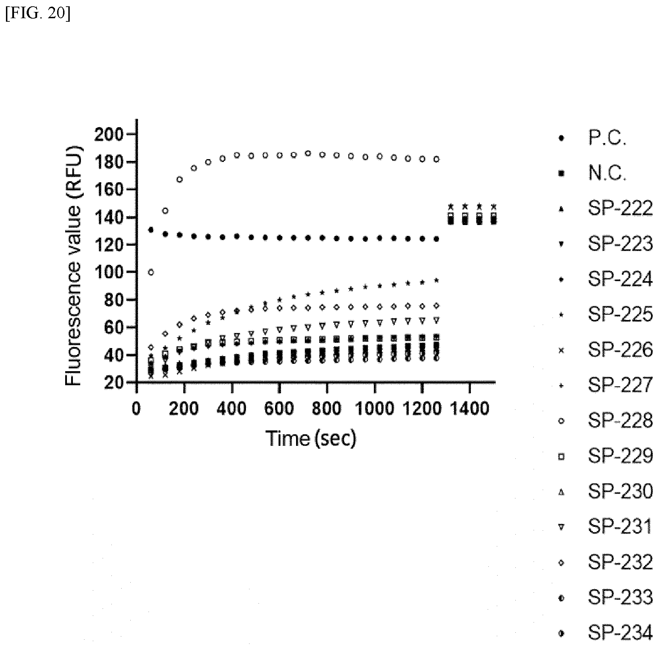

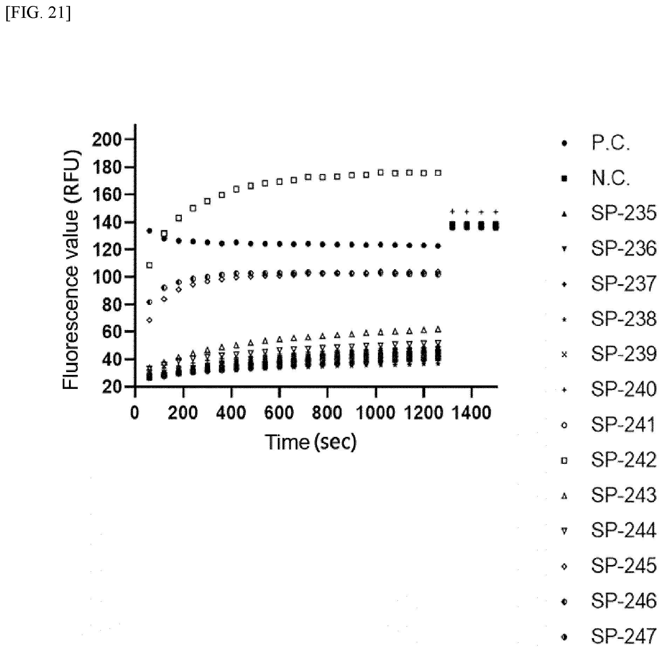

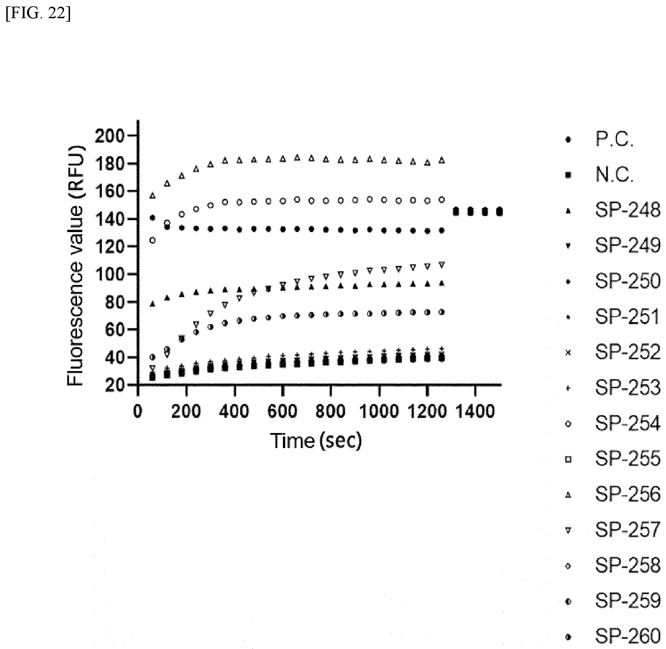

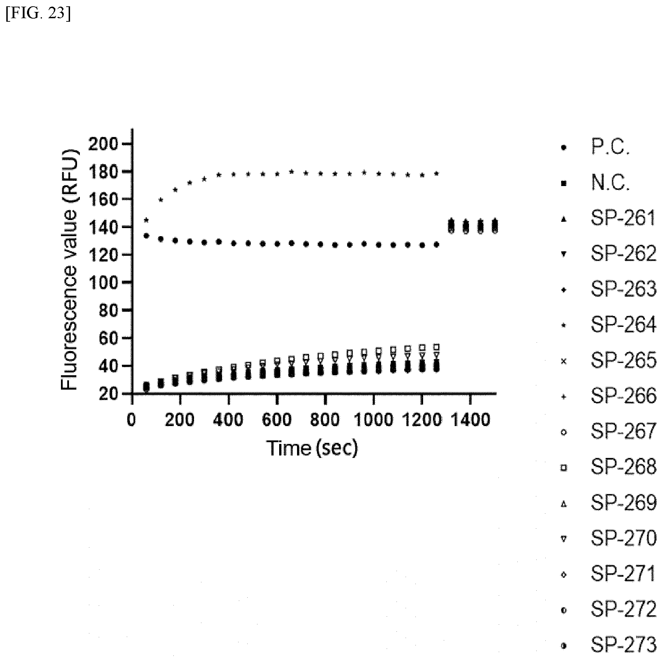

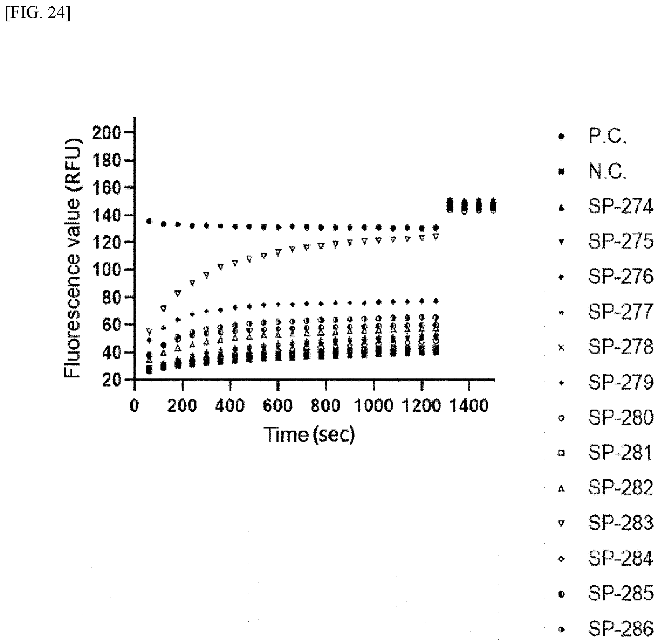

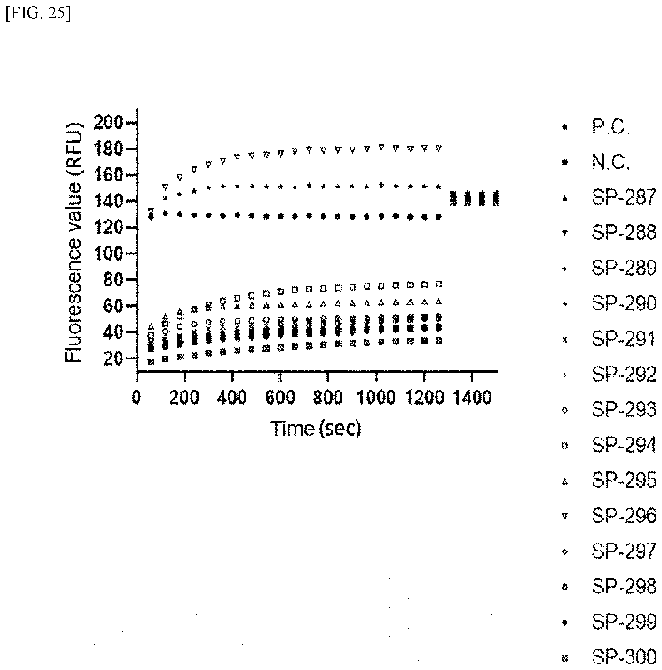

FIG. 1 illustrates the predicted positions of amino acid residues when a peptide having the amino acid sequence DWLRIIWDWVCSVVSDFK (SEQ ID NO: 301) has an alpha helical structure, and in SEQ ID NO: 301, aspartic acid (Asp, D), which is a residue at the first position, is designated as the N-terminus, and lysine (Lys K), which is a residue at the last position, is designated as the C-terminus. In this case, starting from the N-terminus, D, lines are connected in the sequence of the next amino acid residue after D, W, and then the amino acid residue, L, and the position to which the line is finally connected is the C-terminus, K. FIG. 1 shows that in the single structure of the peptide, a hydrophilic region and a hydrophobic region are separated, and each region includes nine amino acid residues. In this case , the types of amino acid residues included in the hydrophilic region are tryptophan (Trp, W), valine (Val, V), leucine (Leu, L), phenylalanine (Phe, F) and isoleucine (Ile, I), all of which are hydrophilic amino acid residues. The types of amino acid residues included in the hydrophobic region consist of the hydrophobic amino acid residues lysine (Lys K), arginine (Arg, R), serine (Ser, S), and aspartic acid (Asp, D) and the hydrophilic amino acid residues cysteine (Cys, C) and isoleucine (Ile, I), and seven of the nine amino acid residues included in the hydrophobic region are hydrophobic amino acid residues. Among the amino acid residues illustrated in FIG. 1, those with a checkered pattern are hydrophilic amino acid residues, and those with other patterns are hydrophobic amino acid residues, so the characteristics of the amino acid residues distributed in each region may be more easily identified.
FIG. 2 illustrates an example of a structure in which gaps may occur in the viral phospholipid bilayer due to the small size of the virus.
FIG. 3 shows the results of confirming the liposome leakage efficacy of SP-001 to SP-013 peptides.
FIG. 4 shows the results of confirming the liposome leakage efficacy of SP-014 to SP-026 peptides.
FIG. 5 shows the results of confirming the liposome leakage efficacy of SP-027 to SP-039 peptides.
FIG. 6 shows the results of confirming the liposome leakage efficacy of SP-040 to SP-052 peptides.
FIG. 7 shows the results of confirming the liposome leakage efficacy of SP-053 to SP-065 peptides.
FIG. 8 shows the results of confirming the liposome leakage efficacy of SP-066 to SP-078 peptides.
FIG. 9 shows the results of confirming the liposome leakage efficacy of SP-079 to SP-091 peptides.
FIG. 10 shows the results of confirming the liposome leakage efficacy of SP-092 to SP-104 peptides.
FIG. 11 shows the results of confirming the liposome leakage efficacy of SP-105 to SP-117 peptides.
FIG. 12 shows the results of confirming the liposome leakage efficacy of SP-118 to SP-130 peptides.
FIG. 13 shows the results of confirming the liposome leakage efficacy of SP-131 to SP-143 peptides.
FIG. 14 shows the results of confirming the liposome leakage efficacy of SP-144 to SP-156 peptides.
FIG. 15 shows the results of confirming the liposome leakage efficacy of SP-157 to SP-169 peptides.
FIG. 16 shows the results of confirming the liposome leakage efficacy of SP-170 to SP-182 peptides.
FIG. 17 shows the results of confirming the liposome leakage efficacy of SP-183 to SP-195 peptides.
FIG. 18 shows the results of confirming the liposome leakage efficacy of SP-196 to SP-208 peptides.
FIG. 19 shows the results of confirming the liposome leakage efficacy of SP-209 to SP-221 peptides.
FIG. 20 shows the results of confirming the liposome leakage efficacy of SP-222 to SP-234 peptides.
FIG. 21 shows the results of confirming the liposome leakage efficacy of SP-235 to SP-247 peptides.
FIG. 22 shows the results of confirming the liposome leakage efficacy of SP-248 to SP-260 peptides.
FIG. 23 shows the results of confirming the liposome leakage efficacy of SP-261 to SP-273 peptides.
FIG. 24 shows the results of confirming the liposome leakage efficacy of SP-274 to SP-286 peptides.
FIG. 25 shows the results of confirming the liposome leakage efficacy of SP-287 to SP-300 peptides.
   The graphs shown in FIGS. 3 to 25 show the results of measuring the fluorescence level over time in each well, with the horizontal axis representing time and the vertical axis representing the fluorescence level. In this case, the data of each graph may be analyzed to indicate that the peptide treated in the wells showing high fluorescence levels has excellent efficacy in damaging (or destroying) the viral lipid membrane.

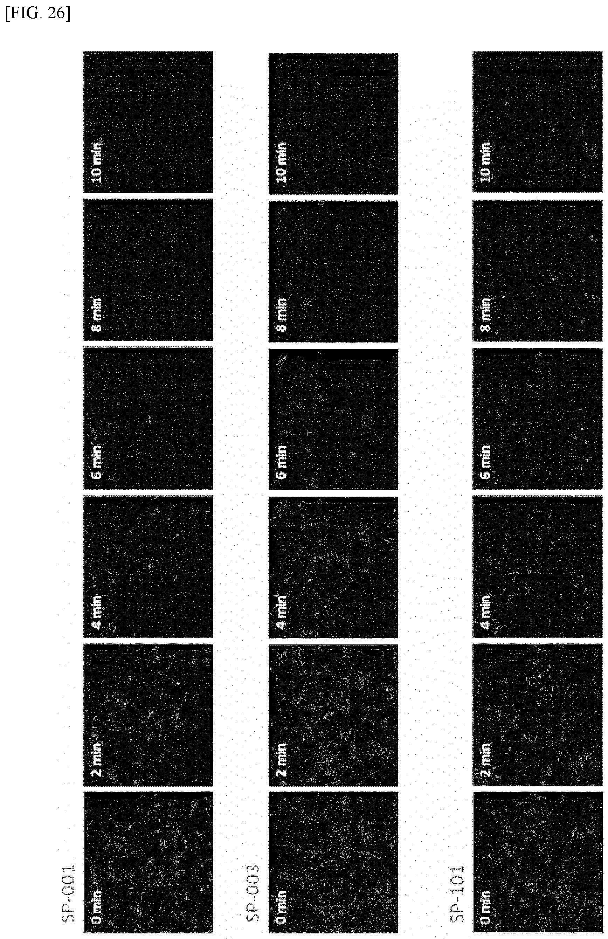

FIG. 26 shows the results of confirming the liposome rupture efficacy of SP-001, SP-003 and SP-101 peptides. Specifically, FIG. 26 shows the changes in fluorescence signals observed immediately (0 min), 2 minutes, 4 minutes, 6 minutes, 8 minutes, and 10 minutes after a liposome-attached coverslip is treated with each of the SP-001, SP-003, and SP-101 peptides at a constant rate.

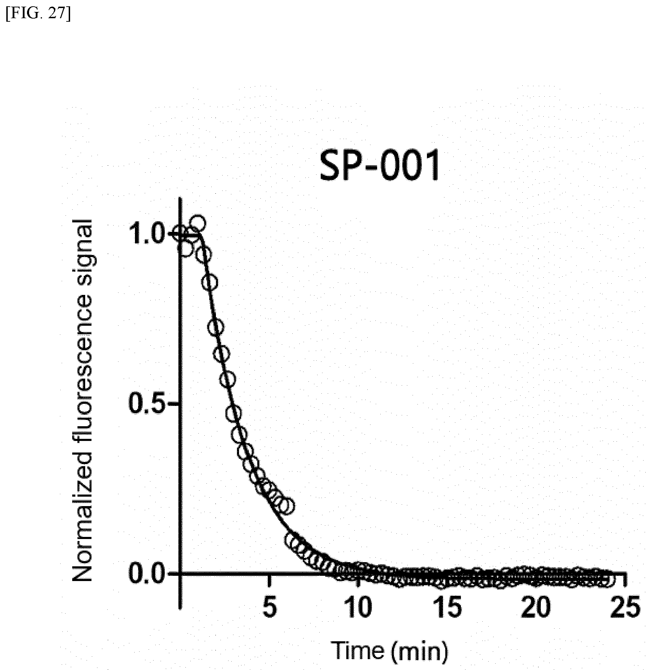

FIG. 27 shows a graph of the fluorescence signal measured at 20 second intervals while the liposome-attached coverslip was treated with the SP-001 peptide at a constant rate. In this case, the vertical axis of the graph indicates the normalized fluorescence signal by converting the fluorescence value immediately after peptide treatment (0 seconds) into 1.

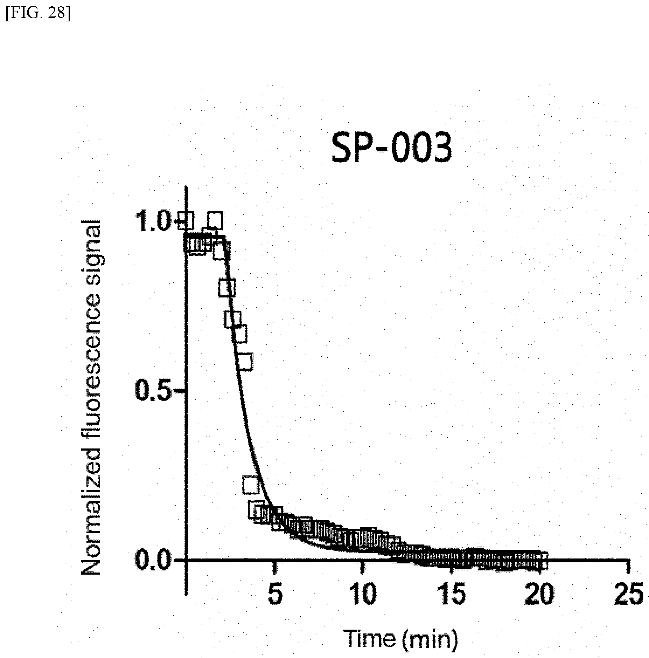

FIG. 28 shows a graph of the fluorescence signal measured at 20 second intervals while the liposome-attached coverslip was treated with the SP-003 peptide at a constant rate. In this case, the vertical axis of the graph indicates the normalized fluorescence signal by converting the fluorescence value immediately after peptide treatment (0 seconds) into 1.

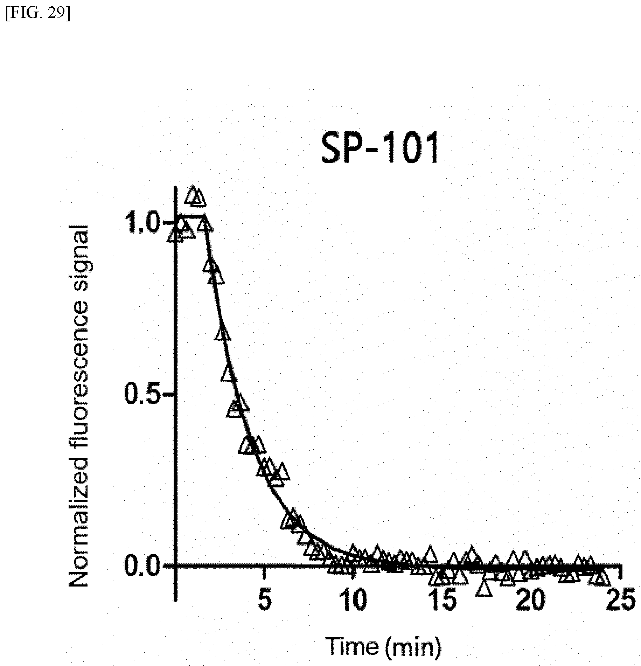

FIG. 29 shows a graph of the fluorescence signal measured at 20 second intervals while the liposome-attached coverslip was treated with the SP-101 peptide at a constant rate. In this case, the vertical axis of the graph indicates the normalized fluorescence signal by converting the fluorescence value immediately after peptide treatment (0 seconds) into 1.

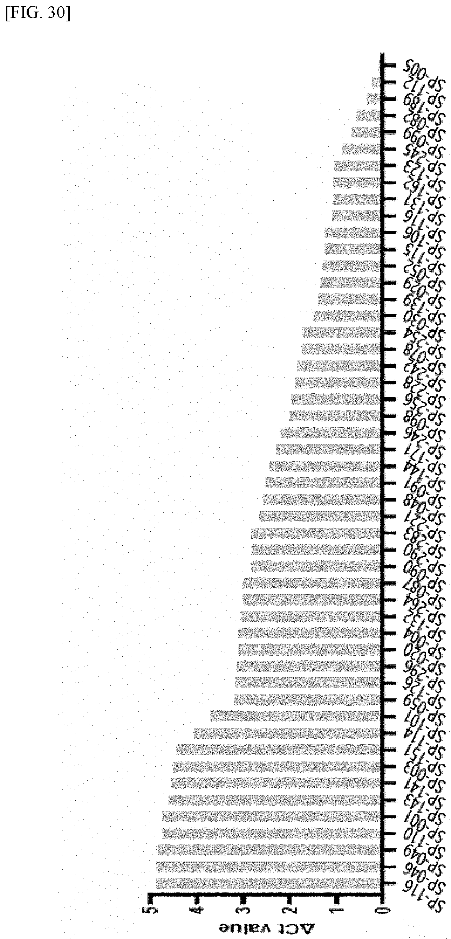

FIG. 30 shows the results of measuring the amount of dengue virus RNA remaining without being degraded by RNase after treating dengue virus with each of 50 types of peptides. In this case, the vertical axis of the graph indicates the increase (ΔCt) in Ct value upon peptide treatment compared to the Ct value of the negative control group.

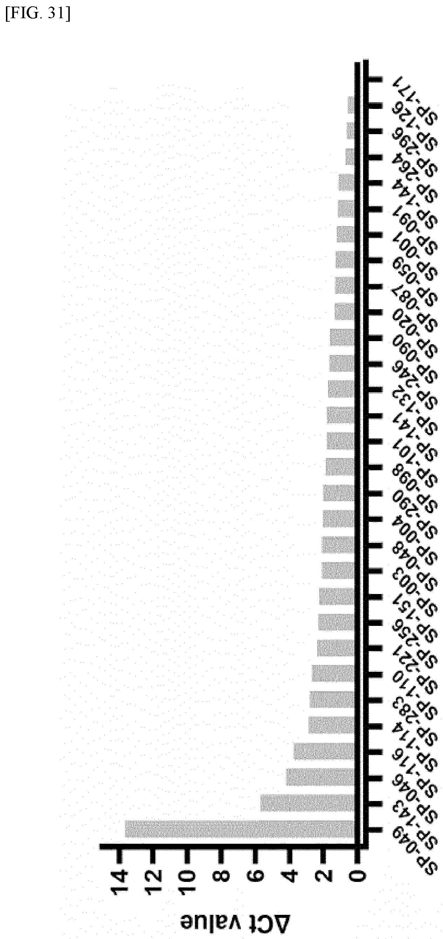

FIG. 31 shows the Ct value measured for viral RNA after dengue virus incubated with specific concentrations of peptides (30 types) was incubated with cells.

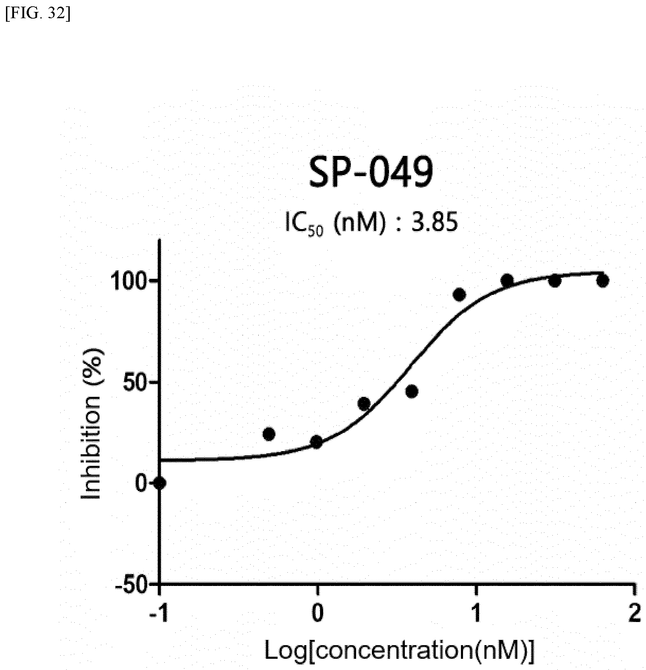

FIG. 32 shows IC₅₀ values calculated based on Ct values measured for viral RNA after dengue virus incubated with various concentrations of the SP-049 peptide was incubated with cells.

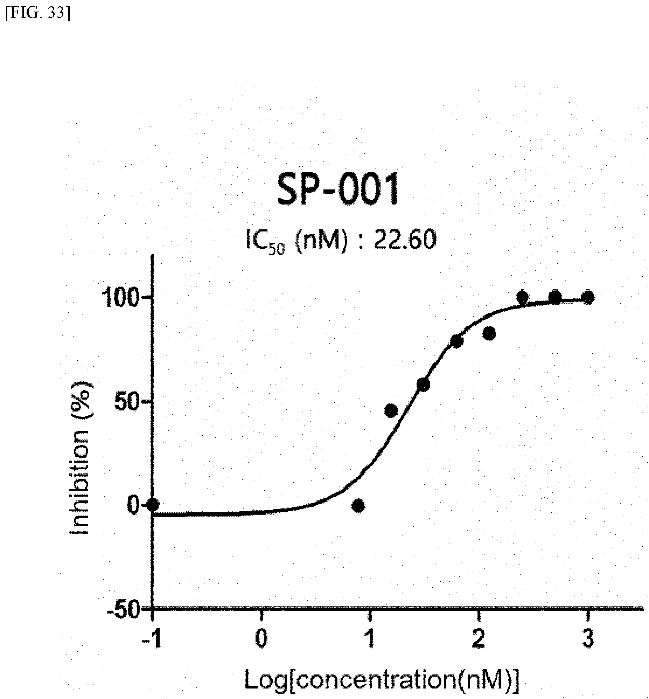

FIG. 33 shows IC₅₀ values calculated based on Ct values measured for viral RNA after dengue virus incubated with various concentrations of the SP-001 peptide was incubated with cells.

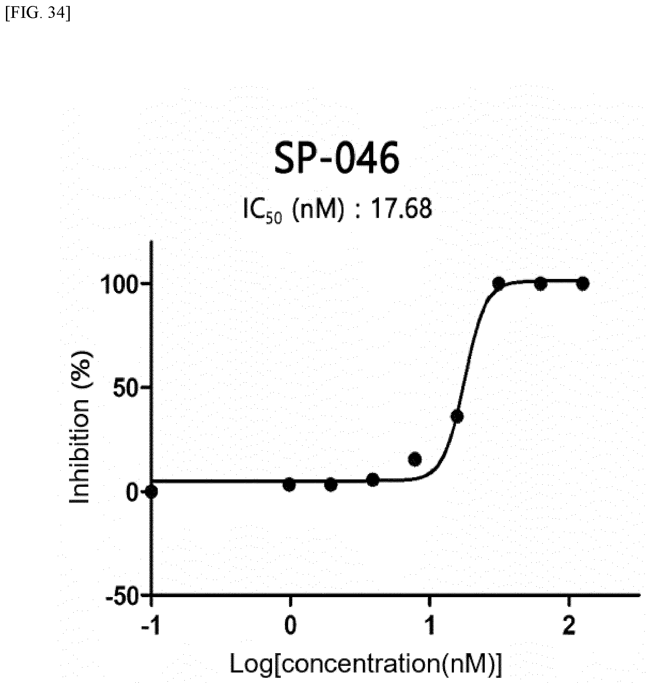

FIG. 34 shows IC₅₀ values calculated based on Ct values measured for viral RNA after dengue virus incubated with various concentrations of the SP-046 peptide was incubated with cells.

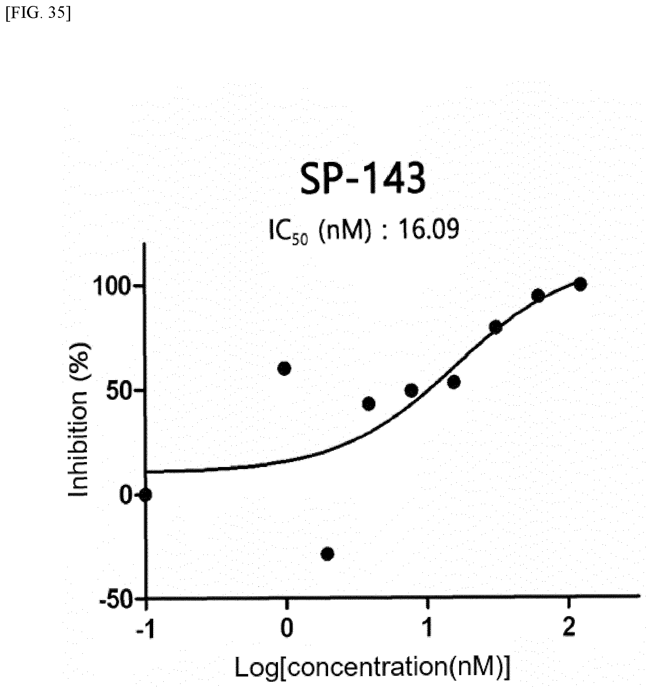

FIG. 35 shows IC₅₀ values calculated based on Ct values measured for viral RNA after dengue virus incubated with various concentrations of the SP-143 peptide was incubated with cells.

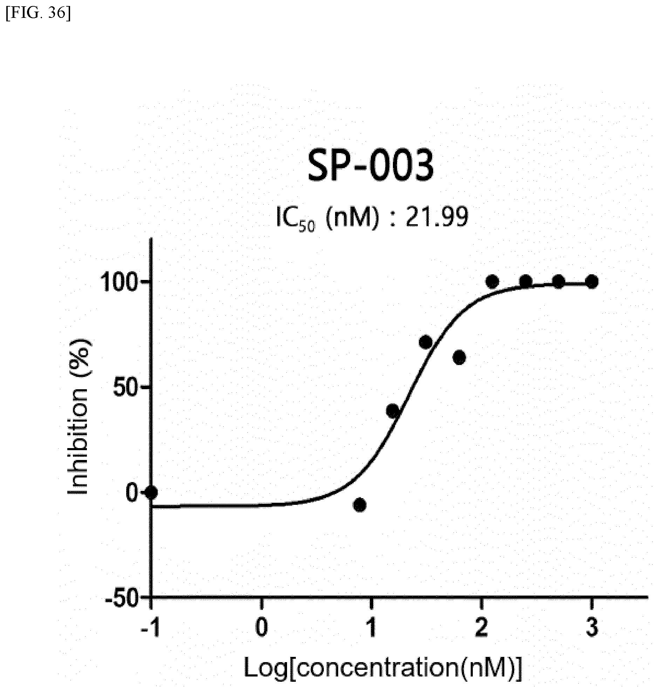

FIG. 36 shows IC₅₀ values calculated based on Ct values measured for viral RNA after dengue virus incubated with various concentrations of the SP-003 peptide was incubated with cells.

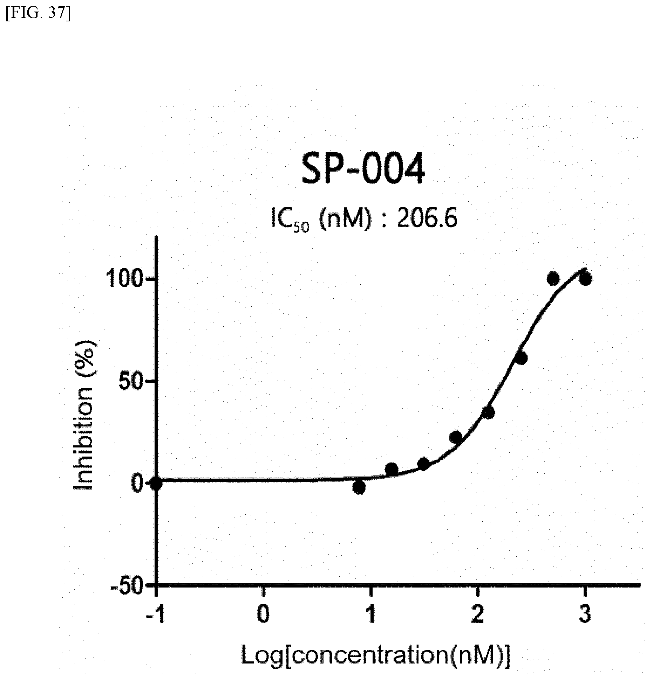

FIG. 37 shows IC₅₀ values calculated based on Ct values measured for viral RNA after dengue virus incubated with various concentrations of the SP-004 peptide was incubated with cells.

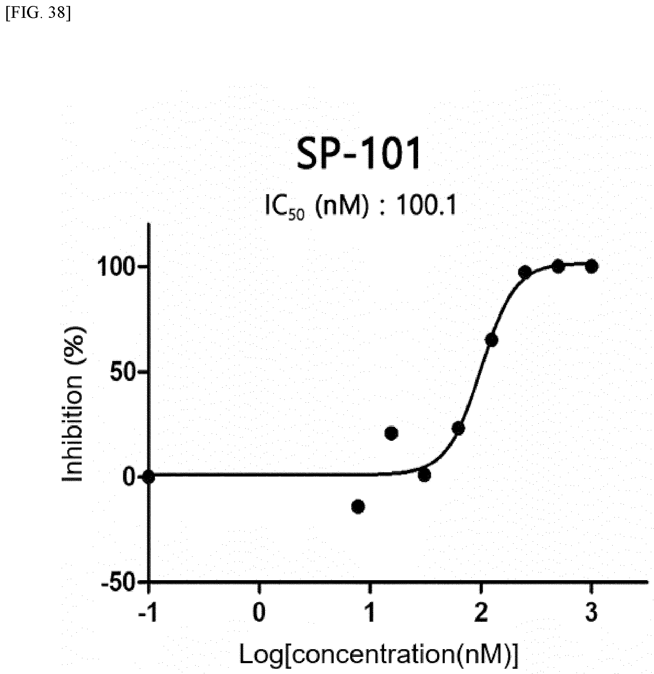

FIG. 38 shows IC₅₀ values calculated based on Ct values measured for viral RNA after dengue virus incubated with various concentrations of the SP-101 peptide was incubated with cells.

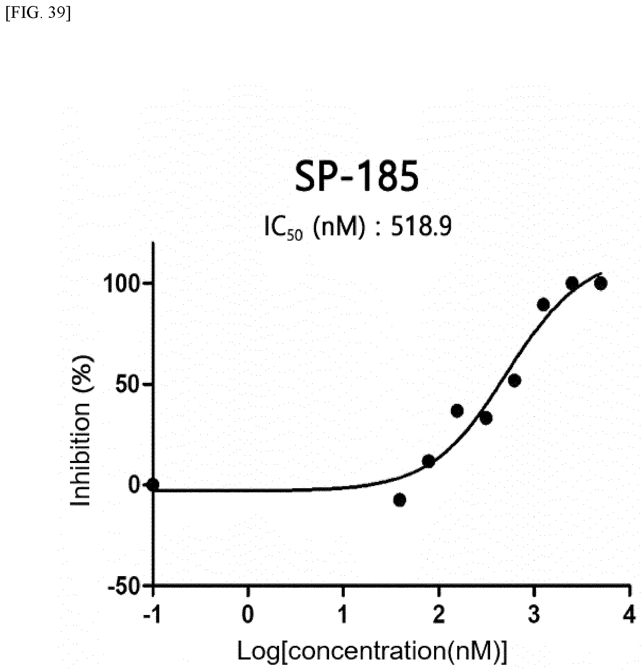

FIG. 39 shows IC₅₀ values calculated based on Ct values measured for viral RNA after dengue virus incubated with various concentrations of the SP-185 peptide was incubated with cells.

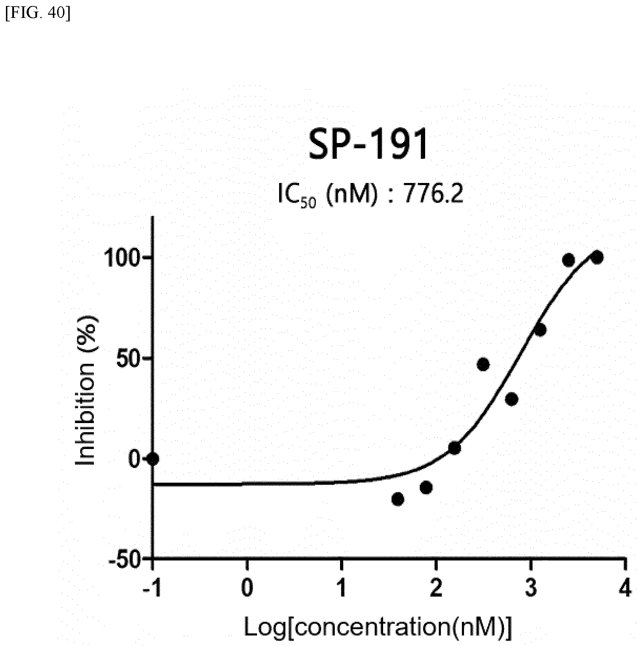

FIG. 40 shows the inhibition rates and IC₅₀ values calculated based on Ct values measured for viral RNA after dengue virus incubated with various concentrations of the SP-191 peptide was incubated with cells.

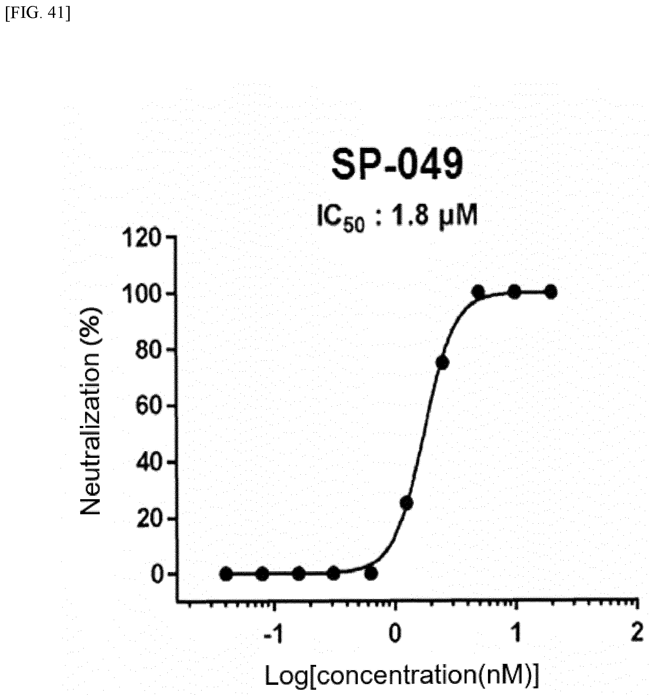

FIG. 41 shows the neutralization abilities and IC₅₀ values calculated based on the percentage of infected cells measured after SARS-CoV2 virus incubated with various concentrations of the SP-049 peptide was incubated with cells.

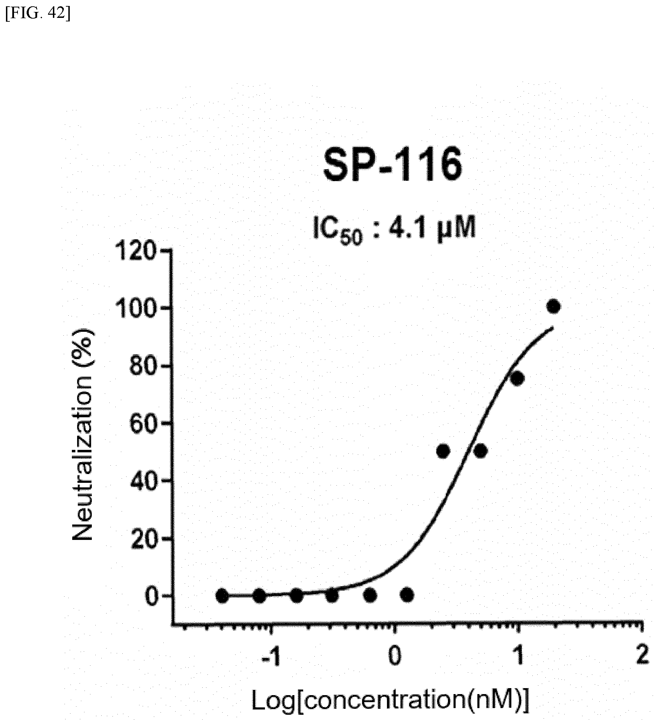

FIG. 42 shows the neutralization abilities and IC₅₀ values calculated based on the percentage of infected cells measured after SARS-CoV2 virus incubated with various concentrations of the SP-116 peptide was incubated with cells.

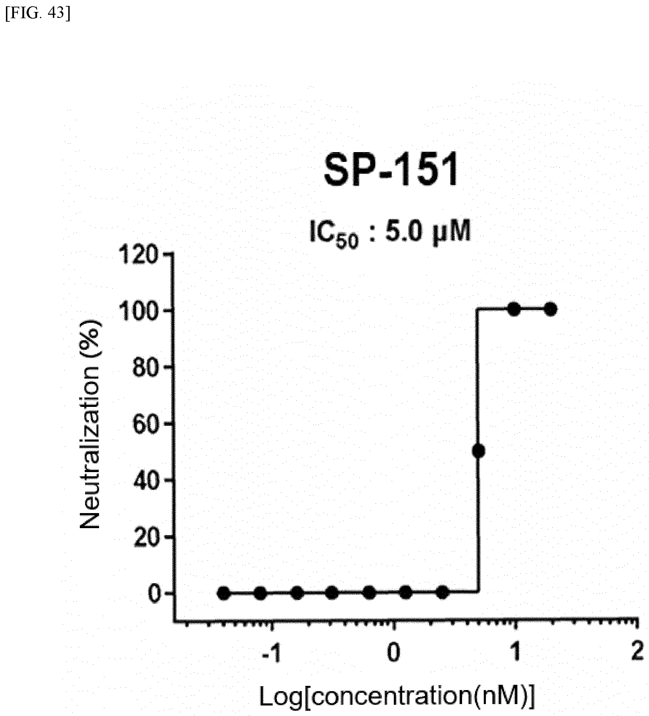

FIG. 43 shows the neutralization abilities and IC₅₀ values calculated based on the percentage of infected cells measured after SARS-CoV2 virus incubated with various concentrations of the SP-151 peptide was incubated with cells.

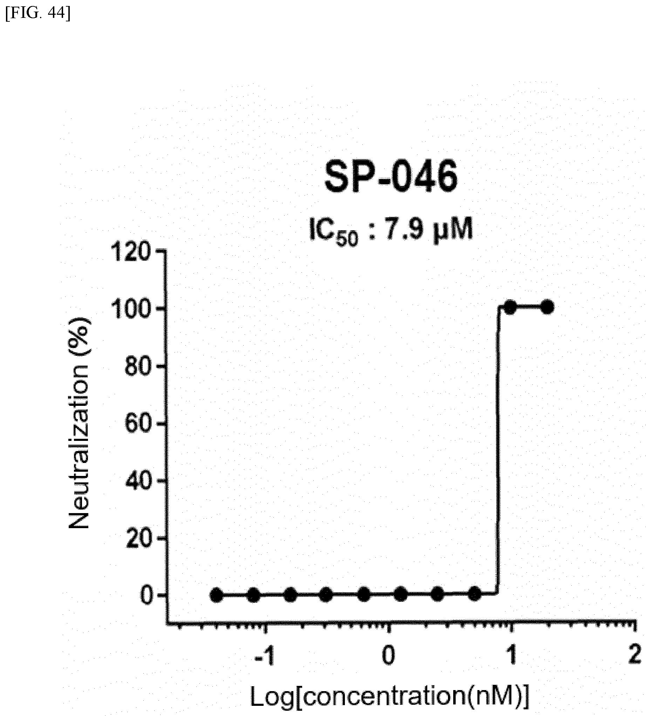

FIG. 44 shows the neutralization abilities and IC₅₀ values calculated based on the percentage of infected cells measured after SARS-CoV2 virus incubated with various concentrations of the SP-046 peptide was incubated with cells.

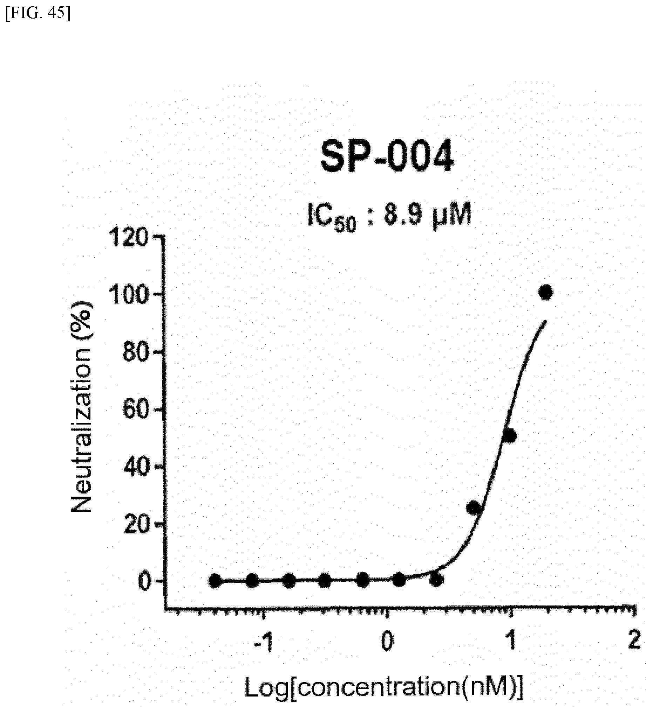

FIG. 45 shows the neutralization abilities and IC₅₀ values calculated based on the percentage of infected cells measured after SARS-CoV2 virus incubated with various concentrations of the SP-004 peptide was incubated with cells.

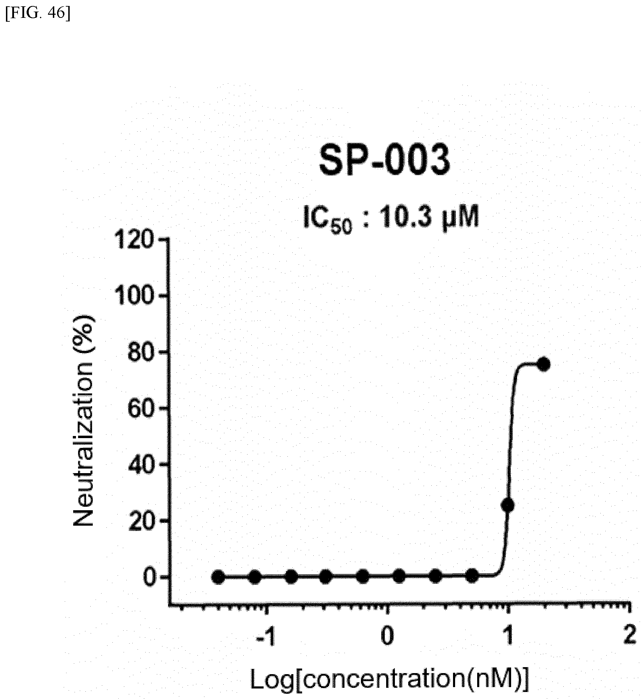

FIG. 46 shows the neutralization abilities and IC₅₀ values calculated based on the percentage of infected cells measured after SARS-CoV2 virus incubated with various concentrations of the SP-003 peptide was incubated with cells.

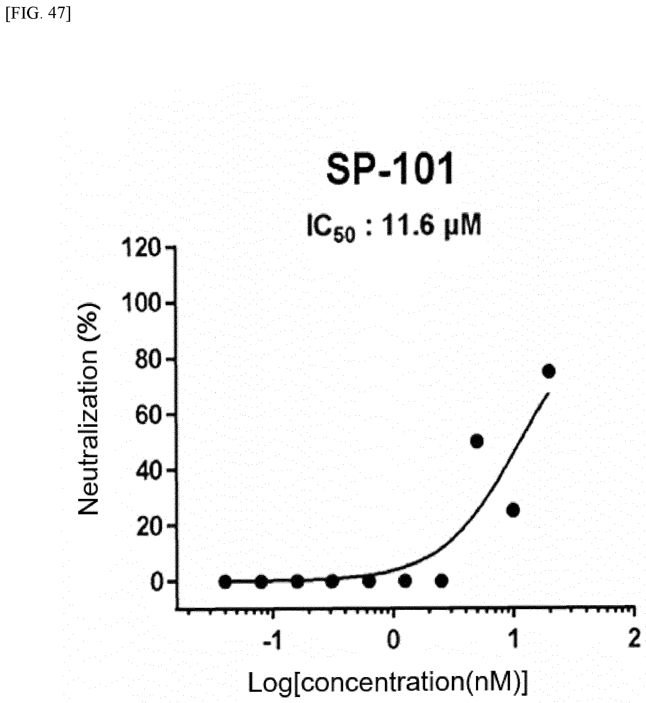

FIG. 47 shows the neutralization abilities and IC₅₀ values calculated based on the percentage of infected cells measured after SARS-CoV2 virus incubated with various concentrations of the SP-101 peptide was incubated with cells.

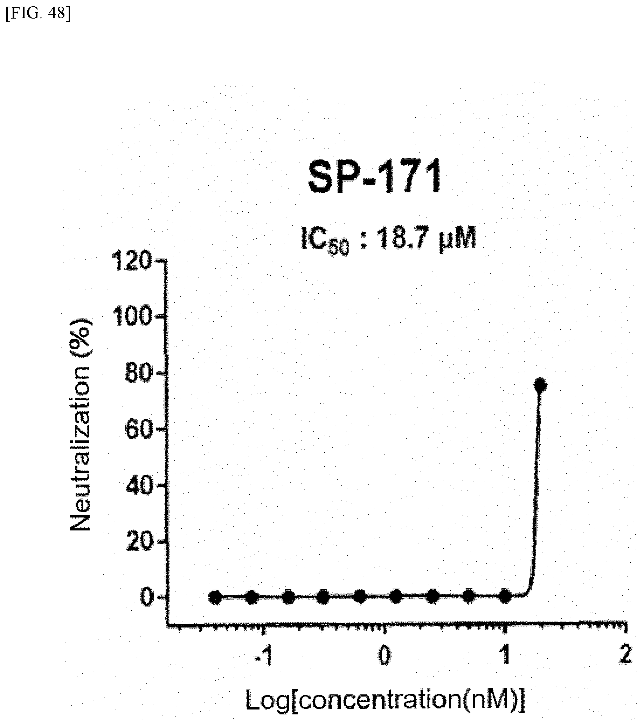

FIG. 48 shows the neutralization abilities and IC₅₀ values calculated based on the percentage of infected cells measured after SARS-CoV2 virus incubated with various concentrations of the SP-171 peptide was incubated with cells.

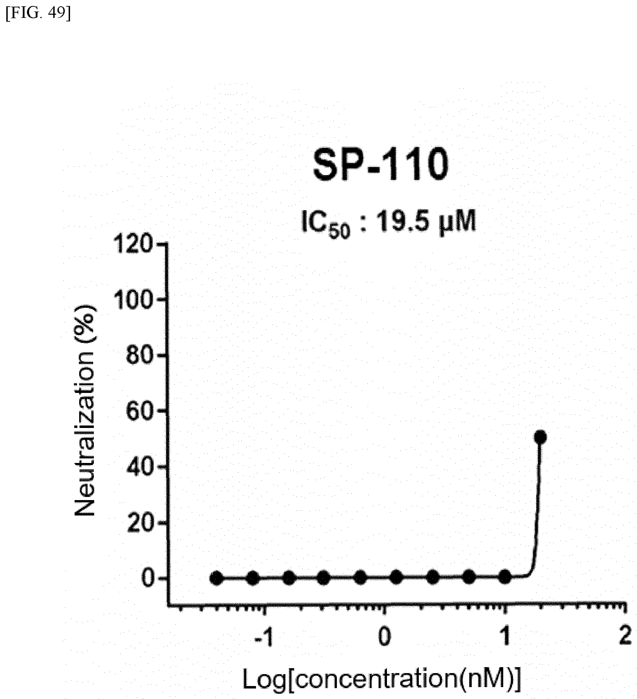

FIG. 49 shows the neutralization abilities and IC₅₀ values calculated based on the percentage of infected cells measured after SARS-CoV2 virus incubated with various concentrations of the SP-110 peptide was incubated with cells.

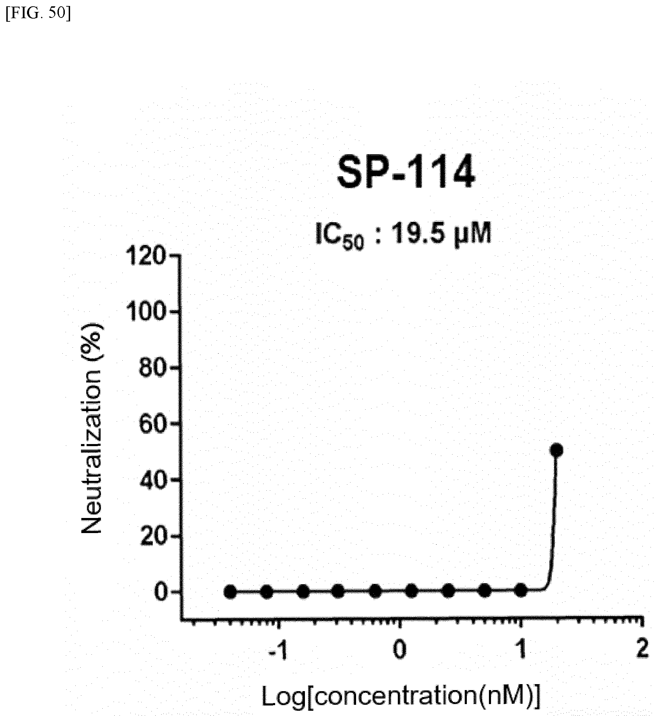

FIG. 50 shows the neutralization abilities and IC₅₀ values calculated based on the percentage of infected cells measured after SARS-CoV2 virus incubated with various concentrations of the SP-114 peptide was incubated with cells.

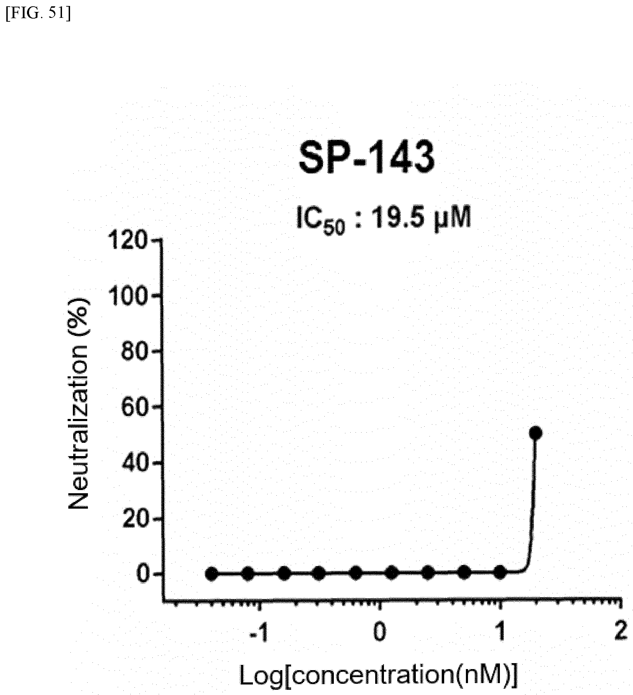

FIG. 51 shows the neutralization abilities and IC₅₀ values calculated based on the percentage of infected cells measured after SARS-CoV2 virus incubated with various concentrations of the SP-143 peptide was incubated with cells.

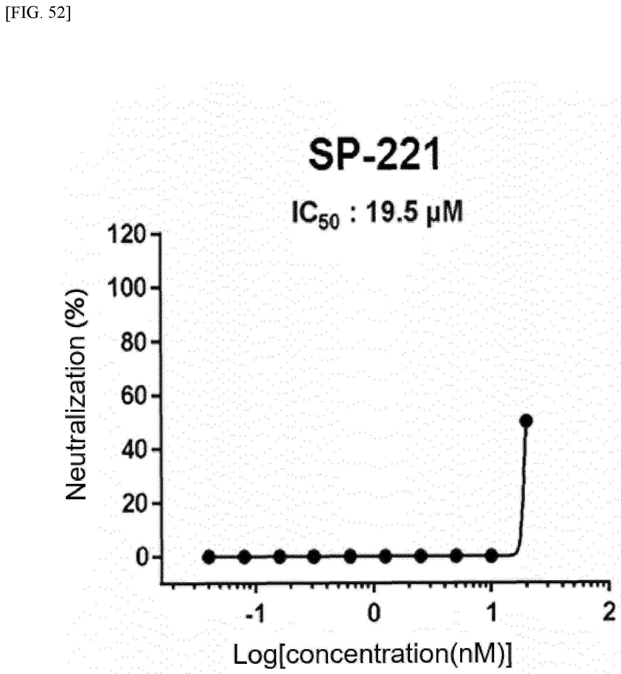

FIG. 52 shows the neutralization abilities and IC₅₀ values calculated based on the percentage of infected cells measured after SARS-CoV2 virus incubated with various concentrations of the SP-221 peptide was incubated with cells.

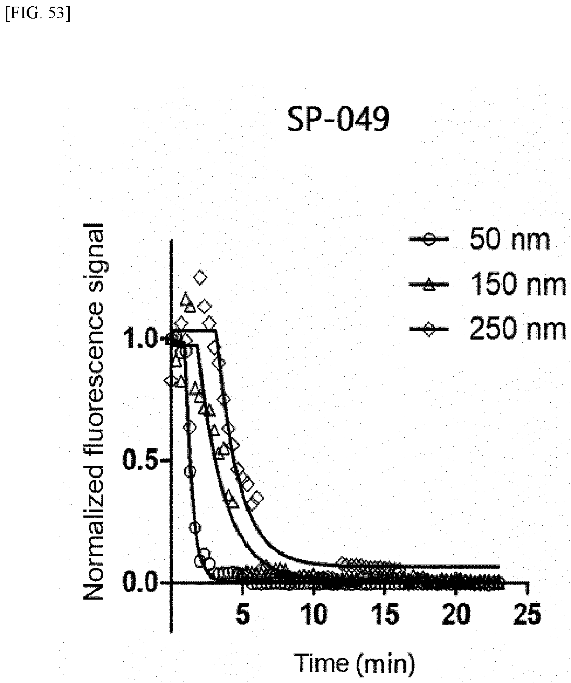

FIG. 53 shows a graph of the fluorescence signal measured at 20 second intervals while the liposome-attached coverslip was treated with the SP-049 peptide at a constant rate. In this case, the vertical axis of the graph indicates the normalized fluorescence signal by converting the fluorescence value immediately after peptide treatment (0 seconds) into 1. FIG. 53 shows the results for liposomes with various sizes (50 nm, 150 nm, and 250 nm) and shows that the normalized fluorescence intensity of smaller liposomes decreases more rapidly than larger liposomes.

### [Best Modes of the Invention]

Some embodiments of the present application provide an antiviral peptide.

Some embodiments of the present application provide an antiviral peptide whose amino acid sequence is one selected from the following sequences:
SEQ ID NO: 1, SEQ ID NOs: 3 to 5, SEQ ID NOs: 8 to 23, SEQ ID NO: 26, SEQ ID NO: 29, SEQ ID NO: 30, SEQ ID NO: 33, SEQ ID NO: 35, SEQ ID NO: 36, SEQ ID NOs: 38 to 40, SEQ ID NO: 43, SEQ ID NOs: 45 to 52, SEQ ID NO: 55, SEQ ID NO: 58, SEQ ID NO: 59, SEQ ID NO: 62, SEQ ID NOs: 64 to 73, SEQ ID NO: 75, SEQ ID NOs: 78 to 87, SEQ ID NOs: 90 to 93, SEQ ID NOs: 97 to 102, SEQ ID NOs: 104 to 106, SEQ ID NOs: 109 to 112, SEQ ID NOs: 114 to 116, SEQ ID NOs: 118 to 123, SEQ ID NO: 126, SEQ ID NO: 127, SEQ ID NOs: 130 to 132, SEQ ID NO: 136, SEQ ID NOs: 138 to 147, SEQ ID NOs: 151 to 155, SEQ ID NO: 162, SEQ ID NO: 164, SEQ ID NOs: 166 to 168, SEQ ID NOs: 170 to 174, SEQ ID NO: 176, SEQ ID NOs: 180 to 182, SEQ ID NOs: 184 to 186, SEQ ID NO: 188, SEQ ID NO: 189, SEQ ID NO: 191, SEQ ID NO: 192, SEQ ID NOs: 194 to 196, SEQ ID NO: 198, SEQ ID NO: 201, SEQ ID NO: 204, SEQ ID NO: 206, SEQ ID NOs: 208 to 211, SEQ ID NO: 215, SEQ ID NO: 218, SEQ ID NO: 219, SEQ ID NO: 221, SEQ ID NO: 224, SEQ ID NO: 225, SEQ ID NO: 228, SEQ ID NO: 229, SEQ ID NO: 231, SEQ ID NO: 232, SEQ ID NO: 236, SEQ ID NO: 237, SEQ ID NO: 240, SEQ ID NOs: 242 to 246, SEQ ID NOs: 248 to 254, SEQ ID NOs: 256 to 258, SEQ ID NOs: 260 to 270, SEQ ID NO: 272, SEQ ID NO: 273, SEQ ID NOs: 275 to 288, SEQ ID NO: 290, SEQ ID NO: 291, SEQ ID NOs: 293 to 296, SEQ ID NO: 298 and SEQ ID NO: 299.

In certain embodiments, the antiviral peptide may be characterized by being an amphipathic peptide.

In certain embodiments, the antiviral peptide may be characterized by being capable of causing damage and/or destruction of a lipid bilayer of a virus.

In this case, the virus may be characterized by belonging to a family of Bunyaviridae, Coronaviridae, Filoviridae, Flaviviridae, Hepadnaviridae, Herpesviridae, Orthomyxoviridae, Poxviridae, Rhabdoviridae, Retroviridae, Togaviridae, or Herpesviridae.

In certain embodiments, the antiviral peptide may be characterized by being capable of forming an α-helix structure.

Some embodiments of the present application provide a pharmaceutical composition for treating a viral disease, including a therapeutically effective amount of the antiviral peptide.

In this case, the viral disease may be a disease caused by an infection of the virus.

In certain embodiments, the pharmaceutical composition further includes a pharmaceutically acceptable carrier and/or pharmaceutically acceptable adjuvant.

In certain embodiments, the viral disease may be a respiratory disease caused by a viral infection or coronavirus disease 2019 (COVID-19).

Some embodiments of the present application provide a method for treating a viral disease, including:
administering a pharmaceutical composition including a therapeutically effective amount of the antiviral peptide to a subject infected with a virus.

Some embodiments of the present application provide a use of the antiviral peptide for treating a viral disease.

Some embodiments of the present application provide a use of the antiviral peptide in the manufacture of a medicament for treating a viral disease.

Some embodiments of the present application provide a method for screening an antiviral peptide candidate group, including selecting peptides that satisfy the following conditions:
1) the peptide consists of 18 amino acid residues;
2) the peptide does not contain proline residue;
3) the peptide includes at least six polar amino acid residues;
4) the peptide contains from four to six charged amino acid residues;
5) the peptide has a hydrophobic moment (µH) value of 0.5 to 0.8, as measured by HeliQuest software;
6) the peptide has a hydrophobicity (H) value of 0.7 to 0.9, as measured by HeliQuest software; and
7) the peptide has a net charge ranging from -2 to 0, as measured by HeliQuest software.

In certain embodiments, the polar amino acid residues may be characterized by being aspartic acid (Asp, D), glutamic acid (Glu, E), lysine (Lys, K), arginine (Arg, R), histidine (His, H), serine (Ser, S), threonine (Thr, T), asparagine (Asn, N), glutamine (Gln, Q) and glycine (Gly, G), and the charged amino acid residues may be characterized by being aspartic acid (Asp, D), glutamic acid (Glu, E), lysine (Lys, K) and arginine (Arg, R).

In certain embodiments, the method may be characterized by selecting a candidate group of antiviral peptides from amino acid sequences disclosed in the Swiss-Prot sequence data.

Some embodiments of the present application provide a method for selecting antiviral peptides, including:
verifying the antiviral efficacy of peptides selected through the method for screening an antiviral peptide candidate group.

In certain embodiments, verifying the antiviral efficacy of selected peptides may be characterized by including at least one selected from the following:
mixing liposomes with the selected peptide and confirming that the liposomes are damaged or destroyed; or
mixing a virus with the selected peptide and confirming that the viral phospholipid bilayer is damaged or destroyed, or confirming that the virus's ability to infect cells is reduced or inactivated.

In certain embodiments, the size of the liposome may be characterized by ranging from 5 nm to 1500 nm.

In certain embodiments, the size of the liposome may be characterized by ranging from 20 nm to 400 nm.

### [Modes of the Invention]

Hereinafter, the content of the invention will be described in more detail through specific exemplary embodiments and examples with reference to the accompanying drawings. It should be noted that the accompanying drawings include some exemplary embodiments of the invention, but not all exemplary embodiments. The content of the invention disclosed by the present specification can be implemented in various ways, and is not limited to the specific exemplary embodiments described herein. These embodiments should be construed as being provided to satisfy the legal requirements applicable to the present specification. A person with ordinary skill in the art to which the invention disclosed herein pertains will be able to conceive of many modifications and other exemplary embodiments of the content of the invention disclosed herein. Therefore, it should be understood that the content of the invention disclosed herein is not limited to the specific exemplary embodiments described herein, and modifications thereof and other exemplary embodiments are also within the scope of the claims.

### Definition of terms

The definitions of the terms used herein are given below.

### About

As used herein, the term "about" refers to an amount, a level, a value, a number, a frequency, a percentage, a dimension, a size, a quantity, a weight, or a length that varies to the degree of 30, 25, 20, 15, 10, 9, 8, 7, 6, 5, 4, 3, 2, or 1% with respect to a reference amount, level, value, number, frequency, percentage, dimension, size, quantity, weight, or length.

### Amino acid sequence notation

Unless otherwise stated, when describing the sequence of a peptide in the present specification, one-letter notation or three-letter notation of an amino acid is used, and it is described in the direction from the N-terminus to the C-terminus. For example, when expressed as RNVP, it refers to a peptide in which arginine, asparagine, valine, and proline are sequentially linked in the direction from the N-terminus to the C-terminus. As another example, when expressed as Thr-Leu-Lys, it refers to a peptide in which threonine, leucine, and lysine are sequentially linked in the direction from the N-terminus to the C-terminus. In the case of amino acids that cannot be represented by one-letter notation, other letters are used to describe these amino acids, and will be described via additional description.

The notation methods of each amino acid are as follows: Alanine (Ala, A); arginine (Arg, R); asparagine (Asn, N); aspartic acid (Asp, D); cysteine (Cys, C); glutamic acid (Glu, E); glutamine (Gln, Q); glycine (Gly, G); histidine (His, H); isoleucine (Ile, I); leucine (Leu, L); lysine (Lys K); methionine (Met, M); phenylalanine (Phe, F); proline (Pro, P); serine (Ser, S); threonine (Thr, T); tryptophan (Trp, W); tyrosine (Tyr, Y); and valine (Val, V).

### Nucleic acid sequence notation

The symbols A, T, C, G and U used in the present specification are to be interpreted as having meanings understood by those skilled in the art. Depending on the context and technology, they may be interpreted as bases, nucleosides or nucleotides on DNA or RNA as appropriate. For example, when meaning a base, each may be interpreted as adenine (A), thymine (T), cytosine (C), guanine (G), or uracil (U) itself, and when meaning a nucleoside, each may be interpreted as adenosine (A), thymidine (T), cytidine (C), guanosine (G), or uridine (U), when meaning a nucleotide in a sequence, each should be construed as meaning a nucleotide including each of the above nucleosides.

In the present specification, the symbol N may be appropriately interpreted as a base, nucleoside, or nucleotide on DNA or RNA, depending on the context and technology. For example, when meaning a base, each may be interpreted as any one of adenine (A), thymine (T), cytosine (C), guanine (G), and uracil (U), and when meaning a nucleoside, each may be interpreted as any one of adenosine (A), thymidine (T), cytidine (C), guanosine (G), and uridine (U), and when meaning a nucleotide in a sequence, each should be construed as meaning a nucleotide including each of the above nucleosides.

### Antiviral

As used herein, the term "antiviral" refers to the property of damaging or destroying the outer membrane of a virus, thereby reducing or inactivating the ability of the virus to infect cells. For example, an "antiviral peptide" refers to a peptide capable of damaging or destroying the outer membrane of a virus, thereby reducing or inactivating the ability of the virus to infect cells.

### Background technology - Known peptides

HCV is an enveloped, single-stranded, positive-sense RNA virus. The single-stranded HCV RNA genome is approximately 9500 nucleotides in length and has a single open reading frame (ORF) encoding a single large protein of approximately 3000 amino acids. In infected cells, this protein is cleaved at various sites by cellular and viral proteases to generate structural and non-structural proteins.

In the case of HCV, the generation of mature nonstructural proteins (NS2, NS3, NS4A, NS4B, NS5A, and NS5B) is influenced by two viral proteases. The first is a metalloprotease located in NS2 that cleaves the NS2-NS3 junction in cis. The second is a serine protease contained within the N-terminal region of NS3 (hereinafter referred to as NS3 protease) and mediates all the subsequent cleavages downstream of NS3, both in cis at the NS3-NS4A cleavage site and in trans at the remaining NS4A-NS4B, NS4B-NS5A, NS5A-NS5B sites. The NS4A protein appears to perform various functions, by acting as a cofactor for the NS3 protease and possibly assisting in the membrane localization of NS3 and other viral replicase components.

Meanwhile, there is NS5A among the non-structural proteins of HCV. As a result of examining the amino acid sequence of NS5A through various studies, it was confirmed that it has an amphipathic α-helix structure at the NH₂-terminus. This amphipathic α-helix is required to mediate membrane binding of NS5A, and is known to anchor the protein by inserting the α-helix into the plane of the membrane. In particular, it is known that disruption of the amphipathic α-helix of NS5A disrupts the membrane binding ability of NS5A and interrupts HCV RNA replication.

Cheng, Guofeng, et al. identified an antiviral peptide from the NS5A protein of HCV (Cheng, Guofeng, et al. "A virocidal amphipathic α-helical peptide that inhibits hepatitis C virus infection in vitro." Proceedings of the National Academy of Sciences 105.8 (2008): 3088-3093.). Specifically, Cheng, Guofeng, et al. confirmed that an amphipathic α-helical peptide (C5A) derived from the membrane anchor domain of the hepatitis C virus (HCV) NS5A protein has antiviral effects against HCV in vitro. C5A has the same amino acid sequence as amino acid sequences Nos. 3 to 20 near the N-terminus of the NS5A protein. Since C5A is derived from the membrane anchor domain of NS5A, which is predicted to be an in-plane amphipathic α-helix, it may destabilize the HCV virion by permeabilizing its envelope. Furthermore, Cheng, Guofeng, et al. compared the antiviral activities of C5A analogs derived from various HCV genotypes. Specifically, the antiviral activities of C5A analogs derived from HCV 1a, 1b, 2a, 3a, 4a, 5a and 6a were compared, and it was confirmed that C5A derived from HCV 1a and C5A analogs derived from HCV 3a (for example, DWLRIIWDWVCSVVSDFK (SEQ ID NO: 301)) exhibited higher antiviral activity (Table 1 in Cheng, Guofeng, et al.).

### 1. Antiviral peptide of present disclosure

### 1.1 Overview - Antiviral peptide of present disclosure

According to one aspect of the present disclosure, an antiviral peptide is disclosed. The antiviral peptide is an amphipathic peptide having both hydrophilic (polar, hydrophilicity) amino acids and hydrophobic (non-polar, hydrophobicity) amino acids. In this case, the antiviral peptide is selected from known amino acid sequences.

Details regarding the structure, characteristics, and the like, which the antiviral peptide of the present disclosure may have, will be described below.

### 1.2 Length of antiviral peptide

The antiviral peptide of the present disclosure consists of 12, 13, 14, 15, 16, 17, 18, 19, 20, 21, 22, 23, 24, 25, 26, 27, 28, 29, 30, 31, 32, 33, 34, 35, or 36 amino acid residues. In other words, the antiviral peptide is a 12-mer, 13-mer, 14-mer, 15-mer, 16-mer, 17-mer, 18-mer, 19-mer, 20-mer, 21-mer, 22-mer, 23-mer, 24-mer, 25-mer, 26-mer, 27-mer, 28-mer, 29-mer, 30-mer, 31-mer, 32-mer, 33-mer, 34-mer, 35-mer, or 36-mer peptide.

As an example, the number of amino acid residues constituting the antiviral peptide may be between any two values selected in the immediately preceding sentence. For example, the antiviral peptide may consist of 16 to 27 amino acid residues.

Preferably, the number of amino acid residues constituting the antiviral peptide may be 18. In other words, the antiviral peptide may consist of 18 amino acid residues. In other words, the antiviral peptide may be an 18-mer peptide.

### 1.3 Amino acid residues of antiviral peptides

The antiviral peptide of the present disclosure includes hydrophilic amino acid residues and hydrophobic amino acid residues. Here, the hydrophilic amino acid residues may include arginine (Arg, R), asparagine (Asn, N), aspartic acid (Asp, D), cysteine (Cys, C), glutamic acid (Glu, E), glutamine (Gln, Q), glycine (Gly, G), histidine (His, H), lysine (Lys, K), serine (Ser, S), and/or threonine (Thr, T). The hydrophobic amino acid residues may include alanine (Ala, A), isoleucine (Ile, I), leucine (Leu, L), methionine (Met, M), proline (Pro, P), valine (Val, V), phenylalanine (Phe, F), tyrosine (Tyr, Y), and/or tryptophan (Trp, W).

In some embodiments, the numbers of hydrophilic amino acid residues and hydrophobic amino acid residues included in the antiviral peptide may be the same. In some other embodiments, the antiviral peptide may include 1, 2, 3, 4, or 5 more hydrophilic amino acid residues than hydrophobic amino acid residues. In some other embodiments, the antiviral peptide may include 1, 2, 3, 4, or 5 more hydrophobic amino acid residues than hydrophilic amino acid residues. In preferred embodiments, the antiviral peptide may include 4 more hydrophobic amino acid residues than hydrophilic amino acid residues.

In some embodiments, when the antiviral peptide is an 18-mer peptide, the antiviral peptide may include 7 hydrophilic amino acid residues and 11 hydrophobic amino acid residues. In some other embodiments, when the antiviral peptide is an 18-mer peptide, the antiviral peptide may include 8 hydrophilic amino acid residues and 10 hydrophobic amino acid residues. In some other embodiments, when the antiviral peptide is an 18-mer peptide, the antiviral peptide may include 9 hydrophilic amino acid residues and 9 hydrophobic amino acid residues. In some other embodiments, when the antiviral peptide is an 18-mer peptide, the antiviral peptide may include 10 hydrophilic amino acid residues and 8 hydrophobic amino acid residues. In some other embodiments, when the antiviral peptide is an 18-mer peptide, the antiviral peptide may include 11 hydrophilic amino acid residues and 7 hydrophobic amino acid residues.

### 1.4 Structure of antiviral peptide - α-helix structure

The antiviral peptide of the present disclosure may form an α-helix structure when reacting with a lipid membrane. In this case, the reaction of the antiviral peptide with a lipid membrane may mean a case where the antiviral peptide comes into contact with a lipid membrane (specifically, a phospholipid bilayer).

If the antiviral peptide has an α-helix structure, the positions of the amino acid residues may be schematically represented as shown in FIG. 1, when viewed from the top down along the axis of the α-helix structure. FIG. 1 exemplifies one of the known antiviral peptides in order to describe the α-helix structure of an antiviral peptide, and the amino acid sequence of the peptide illustrated in FIG. 1 is N-terminus-DWLRIIWDWVCSVVSDFK (SEQ ID NO: 301)-C-terminus. When the peptide has an α-helix structure rotating around a virtual first axis and then the peptide is viewed in one direction parallel to the virtual first axis, each amino acid can be viewed as illustrated in FIG. 1. In this case, when one virtual plane including the virtual first axis (the virtual plane is shown by a dashed dotted line in FIG. 1) is carefully selected, the peptide may be divided into hydrophilic region and hydrophobic region by the virtual plane. In this case, each of the two regions divided by the virtual plane includes nine amino acid residues.

In the present specification, the hydrophilic region may refer to a region including more hydrophilic amino acid residues among the two regions divided by the virtual plane, and the hydrophobic region may refer to the other region of the two divided regions, which is not the hydrophilic region.

In some embodiments, the hydrophilic region may comprise only hydrophilic amino acid residues and not comprise hydrophobic amino acid residues. That is, the hydrophilic region may comprise nine hydrophilic amino acid residues.

In some other embodiments, the hydrophilic region may comprise eight hydrophilic amino acid residues and one hydrophobic amino acid residue.

In some other embodiments, the hydrophilic region may comprise seven hydrophilic amino acid residues and two hydrophobic amino acid residues.

In some other embodiments, the hydrophilic region may comprise six hydrophilic amino acid residues and three hydrophobic amino acid residues.

In some embodiments, the hydrophobic region may comprise only hydrophobic amino acid residues and not comprise hydrophilic amino acid residues. That is, the hydrophobic region may comprise nine hydrophobic amino acid residues.

In some other embodiments, the hydrophobic region may comprise eight hydrophobic amino acid residues and one hydrophilic amino acid residue.

In some other embodiments, the hydrophobic region may comprise seven hydrophobic amino acid residues and two hydrophilic amino acid residues.

In some other embodiments, the hydrophobic region may comprise six hydrophobic amino acid residues and three hydrophilic amino acid residues.

In an embodiment, each hydrophilic amino acid or hydrophilic amino acid residue may be any one selected from arginine (Arg, R), asparagine (Asn, N), aspartic acid (Asp, D), cysteine (Cys, C), glutamic acid (Glu, E), glutamine (Gln, Q), glycine (Gly, G), histidine (His, H), lysine (Lys, K), serine (Ser, S), and threonine (Thr, T).

In an embodiment, each hydrophobic amino acid or hydrophobic amino acid residue may be any one selected from alanine (Ala, A), isoleucine (Ile, I), leucine (Leu, L), methionine (Met, M), valine (Val, V), phenylalanine (Phe, F), tyrosine (Tyr, Y), and tryptophan (Trp, W).

### 1.5 Characteristics of antiviral peptides - amphipathicity

The antiviral peptide of the present disclosure is an amphipathic peptide. Specifically, the antiviral peptide does not have amphipathic characteristics when having a linear structure, but does have amphipathic characteristics when having an α-helix structure. This is because, due to the characteristics of the amino acid sequence of the antiviral peptide, when the antiviral peptide has a linear structure, there is no region in which hydrophilic or hydrophobic amino acid residues are intensively distributed in a single structure, but when the antiviral peptide has an α-helix structure, there is a region in which hydrophilic or hydrophobic amino acid residues are intensively distributed in a single structure. That is, the antiviral peptide has amphipathic characteristics when it forms an α-helix structure, dither dure to interaction with lipid membranes or for other reasons.

The amphipathic characteristics mean that when the peptide is considered to have a three-dimensional structure such as an α-helix structure, one side of the peptide exhibits hydrophilic (polar, hydrophilicity) properties and the other side exhibits hydrophobic (non-polar, hydrophobicity) properties in the three-dimensional structure. That is, when the peptide has amphipathicity, it means that the peptide has both hydrophobic and hydrophilic properties.

The amphipathic characteristics of the antiviral peptide may be described through the distribution of hydrophilic amino acid residues and hydrophobic amino acid residues in the three-dimensional helix structure of the antiviral peptide, as described above in section <<1.4 Structure of antiviral peptide - α-helix structure>>. Specifically, in any one region of the two regions into which the α-helix structure is divided by the virtual plane, more hydrophilic amino acid residues are distributed than hydrophobic amino acid residues, and in the other region, more hydrophobic amino acid residues are distributed than hydrophilic amino acid residues, so that the antiviral peptide having the α-helix structure has both hydrophilic property and hydrophobic property in a single structure.

The amphipathic characteristics are presumed to be one of the reasons for the efficacy of the antiviral peptide in acting on phospholipid bilayers.

As an example, the amphipathic characteristics of the antiviral peptide may be measured using HeliQuest software. In some embodiments, when the sequence of the antiviral peptide is input into HeliQuest software, a hydrophobic moment (µH) value of any one of 0.5 to 0.8 may be output. That is, the antiviral peptide may have any one of hydrophobic moment values (hydrophobic moment values calculated by HeliQuest software) of 0.5 to 0.8. In some embodiments, when the sequence of the antiviral peptide is input into HeliQuest software, a hydrophobicity (H) value of any one of 0.7 to 0.9 may be output. That is, the antiviral peptide may have any one of hydrophobic values (hydrophobic values calculated by HeliQuest software) of 0.7 to 0.9. In some embodiments, when the sequence of the antiviral peptide is input into HeliQuest software, a net charge of any one ranging from -2 to 0 may be output. That is, the antiviral peptide may have a net charge (net charge calculated by HeliQuest software) of any one ranging from -2 to 0.

### 1.6 Sequence of antiviral peptide

The amino acid sequence of the antiviral peptide disclosed in the present disclosure may be any one selected from SEQ ID NO: 1, SEQ ID NOs: 3 to 5, SEQ ID NOs: 8 to 23, SEQ ID NO: 26, SEQ ID NO: 29, SEQ ID NO: 30, SEQ ID NO: 33, SEQ ID NO: 35, SEQ ID NO: 36, SEQ ID NOs: 38 to 40, SEQ ID NO: 43, SEQ ID NOs: 45 to 52, SEQ ID NO: 55, SEQ ID NO: 58, SEQ ID NO: 59, SEQ ID NO: 62, SEQ ID NOs: 64 to 73, SEQ ID NO: 75, SEQ ID NOs: 78 to 87, SEQ ID NOs: 90 to 93, SEQ ID NOs: 97 to 102, SEQ ID NOs: 104 to 106, SEQ ID NOs: 109 to 112, SEQ ID NOs: 114 to 116, SEQ ID NOs: 118 to 123, SEQ ID NO: 126, SEQ ID NO: 127, SEQ ID NOs: 130 to 132, SEQ ID NO: 136, SEQ ID NOs: 138 to 147, SEQ ID NOs: 151 to 155, SEQ ID NO: 162, SEQ ID NO: 164, SEQ ID NOs: 166 to 168, SEQ ID NOs: 170 to 174, SEQ ID NO: 176, SEQ ID NOs: 180 to 182, SEQ ID NOs: 184 to 186, SEQ ID NO: 188, SEQ ID NO: 189, SEQ ID NO: 191, SEQ ID NO: 192, SEQ ID NOs: 194 to 196, SEQ ID NO: 198, SEQ ID NO: 201, SEQ ID NO: 204, SEQ ID NO: 206, SEQ ID NOs: 208 to 211, SEQ ID NO: 215, SEQ ID NO: 218, SEQ ID NO: 219, SEQ ID NO: 221, SEQ ID NO: 224, SEQ ID NO: 225, SEQ ID NO: 228, SEQ ID NO: 229, SEQ ID NO: 231, SEQ ID NO: 232, SEQ ID NO: 236, SEQ ID NO: 237, SEQ ID NO: 240, SEQ ID NOs: 242 to 246, SEQ ID NOs: 248 to 254, SEQ ID NOs: 256 to 258, SEQ ID NOs: 260 to 270, SEQ ID NO: 272, SEQ ID NO: 273, SEQ ID NOs: 275 to 288, SEQ ID NO: 290, SEQ ID NO: 291, SEQ ID NOs: 293 to 296, SEQ ID NO: 298 and SEQ ID NO: 299, or a sequence having 83, 84, 85, 86, 87, 88, 89, 90, 91, 92, 93, 94, 95, 96, 97, 98, or 99% or more homology (sequence identity) thereto.

In certain embodiments, the amino acid sequence of the antiviral peptide may be any one selected from SEQ ID NO: 1, SEQ ID NO: 3, SEQ ID NO: 4, SEQ ID NO: 5, SEQ ID NO: 20, SEQ ID NO: 29, SEQ ID NO: 30, SEQ ID NO: 46, SEQ ID NO: 48, SEQ ID NO: 49, SEQ ID NO: 52, SEQ ID NO: 59, SEQ ID NO: 78, SEQ ID NO: 82, SEQ ID NO: 87, SEQ ID NO: 90 SEQ ID NO: 91, SEQ ID NO: 98, SEQ ID NO: 99, SEQ ID NO: 101, SEQ ID NO: 106, SEQ ID NO: 110, SEQ ID NO: 112, SEQ ID NO: 114, SEQ ID NO: 115, SEQ ID NO: 116, SEQ ID NO: 123, SEQ ID NO: 126, SEQ ID NO: 131, SEQ ID NO: 132, SEQ ID NO: 139, SEQ ID NO: 141, SEQ ID NO: 143, SEQ ID NO: 144, SEQ ID NO: 151, SEQ ID NO: 162, SEQ ID NO: 167, SEQ ID NO: 171, SEQ ID NO: 189, SEQ ID NO: 221, SEQ ID NO: 228, SEQ ID NO: 242, SEQ ID NO: 245, SEQ ID NO: 246, SEQ ID NO: 254, SEQ ID NO: 256, SEQ ID NO: 264, SEQ ID NO: 283, SEQ ID NO: 290 and SEQ ID NO: 296, or a sequence having 83, 84, 85, 86, 87, 88, 89, 90, 91, 92, 93, 94, 95, 96, 97, 98, or 99% or more homology (sequence identity) thereto.

In certain embodiments, the amino acid sequence of the antiviral peptide may be any one selected from SEQ ID NO: 1, SEQ ID NO: 3, SEQ ID NO: 4, SEQ ID NO: 20, SEQ ID NO: 46, SEQ ID NO: 48, SEQ ID NO: 49, SEQ ID NO: 59, SEQ ID NO: 87, SEQ ID NO: 90, SEQ ID NO: 91, SEQ ID NO: 98, SEQ ID NO: 101, SEQ ID NO: 110, SEQ ID NO: 114, SEQ ID NO: 116, SEQ ID NO: 126, SEQ ID NO: 132, SEQ ID NO: 141, SEQ ID NO: 143, SEQ ID NO: 144, SEQ ID NO: 151, SEQ ID NO: 221, SEQ ID NO: 246, SEQ ID NO: 256, SEQ ID NO: 264, SEQ ID NO: 283, SEQ ID NO: 290 and SEQ ID NO: 296, or a sequence having 83, 84, 85, 86, 87, 88, 89, 90, 91, 92, 93, 94, 95, 96, 97, 98, or 99% or more homology (sequence identity) thereto.

In certain embodiments, the amino acid sequence of the antiviral peptide may be any one selected from SEQ ID NO: 1, SEQ ID NO: 3, SEQ ID NO: 4, SEQ ID NO: 46, SEQ ID NO: 49, SEQ ID NO: 101, SEQ ID NO: 143, SEQ ID NO: 185 and SEQ ID NO: 191, or a sequence having 83, 84, 85, 86, 87, 88, 89, 90, 91, 92, 93, 94, 95, 96, 97, 98, or 99% or more homology (sequence identity) thereto.

In certain embodiments, the amino acid sequence of the antiviral peptide may be any one selected from SEQ ID NO: 3, SEQ ID NO: 4, SEQ ID NO: 46, SEQ ID NO: 49, SEQ ID NO: 101, SEQ ID NO: 110, SEQ ID NO: 114, SEQ ID NO: 116, SEQ ID NO: 143, SEQ ID NO: 151, SEQ ID NO: 171 and SEQ ID NO: 221, or a sequence having 83, 84, 85, 86, 87, 88, 89, 90, 91, 92, 93, 94, 95, 96, 97, 98, or 99% or more homology (sequence identity) thereto.

In certain embodiments, the amino acid sequence of the antiviral peptide may be any one selected from SEQ ID NO: 1, SEQ ID NO: 3 and SEQ ID NO: 101, or a sequence having 83, 84, 85, 86, 87, 88, 89, 90, 91, 92, 93, 94, 95, 96, 97, 98, or 99% or more homology (sequence identity) thereto.

In certain embodiments, the amino acid sequence of the antiviral peptide may be SEQ ID NO: 49 or a sequence having 83, 84, 85, 86, 87, 88, 89, 90, 91, 92, 93, 94, 95, 96, 97, 98, or 99% or more homology (sequence identity) thereto.

Meanwhile, the antiviral effects of the peptides selected by the method for selecting an antiviral peptide candidate were confirmed through Experimental Examples 3 to 7, as described below.

### 1.7 Method for producing antiviral peptide

The antiviral peptide disclosed in the present disclosure may be produced by known peptide synthesis methods. For example, the antiviral peptide may be synthesized by general solid-phase peptide synthesis (SPPS) peptide synthesis methods. As a specific example, the antiviral peptide of the present disclosure may be produced by the method disclosed in Experimental Example 2 of the present application.

### 2. Method for screening antiviral peptides

### 2.1 Method for screening a candidate group for antiviral peptides

The inventors of the present application screened a novel antiviral peptide candidate based on the characteristics of a peptide having an amino acid sequence DWLRIIWDWVCSVVSDFK (SEQ ID NO: 301) derived from HCV genotype 3a.

Accordingly, as one aspect of the present disclosure, a method for screening a candidate group for antiviral peptides is disclosed. Detail methods of the screening method will be described below.

### 2.1.1 Peptide conditions

The method for screening a candidate group for antiviral peptides of the present disclosure includes selecting, finding, and/or searching for peptides that satisfy at least one of the following conditions:
1) the peptide consists of 12 to 36 amino acid residues (or the length of the peptide is 12-mer to 36-mer);
2) the peptide does not comprise proline residues;
3) the peptide comprises at least six polar amino acid residues;
4) the peptide comprises from four to six charged amino acid residues;
5) the peptide has a hydrophobic moment (µH) value of 0.5 to 0.8;
6) the peptide has a hydrophobicity (H) value of 0.7 to 0.9; and
7) the peptide has a net charge ranging from -2 to 0.

In some embodiments, the polar amino acid residues may be aspartic acid (Asp, D), glutamic acid (Glu, E), lysine (Lys, K), arginine (Arg, R), histidine (His, H), serine (Ser, S), threonine (Thr, T), asparagine (Asn, N), glutamine (Gln, Q) and glycine (Gly, G).

In some embodiments, the charged amino acid residues may be aspartic acid (Asp, D), glutamic acid (Glu, E), lysine (Lys, K) and arginine (Arg, R).

In some embodiments, the hydrophobic moment (µH) value of the peptide may refer to a value as measured by HeliQuest software.

In some embodiments, the hydrophobicity (H) value may refer to a value as measured by HeliQuest software.

In some embodiments, the net charge of the peptide may refer to a value as measured by HeliQuest software.

In some embodiments, the method for screening an antiviral peptide candidate group may comprise selecting, finding, and/or searching for peptides that satisfy the following conditions:
the number of amino acid residues constituting the peptide is 12, 13, 14, 15, 16, 17, 18, 19, 20, 21, 22, 23, 24, 25, 26, 27, 28, 29, 30, 31, 32, 33, 34, 35 or 36, or falls within a range defined by any two numerical values selected from these numerical values (for example, 18 to 27).

In some embodiments, the method for screening an antiviral peptide candidate group may comprise selecting, finding, and/or searching for peptides that satisfy the following conditions:
among the amino acid residues constituting the peptide, the number of charged amino acid residues is 4, 5, or 6, or falls within a range defined by any two numerical values selected from these numerical values (for example, 4 to 6).

In some embodiments, the method for screening a candidate group for antiviral peptides may include selecting, finding, and/or searching for peptides that satisfy the following conditions:
the hydrophobic moment hydrophobic moment (µH) value of the peptide is about 0.50, about 0.51, about 0.52, about 0.53, about 0.54, about 0.55, about 0.56, about 0.57, about 0.58, about 0.59, about 0.60, about 0.61, about 0.62, about 0.63, about 0.64, about 0.65, about 0.66, about 0.67, about 0.68, about 0.69, about 0.70, about 0.71, about 0.72, about 0.73, about 0.74, about 0.75, about 0.76, about 0.77, about 0.78, about 0.79 or about 0.80, or falls within a range defined by any two numerical values selected from these numerical values (for example, 0.50 to 0.80).

In some embodiments, the method for screening a candidate group for antiviral peptides may include selecting, finding, and/or searching for peptides that satisfy the following conditions:
the hydrophobicity (H) value of the peptide is about 0.70, about 0.71, about 0.72, about 0.73, about 0.74, about 0.75, about 0.76, about 0.77, about 0.78, about 0.79, about 0.80, about 0.81, about 0.82, about 0.83, about 0.84, about 0.85, about 0.86, about 0.87, about 0.88, about 0.89 or about 0.90, or falls within a range defined by any two numerical values selected from these numerical values (for example, 0.70 to 0.90).

In some embodiments, the method for screening a candidate group for antiviral peptides may include selecting, finding, and/or searching for peptides that satisfy the following conditions:
the net charge of the peptide is about -2.0, about -1.9, about -1.8, about -1.7, about -1.6, about -1.5, about -1.4, about -1.3, about -1.2, about -1.1, about -1.0, about -0.9, about -0.8 , about -0.7, about -0.6, about -0.5, about -0.4, about -0.3, about -0.2, about - 0.1 or about 0.0, or falls within a range defined by any two numerical values selected from these numerical values (for example, -2.0 to 0.0).

In specific embodiments, the method for screening a candidate group for antiviral peptides includes selecting, finding, and/or searching for peptides that satisfy at least one of the following conditions:
1) the peptide consists of 18 amino acid residues (in other words, the length of the peptide is 18-mer);
2) the peptide does not comprise proline residues;
3) the peptide includes at least six polar amino acid residues, wherein the polar amino acid residues are aspartic acid (Asp, D), glutamic acid (Glu, E), lysine (Lys, K), arginine (Arg, R), histidine (His, H), serine (Ser, S), threonine (Thr, T), asparagine (Asn, N), glutamine (Gln, Q) and glycine (Gly, G);
4) the peptide comprises from four to six charged amino acid residues, wherein the charged amino acid residues are aspartic acid (Asp, D), glutamic acid (Glu, E), lysine (Lys, K) and arginine (Arg, R);
5) the peptide has a hydrophobic moment (µH) value of 0.5 to 0.8, as measured by HeliQuest software;
6) the peptide has a hydrophobicity (H) value of 0.7 to 0.9, as measured by HeliQuest software; and
7) the peptide has a net charge ranging from -2 to 0, as measured by HeliQuest software.

### 2.1.2 Use of database

The method for screening a candidate group for antiviral peptides of the present disclosure may include selecting, searching, and/or searching for peptides that satisfy the conditions disclosed in Section <<2.2 Peptide conditions>> of the present disclosure from amino acid sequences disclosed in a known database:
In certain embodiments, the known database may be the SwissProt Database (or Swiss-Prot sequence data).

In certain embodiments, the amino acid sequences disclosed in the known database may be amino acid sequences derived from the proteins of a human or non-human animal (for example, rat, mouse, or yeast).

### 2.1.3 Examples of screened candidate group for antiviral peptides

The present inventors have selected peptides represented by any one of the amino acid sequences of SEQ ID NOs: 1 to 300 as candidate groups for antiviral peptides through the method for screening a candidate group for antiviral peptides, which includes selecting peptides that satisfy the following conditions from the amino acid sequences disclosed in the Swiss-Prot sequence data.

### 2.2 Antiviral peptide selection method

According to one aspect of the present disclosure, a method for selecting antiviral peptides is disclosed. The method for selecting antiviral peptides includes selecting antiviral peptides from peptides selected by the method for screening a candidate group for antiviral peptides disclosed in Section <<2.1 Method for screening a candidate group for antiviral peptides>> of the present disclosure. In this case, selecting antiviral peptides mean the process of measuring, confirming, determining, and/or experimenting the antiviral effect of the selected peptides.

In this case, the process of measuring, confirming, determining, and/or experimenting the antiviral effect may include at least one selected from the following:
mixing liposomes (or a solution including liposomes) with the selected peptide (or a solution including the selected peptide) and confirming that the liposomes are damaged or destroyed; or
mixing a virus (or a solution including virus) with the selected peptide (or a solution including the selected peptide) and confirming that the viral phospholipid bilayer is damaged or destroyed, or confirming that the virus's ability to infect cells is reduced or inactivated.

In some embodiments, the size of the liposome (or the distance between one phosphate group moiety and the furthest phosphate group moiety in the phospholipid constituting the liposome) may be 5 nm, 6 nm, 7 nm, 8 nm, 9 nm, 10 nm, 20 nm, 30 nm, 40 nm, 50 nm, 60 nm, 70 nm, 80 nm, 90 nm, 100 nm, 150 nm, 200 nm, 250 nm, 300 nm, 350 nm, 400 nm, 500 nm, 600 nm, 700 nm, 800 nm, 900 nm, 1000 nm, 1100 nm, 1200 nm, 1300 nm, 1400 nm or 1500 nm, or a value between any two values selected from these values (for example, 55 nm), or may fall within a range defined by any two numerical values selected from these numerical values (for example, 5 nm to 1500 nm).

In certain embodiments, the size of the liposome (or the distance between one phosphate group moiety and the furthest phosphate group moiety in the phospholipid constituting the liposome) may range from 20 nm to 400 nm.

In certain embodiments, the size of the liposome (or the distance between one phosphate group moiety and the furthest phosphate group moiety in the phospholipid constituting the liposome) may be 55 nm, 150 nm, or 250 nm.

In certain embodiments, the virus may be belonging to a family of *Bunyaviridae, Coronaviridae, Filoviridae, Flaviviridae, Hepadnaviridae, Herpesviridae, Orthomyxoviridae, Poxviridae, Rhabdoviridae, Retroviridae, Togaviridae,* or *Herpesviridae.* For example, the virus may be at least one virus selected from the group consisting of Sin Nombre Hantavirus which belongs to the *Bunyaviridae* family; coronavirus which belongs to the *Coronaviridae* family and is involved in various acute respiratory syndromes; Ebola virus and Marburg virus which belong to the *Filoviridae* family; West Nile virus, yellow fever virus, dengue fever virus, and hepatitis C virus which belong to the *Flaviviridae* family; hepatitis B virus which belongs to the *Hepadnaviridae* family; herpes simplex 1 virus and herpes simplex 2 virus which belong to the *Herpesviridae* family; influenza virus which belongs to the *Orthomyxoviridae* family; smallpox virus, vaccinia virus, molluscum contagiosum virus, and monkeypox virus which belong to the *Poxviridae* family; rabies virus which belongs to the *Rhabdoviridae* family; human Immunodeficiency virus (HIV) which belongs to the *Retroviridae* family; chikungunya virus which belongs to the *Togaviridae* family; pseudorabies virus and human herpes virus (HHV) which belong to the family of *Herpesviridae*; and influenza virus which belongs to the *Orthomyxoviridae* family.

In certain embodiments, the virus may be dengue virus or SARS-CoV-2 virus.

The present inventors have selected peptides, represented by any one amino acid sequence selected from SEQ ID NO: 1, SEQ ID NOs: 3 to 5, SEQ ID NOs: 8 to 23, SEQ ID NO: 26, SEQ ID NO: 29, SEQ ID NO: 30, SEQ ID NO: 33, SEQ ID NO: 35, SEQ ID NO: 36, SEQ ID NOs: 38 to 40, SEQ ID NO: 43, SEQ ID NOs: 45 to 52, SEQ ID NO: 55, SEQ ID NO: 58, SEQ ID NO: 59, SEQ ID NO: 62, SEQ ID NOs: 64 to 73, SEQ ID NO: 75, SEQ ID NOs: 78 to 87, SEQ ID NOs: 90 to 93, SEQ ID NOs: 97 to 102, SEQ ID NOs: 104 to 106, SEQ ID NOs: 109 to 112, SEQ ID NOs: 114 to 116, SEQ ID NOs: 118 to 123, SEQ ID NO: 126, SEQ ID NO: 127, SEQ ID NOs: 130 to 132, SEQ ID NO: 136, SEQ ID NOs: 138 to 147, SEQ ID NOs: 151 to 155, SEQ ID NO: 162, SEQ ID NO: 164, SEQ ID NOs: 166 to 168, SEQ ID NOs: 170 to 174, SEQ ID NO: 176, SEQ ID NOs: 180 to 182, SEQ ID NOs: 184 to 186, SEQ ID NO: 188, SEQ ID NO: 189, SEQ ID NO: 191, SEQ ID NO: 192, SEQ ID NOs: 194 to 196, SEQ ID NO: 198, SEQ ID NO: 201, SEQ ID NO: 204, SEQ ID NO: 206, SEQ ID NOs: 208 to 211, SEQ ID NO: 215, SEQ ID NO: 218, SEQ ID NO: 219, SEQ ID NO: 221, SEQ ID NO: 224, SEQ ID NO: 225, SEQ ID NO: 228, SEQ ID NO: 229, SEQ ID NO: 231, SEQ ID NO: 232, SEQ ID NO: 236, SEQ ID NO: 237, SEQ ID NO: 240, SEQ ID NOs: 242 to 246, SEQ ID NOs: 248 to 254, SEQ ID NOs: 256 to 258, SEQ ID NOs: 260 to 270, SEQ ID NO: 272, SEQ ID NO: 273, SEQ ID NOs: 275 to 288, SEQ ID NO: 290, SEQ ID NO: 291, SEQ ID NOs: 293 to 296, SEQ ID NO: 298 and SEQ ID NO: 299, from peptides represented by any one amino acid sequence of SEQ ID NOs: 1 to 300, as antiviral peptides.

### 3. Effect of antiviral peptide of present disclosure

### 3.1 Overview - Effects of antiviral peptides

The antiviral peptide of the present disclosure causes damage to the outer membrane (or phospholipid bilayer) of the virus.

The antiviral peptide of the present disclosure may cause destruction of the outer membrane (or phospholipid bilayer) of the virus. Specifically, the antiviral peptide may cause destruction of the viral phospholipid bilayer by causing damage to the viral phospholipid bilayer.

The antiviral peptide of the present disclosure may cause the virus's ability to infect cells to be reduced or inactivated. Specifically, the antiviral peptide may cause the virus's ability to infect cells to be reduced or inactivated by causing damage (or destruction) to the phospholipid bilayer.

The antiviral peptide of the present disclosure may cause the virus's ability to proliferate (or replicate) to be reduced or inactivated. Specifically, the antiviral peptide may cause the virus's ability to proliferate (or replicate) to be reduced or inactivated by causing damage (or destruction) to the phospholipid bilayer.

### 3.2 Effect of antiviral peptide on damage (or destruction) of viral outer membrane

The antiviral peptide of the present disclosure causes damage to the outer membrane (or phospholipid bilayer) of the virus. Further, the antiviral peptide may cause destruction of the viral outer membrane (or phospholipid bilayer) by causing damage to the viral outer membrane (or phospholipid bilayer).

Here, the damage (or destruction) to the viral outer membrane (or phospholipid bilayer) by the antiviral peptide may be caused by the insertion of the antiviral peptide into the phospholipid bilayer of the virus. Those skilled in the art who study the antiviral peptide disclosed in the present specification understand that the mechanism by which the antiviral peptide is inserted into the viral phospholipid bilayer to damage (or destroy) the viral phospholipid bilayer is related to structural characteristics according to the size of the virus. The detailed explanation on this is as follows.

The size surrounded by an outer membrane is much smaller in viruses than in cells. Due to this difference in size, the phospholipid bilayer of the viral outer membrane has structural characteristics that distinguish it from the phospholipid bilayer of the cellular outer membrane. In order to explain in detail for the structural characteristics of the phospholipid bilayer of the viral outer membrane, for convenience of calculation, the shapes of the virus and cells are assumed to be perfect spheres, and the differences are compared through the formula for calculating the surface area of a sphere.

First, the phospholipids that make up the phospholipid bilayer of the outer membrane may be divided into an inner layer and an outer layer. Here, the inner layer refers to the layer of the phospholipids in the phospholipid bilayer in which the phosphate group moiety faces the inside of the cell. The outer layer refers to the layer of phospholipids in the phospholipid bilayer in which the phosphate group moiety faces the outside of the cell.

When the distance between any one phosphate moiety and the furthest phosphate moiety in the outer layer is called the diameter (=2R) and the length of the fatty acid moiety of the phospholipid is called an a (assuming that the phosphate group moiety of the phospholipid has no volume and length), (Surface area consisting of phosphate group moieties in the outer layer) = 4 × π × R3 (Surface area consisting of phosphate group moieties in the inner layer) = 4 × π × (R - 2a)3 (Surface area ratio) = (Surface area consisting of phosphate group moieties in the inner layer) / (Surface area consisting of phosphate group moieties in the outer layer) = (R - 2a)3/ R3 = (1-(2a/R))3.

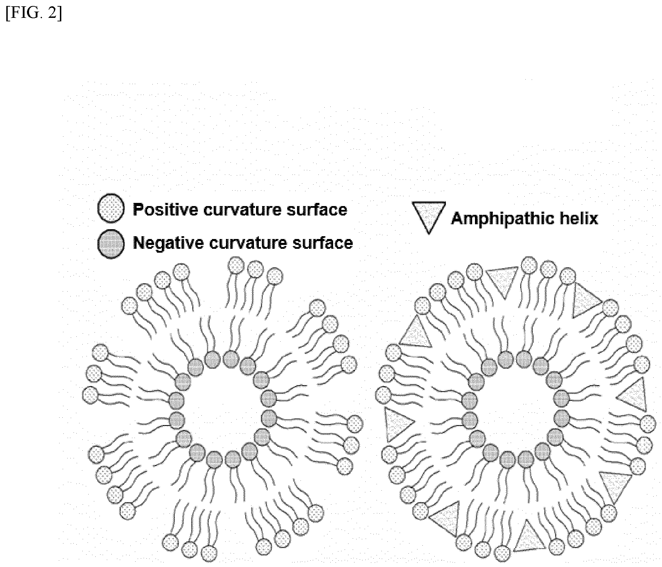

In this case, the length of the fatty acid (a) is generally a fixed value, and the value of R varies greatly depending on the size of the virus or cell. In consideration of this point, as the value of R increases, the surface area ratio approaches 1, and as the value of R decreases, the surface area ratio decreases. In other words, since viruses with low R values have a larger difference in the surface area ratio between the inner layer and the outer layer than cells with high R values, the virus has a structure in which the spacing between the phosphate groups on its outer layer is more widely separated compared to that of cell (see FIG. 2).

That is, the viral phospholipid bilayer may have a structure in which there are gaps that are not present in cells or that are larger than those in cells. In consideration of these structural differences, it is expected that the antiviral peptide of the present disclosure will be able to be more easily inserted into viral phospholipid bilayers than cellular ones. In this case, there are experimental results showing that the antiviral peptide inserted into the viral phospholipid bilayer may cause damage or destruction of the phospholipid bilayer, but there is no exact known mechanism for this.

Therefore, it is expected that the antiviral peptide of the present disclosure will only cause damage or destruction of the viral phospholipid bilayer, and will not a significant effect on cells. The reason for this, as described above, is that viruses have a smaller overall size (or diameter), and thus an outer membrane with a greater curvature than cells.

Through the above mechanism, the antiviral peptide of the present disclosure may not only cause damage or destruction of the phospholipid bilayer of a specific virus, but may also cause general damage or destruction of the phospholipid bilayer of viruses that have an outer membrane of a phospholipid bilayer.

It can be confirmed through the experimental methods and results disclosed in Experimental Examples 3 to 5 that the antiviral peptide of the present disclosure may cause damage or destruction of the viral phospholipid bilayer.

Furthermore, through an experiment in which liposomes with different sizes were used in Experimental Example 4.2, it can be seen that the antiviral peptide of the present disclosure caused a high level of destruction (or damage) of the phospholipid bilayer of liposomes with small diameters. These results show that due to the difference in size between cells and viruses, the antiviral peptide of the present disclosure may cause damage (or destruction) of the phospholipid bilayer of viruses other than cells.

### 3.3 Target virus

In the present disclosure, viruses whose phospholipid bilayer is damaged, destroyed, or the like by the antiviral peptide are viruses that have an envelope (or membrane) of a phospholipid bilayer.

The virus may be a virus belonging to a family of *Bunyaviridae, Coronaviridae, Filoviridae, Flaviviridae, Hepadnaviridae, Herpesviridae, Orthomyxoviridae, Poxviridae, Rhabdoviridae, Retroviridae, Togaviridae,* or *Herpesviridae.* For example, the virus may be at least one virus selected from the group consisting of Sin Nombre Hantavirus which belongs to the *Bunyaviridae* family; coronavirus which belongs to the *Coronaviridae* family and is involved in various acute respiratory syndromes; Ebola virus and Marburg virus which belong to the *Filoviridae* family; West Nile virus, yellow fever virus, dengue fever virus, and hepatitis C virus which belong to the *Flaviviridae* family; hepatitis B virus which belongs to the *Hepadnaviridae* family; herpes simplex 1 virus and herpes simplex 2 virus which belong to the *Herpesviridae* family; influenza virus which belongs to the *Orthomyxoviridae* family; smallpox virus, vaccinia virus, molluscum contagiosum virus, and monkeypox virus which belong to the *Poxviridae* family; rabies virus which belongs to the *Rhabdoviridae* family; human Immunodeficiency virus (HIV) which belongs to the *Retroviridae* family; chikungunya virus which belongs to the *Togaviridae* family; pseudorabies virus and human herpes virus (HHV) which belong to the family of *Herpesviridae*; and influenza virus which belongs to the *Orthomyxoviridae* family.

In certain embodiments, the virus may be dengue virus or SARS-CoV-2 virus.

### 4. Use of antiviral peptide of present disclosure

### 4.1 Overview of use of antiviral peptide of present disclosure

According to one aspect of the present disclosure, the antiviral peptide of the present disclosure may be used to cause destruction or damage to the lipid bilayer of a virus. The destruction or damage to the lipid bilayer reduces the virus's infectivity, which can be expected to have a preventive or therapeutic effect on viral infections (or related diseases). That is, the antiviral peptide may be used as an effective therapeutic agent for viral infections (or related diseases) by inducing destruction or damage to the lipid bilayer. Here, the virus is a virus having an envelope (or membrane) of a phospholipid bilayer.

Various embodiments in which the antiviral peptide is used in connection with the treatment of viral infections (or related diseases) will be described below.

### 4.2 Target disease

A target disease for which the antiviral peptide of the present disclosure may be used for therapeutic purposes is a viral infection (or a related disease).

In some embodiments, a virus that is the target of lipid bilayer destruction (or damage) or treatment with the antiviral peptide may be a virus belonging to a family of *Bunyaviridae, Coronaviridae, Filoviridae, Flaviviridae, Hepadnaviridae, Herpesviridae, Orthomyxoviridae, Poxviridae, Rhabdoviridae, Retroviridae, Togaviridae,* or *Herpesviridae.* For example, the virus may be at least one virus selected from the group consisting of Sin Nombre Hantavirus which belongs to the *Bunyaviridae* family; coronavirus which belongs to the *Coronaviridae* family and is involved in various acute respiratory syndromes; Ebola virus and Marburg virus which belong to the *Filoviridae* family; West Nile virus, yellow fever virus, dengue fever virus, and hepatitis C virus which belong to the *Flaviviridae* family; hepatitis B virus which belongs to the *Hepadnaviridae* family; herpes simplex 1 virus and herpes simplex 2 virus which belong to the *Herpesviridae* family; influenza virus which belongs to the *Orthomyxoviridae* family; smallpox virus, vaccinia virus, molluscum contagiosum virus, and monkeypox virus which belong to the *Poxviridae* family; rabies virus which belongs to the *Rhabdoviridae* family; human Immunodeficiency virus (HIV) which belongs to the *Retroviridae* family; chikungunya virus which belongs to the *Togaviridae* family; pseudorabies virus and human herpes virus (HHV) which belong to the family of *Herpesviridae*; and influenza virus which belongs to the *Orthomyxoviridae* family.

In certain embodiments, the virus may be dengue virus or SARS-CoV-2 virus.

In certain embodiments, the target disease may be a respiratory disease caused by a viral infection or a coronavirus disease 2019 (COVID-19).

### 4.3 Pharmaceutical composition including the antiviral peptide

### 4.3.1 Overview of pharmaceutical composition

According to one aspect of the present disclosure, a pharmaceutical composition for treating or preventing a viral disease is disclosed. The pharmaceutical composition includes a therapeutically effective amount of the antiviral peptide. In some examples, the pharmaceutical composition may further include a pharmaceutically acceptable carrier and/or adjuvant. Here, the pharmaceutically acceptable carrier may be for appropriately formulating the pharmaceutical composition for treating or preventing a viral disease.

The antiviral peptide is an antiviral peptide disclosed in detail in section <<1. Antiviral peptide of present disclosure>> of the present specification.

The viral disease refers to a disease caused by infection with a virus disclosed in section <<4.2 Target disease>> of the present disclosure.

### 4.3.2 Examples of pharmaceutical composition formulations

As a method for formulating the pharmaceutical composition of the present disclosure, any known formulation method used for peptides may be used.

As an embodiment, the pharmaceutical composition may be formulated as a troche, a lozenge, a tablet, an aqueous suspension, an oily suspension, a prepared powder, granules, an emulsion, a hard capsule, a soft capsule, a syrup, or an elixir. As another embodiment, the pharmaceutical composition may be formulated as an injectable solution, a suppository, a powder for inhalation, an aerosol for spraying, an ointment, a powder for application, an oil, or a cream. As another embodiment, the pharmaceutical composition may be formulated as an injectable solution. Specifically, a therapeutically effective amount of the antiviral peptide is mixed with a stabilizer or buffer in water to prepare a solution or suspension, and the solution or suspension may be formulated for unit administration in an ampoule or vial. As another embodiment, the pharmaceutical composition may be formulated as an aerosol by mixing a propellant, and the like with additives together to prepare a water-dispersed concentrate or wet powder. As another embodiment, when the pharmaceutical composition is formulated for transdermal use, a therapeutically effective amount of the antiviral peptide may be added to animal fat, vegetable fat, wax, paraffin, starch, tragacanth, cellulose derivatives, polyethylene glycol, silicone, bentonite, silica, talc, zinc oxide, and the like as a carrier to prepare an ointment, cream, application powder, oil, topical skin preparation, and the like.

### 4.3.3 Examples of pharmaceutically acceptable carrier

The pharmaceutically acceptable carrier is typically used in formulation, and includes saline, sterile water, Ringer's solution, buffered saline, cyclodextrin, a dextrose solution, a maltodextrin solution, glycerol, ethanol, liposomes, and the like, but is not limited thereto, and may further include other typical additives such as an antioxidant and a buffer, if necessary. Further, the pharmaceutical composition may be formulated into an injectable formulation, such as an aqueous solution, a suspension, and an emulsion, a pill, a capsule, granules, or a tablet by additionally adding a diluent, a dispersant, a surfactant, a binder, a lubricant, and the like. With regard to suitable pharmaceutically acceptable carriers and formulations, the pharmaceutical composition may preferably be formulated according to each ingredient using the methods disclosed in Remington's Pharmaceutical Sciences (19th edition, 1995). As an embodiment, the pharmaceutical composition may include, as a pharmaceutically acceptable carrier, a binder such as lactose, saccharose, sorbitol, mannitol, starch, amylopectin, cellulose or gelatin; an excipient such as dicalcium phosphate; a disintegrant such as corn starch or sweet potato starch; a lubricant such as magnesium stearate, calcium stearate, sodium stearyl fumarate, or polyethylene glycol wax; a sweetener; a flavoring agent; a syrup; a liquid carrier such as a fatty oil; a sterilized aqueous solution; propylene glycol; polyethylene glycol; an injectable ester such as ethyl oleate; a suspension; an emulsion; a lyophilized preparation; an external preparation; a stabilizer; a buffer; an animal oil; a vegetable oil; wax; paraffin; starch; tragacanth; cellulose derivatives; polyethylene glycol; silicone; bentonite; silica; talc; zinc oxide, or any suitable combination thereof.

### 4.4 Treatment method using antiviral peptide

### 4.4.1 Overview of treatment method

According to one aspect of the present disclosure, a method for treating a viral disease is disclosed. The treatment method includes administering a pharmaceutical composition for treating a viral disease to a subject using a suitable mode of administration, a suitable dosage regimen, and a suitable dose. In this case, the subject of administration may refer to an individual infected with a virus. In this case, the subject of administration may be a human or a non-human animal.

The pharmaceutical composition for treating a viral disease is the pharmaceutical composition disclosed in detail in section <<4.3. Pharmaceutical composition including the antiviral peptide>> of the present disclosure.

The viral disease refers to a disease caused by infection with a virus disclosed in section <<4.2 Target disease>> of the present disclosure.

### 4.4.2 Examples of administration method

The treatment method of the present disclosure may involve administering a suitable formulation of a therapeutically effective antiviral peptide to a subject using a suitable administration method. In an embodiment, the treatment method may be achieved by administering a suitable formulation of a therapeutically effective antiviral peptide by one of the following methods: oral administration, parenteral administration, intravenous administration, intraperitoneal administration, intramuscular administration, transdermal administration, and subcutaneous administration.

### 4.4.3 Examples of dose

The treatment method of the present disclosure may involve administering a suitable formulation of a therapeutically effective antiviral peptide to a subject using a suitable administration method. As an embodiment, the dose may be about 0.01 mg to 1000 mg of the antiviral peptide per kg of the subject's body weight.

### 4.4.4 Examples of administration period

The treatment method of the present disclosure may involve administering a suitable formulation of a therapeutically effective antiviral peptide to a subject using a suitable administration method. As an embodiment, the pharmaceutical composition including an appropriate dose of the antiviral peptide may be administered once a day. As another embodiment, the administration period may be one in which the pharmaceutical composition including an appropriate dose of the antiviral peptide is administered at least twice a day. As another embodiment, the administration period may be one in which the pharmaceutical composition including an appropriate dose of the antiviral peptide is administered at intervals of 1 hour, 2 hours, 6 hours, 12 hours, 24 hours, 2 days, 3 days, 1 week, 2 weeks, 1 month, and/or 3 months.

### 4.5 Therapeutic use of antiviral peptide

According to one aspect of the present disclosure, an use of the antiviral peptide for treating a viral disease is disclosed. In this case, the antiviral peptide refers to the antiviral peptide disclosed by the present disclosure. In this case, the viral disease refers to a disease caused by infection with a virus disclosed in section <<4.2 Target disease>> of the present disclosure.

### 4.6 Use of the antiviral peptide in manufacture of therapeutic agent

According to one aspect of the present disclosure, an use of the antiviral peptide for the manufacture of a medicament for treating a viral disease is disclosed. In this case, the antiviral peptide refers to the antiviral peptide disclosed by the present disclosure. In this case, the viral disease refers to a disease caused by infection with a virus disclosed in section <<4.2 Target disease>> of the present disclosure.

### Possible embodiments of invention

Hereinafter, possible embodiments of the invention provided by the present specification will be listed. The following embodiments provided in this section are merely examples of the invention. Therefore, the invention provided by the present specification should not be construed as being limited to the following examples. The brief description described with the example numbers is also for the convenience of distinguishing the examples, and cannot be construed as a limitation on the invention disclosed in the present specification.

### Antiviral peptide

### Embodiment 1. Antiviral Peptide 1

An antiviral peptide, which is represented by an amino acid sequence of any one of the following amino acid sequences or an amino acid sequence having 83, 84, 85, 86, 87, 88, 89, 90, 91, 92, 93, 94, 95, 96, 97, 98, or 99% or more homology (sequence identity) thereto:
SEQ ID NO: 1, SEQ ID NOs: 3 to 5, SEQ ID NOs: 8 to 23, SEQ ID NO: 26, SEQ ID NO: 29, SEQ ID NO: 30, SEQ ID NO: 33, SEQ ID NO: 35, SEQ ID NO: 36, SEQ ID NOs: 38 to 40, SEQ ID NO: 43, SEQ ID NOs: 45 to 52, SEQ ID NO: 55, SEQ ID NO: 58, SEQ ID NO: 59, SEQ ID NO: 62, SEQ ID NOs: 64 to 73, SEQ ID NO: 75, SEQ ID NOs: 78 to 87, SEQ ID NOs: 90 to 93, SEQ ID NOs: 97 to 102, SEQ ID NOs: 104 to 106, SEQ ID NOs: 109 to 112, SEQ ID NOs: 114 to 116, SEQ ID NOs: 118 to 123, SEQ ID NO: 126, SEQ ID NO: 127, SEQ ID NOs: 130 to 132, SEQ ID NO: 136, SEQ ID NOs: 138 to 147, SEQ ID NOs: 151 to 155, SEQ ID NO: 162, SEQ ID NO: 164, SEQ ID NOs: 166 to 168, SEQ ID NOs: 170 to 174, SEQ ID NO: 176, SEQ ID NOs: 180 to 182, SEQ ID NOs: 184 to 186, SEQ ID NO: 188, SEQ ID NO: 189, SEQ ID NO: 191, SEQ ID NO: 192, SEQ ID NOs: 194 to 196, SEQ ID NO: 198, SEQ ID NO: 201, SEQ ID NO: 204, SEQ ID NO: 206, SEQ ID NOs: 208 to 211, SEQ ID NO: 215, SEQ ID NO: 218, SEQ ID NO: 219, SEQ ID NO: 221, SEQ ID NO: 224, SEQ ID NO: 225, SEQ ID NO: 228, SEQ ID NO: 229, SEQ ID NO: 231, SEQ ID NO: 232, SEQ ID NO: 236, SEQ ID NO: 237, SEQ ID NO: 240, SEQ ID NOs: 242 to 246, SEQ ID NOs: 248 to 254, SEQ ID NOs: 256 to 258, SEQ ID NOs: 260 to 270, SEQ ID NO: 272, SEQ ID NO: 273, SEQ ID NOs: 275 to 288, SEQ ID NO: 290, SEQ ID NO: 291, SEQ ID NOs: 293 to 296, SEQ ID NO: 298 and SEQ ID NO: 299.

### Embodiment 2. Antiviral Peptide 2

The antiviral peptide of Embodiment 1, wherein the amino acid sequence of the antiviral peptide is an amino acid sequence of any one of the following amino acid sequences:
SEQ ID NO: 1, SEQ ID NO: 3, SEQ ID NO: 4, SEQ ID NO: 5, SEQ ID NO: 20, SEQ ID NO: 29, SEQ ID NO: 30, SEQ ID NO: 46, SEQ ID NO: 48, SEQ ID NO: 49, SEQ ID NO: 52, SEQ ID NO: 59, SEQ ID NO: 78, SEQ ID NO: 82, SEQ ID NO: 87, SEQ ID NO: 90 SEQ ID NO: 91, SEQ ID NO: 98, SEQ ID NO: 99, SEQ ID NO: 101, SEQ ID NO: 106, SEQ ID NO: 110, SEQ ID NO: 112, SEQ ID NO: 114, SEQ ID NO: 115, SEQ ID NO: 116, SEQ ID NO: 123, SEQ ID NO: 126, SEQ ID NO: 131, SEQ ID NO: 132, SEQ ID NO: 139, SEQ ID NO: 141, SEQ ID NO: 143, SEQ ID NO: 144, SEQ ID NO: 151, SEQ ID NO: 162, SEQ ID NO: 167, SEQ ID NO: 171, SEQ ID NO: 189, SEQ ID NO: 221, SEQ ID NO: 228, SEQ ID NO: 242, SEQ ID NO: 245, SEQ ID NO: 246, SEQ ID NO: 254, SEQ ID NO: 256, SEQ ID NO: 264, SEQ ID NO: 283, SEQ ID NO: 290 and SEQ ID NO: 296.

### Embodiment 3. Antiviral Peptide 3

The antiviral peptide of Embodiment 1, wherein the amino acid sequence of the antiviral peptide is an amino acid sequence of any one of the following amino acid sequences:
SEQ ID NO: 1, SEQ ID NO: 3, SEQ ID NO: 4, SEQ ID NO: 20, SEQ ID NO: 46, SEQ ID NO: 48, SEQ ID NO: 49, SEQ ID NO: 59, SEQ ID NO: 87, SEQ ID NO: 90, SEQ ID NO: 91, SEQ ID NO: 98, SEQ ID NO: 101, SEQ ID NO: 110, SEQ ID NO: 114, SEQ ID NO: 116, SEQ ID NO: 126, SEQ ID NO: 132, SEQ ID NO: 141, SEQ ID NO: 143, SEQ ID NO: 144, SEQ ID NO: 151, SEQ ID NO: 221, SEQ ID NO: 246, SEQ ID NO: 256, SEQ ID NO: 264, SEQ ID NO: 283, SEQ ID NO: 290 and SEQ ID NO: 296.

### Embodiment 4. Antiviral Peptide 4

The antiviral peptide of Embodiment 1, wherein the amino acid sequence of the antiviral peptide is an amino acid sequence of any one of the following amino acid sequences:
SEQ ID NO: 1, SEQ ID NO: 3, SEQ ID NO: 4, SEQ ID NO: 46, SEQ ID NO: 49, SEQ ID NO: 101, SEQ ID NO: 143, SEQ ID NO: 185 and SEQ ID NO: 191.

### Embodiment 5. Antiviral Peptide 5

The antiviral peptide of Embodiment 1, wherein the amino acid sequence of the antiviral peptide is an amino acid sequence of any one of the following amino acid sequences:
SEQ ID NO: 3, SEQ ID NO: 4, SEQ ID NO: 46, SEQ ID NO: 49, SEQ ID NO: 101, SEQ ID NO: 110, SEQ ID NO: 114, SEQ ID NO: 116, SEQ ID NO: 143, SEQ ID NO: 151, SEQ ID NO: 171 and SEQ ID NO: 221.

### Structure and characteristics of antiviral peptide

### Embodiment 6. Structure of antiviral peptide 1

The antiviral peptide of any one of Embodiments 1 to 5, wherein the antiviral peptide is capable of forming an α-helix structure.

### Embodiment 7. Structure of antiviral peptide 2

The antiviral peptide of any one of Embodiments 1 to 6, wherein the antiviral peptide is capable of forming an α-helix structure when the antiviral peptide reacts with a lipid membrane.

### Embodiment 8. Characteristics of antiviral peptide 1

The antiviral peptide of any one of Embodiments 1 to 7, wherein the antiviral peptide is an amphipathic peptide.

### Embodiment 9. Characteristics of antiviral peptide 2

The antiviral peptide of any one of Embodiments 1 to 8, wherein the antiviral peptide has amphipathic characteristics when the antiviral peptide forms an α-helix structure.

### Effect of antiviral peptide

### Embodiment 10. Effect of antiviral peptide 1

The antiviral peptide of any one of Embodiments 1 to 9, wherein the antiviral peptide is capable of causing damage to an outer membrane (or a lipid bilayer) of a virus.

### Embodiment 11. Effect of antiviral peptide 2

The antiviral peptide of any one of Embodiments 1 to 10, wherein the antiviral peptide is capable of causing destruction of an outer membrane (or a lipid bilayer) of a virus.

### Embodiment 12. Effect of antiviral peptide 3

The antiviral peptide of any one of Embodiments 1 to 11, wherein the antiviral peptide is capable of causing the virus's ability to infect cells to be reduced or inactivated.

### Embodiment 13. Effect of antiviral peptide 4

The antiviral peptide of any one of Embodiments 1 to 12, wherein the antiviral peptide is capable of causing the virus's ability to proliferate (or replicate) to be reduced or inactivated.

### Embodiment 14. Effect of antiviral peptide 5

The antiviral peptide of any one of Embodiments 10 to 13, wherein the virus belongs to a family of Bunyaviridae, Coronaviridae, Filoviridae, Flaviviridae, Hepadnaviridae, Herpesviridae, Orthomyxoviridae, Poxviridae, Rhabdoviridae, Retroviridae, Togaviridae, or Herpesviridae.

### Use of antiviral peptide

### Embodiment 15. Pharmaceutical composition including antiviral peptide

A pharmaceutical composition for treating a viral disease, including a therapeutically effective amount of an antiviral peptide,
wherein the antiviral peptide is the antiviral peptide of any one of Embodiments 1 to 14, and
the viral disease is caused by a viral infection.

### Embodiment 16. Pharmaceutical composition including antiviral peptide

The pharmaceutical composition of Embodiment 15, wherein the pharmaceutical composition further comprises a pharmaceutically acceptable carrier and/or pharmaceutically acceptable adjuvant.

### Embodiment 17. Pharmaceutical composition including antiviral peptide

The pharmaceutical composition of any one of Embodiments 15 and 16, wherein the viral disease is caused by infection with a virus belonging to a family of Bunyaviridae, Coronaviridae, Filoviridae, Flaviviridae, Hepadnaviridae, Herpesviridae, Orthomyxoviridae, Poxviridae, Rhabdoviridae, Retroviridae, Togaviridae, or Herpesviridae.

### Embodiment 18. Treatment method using antiviral peptide

A method for treating a viral disease, including:
administering a pharmaceutical composition including a therapeutically effective amount of an antiviral peptide to a subject infected with a virus,
wherein the antiviral peptide is the antiviral peptide of any one of Embodiments 1 to 14.

### Embodiment 19. Therapeutic use of antiviral peptide

A use of an antiviral peptide of any one of Embodiments 1 to 14 for treating a viral disease.

### Embodiment 20. Use of antiviral peptide in manufacture of therapeutic agent

A use of an antiviral peptide of any one of Embodiments 1 to 14 for the manufacture of a medicament for treating a viral disease.

### Method for selecting antiviral peptides

### Embodiment 21. Method for selecting antiviral peptides 1

A method for screening an antiviral peptide candidate group, including selecting, finding, and/or searching for peptides that satisfy at least one of the following conditions:
1) the peptide consists of 18 amino acid residues;
2) the peptide does not comprise proline residues;
3) the peptide comprises at least six polar amino acid residues;
4) the peptide comprises from four to six charged amino acid residues;
5) the peptide has a hydrophobic moment (µH) value of 0.5 to 0.8, as measured by HeliQuest software;
6) the peptide has a hydrophobicity (H) value of 0.7 to 0.9, as measured by HeliQuest software; and
7) the peptide has a net charge ranging from -2 to 0, as measured by HeliQuest software.

### Embodiment 22. Method for selecting antiviral peptides 2

The method of Embodiment 21, further including a process of verifying the antiviral efficacy of the selected peptides.

### Embodiment 23. Method for selecting antiviral peptides 3

The method of Embodiment 22, wherein the verifying of the antiviral efficacy of selected peptides comprises at least one selected from the following:
mixing liposomes with the selected peptide and confirming that the liposomes are damaged or destroyed; or
mixing a virus with the selected peptide and confirming that the viral phospholipid bilayer is damaged or destroyed, or confirming that the virus's ability to infect cells is reduced or inactivated.

### Embodiment 24. Method for selecting antiviral peptides 4

The method of Embodiment 23, wherein the size of the liposome ranges from 5 nm to 1500 nm.

Hereinafter, the invention provided by the present specification will be described in more detail with reference to the following experimental examples. These experimental examples are only for exemplifying the content disclosed by the present specification, and it will be obvious to those skilled in the art that the scope of the content disclosed by the present specification should not be interpreted as being limited by these experimental examples.

### Experimental Example 1. Selection of peptide according to an embodiment of present disclosure

The present inventors selected, among peptides disclosed in Swiss-Prot sequence data, 300 peptides, which 1) consist of 18 amino acid residues, 2) do not comprise proline residues among the 18 amino acid residues, 3) include at least six polar amino acid residues among the 18 amino acid residues, 4) comprise from four to six charged amino acid residues among the 18 amino acid residues, 5) have a hydrophobic moment (µH) value of 0.5 to 0.8, as measured by HeliQuest software, 6) have a hydrophobicity (H) value of 0.7 to 0.9, as measured by HeliQuest software; and 7) have a net charge ranging from -2 to 0, as measured by HeliQuest software. In this case, the polar amino acid residues consist of aspartic acid (Asp, D), glutamic acid (Glu, E), lysine (Lys, K), arginine (Arg, R), histidine (His, H), serine (Ser, S), threonine (Thr, T), asparagine (Asn, N), glutamine (Gln, Q) and glycine (Gly, G), and the charged amino acid residues consist of aspartic acid (Asp, D), glutamic acid (Glu, E), lysine (Lys, K) and arginine (Arg, R).

Information on the amino acid sequences, hydrophobicity values, and hydrophobic moment values of the 300 selected peptides (SP-001 to SP-300) is disclosed in the following Table 1.

**[Table 1]**

| Peptide | Amino acid sequence | SEQ ID NO | Hydrophobicity (HeliQuest) | Hydrophobic moment (HeliQuest) |
|---|---|---|---|---|
| SP-001 | ESLKNFWESLWKFFTHFF | 1 | 0.739 | 0.733 |
| SP-002 | ITKFYKSFQDLWDMVIHI | 2 | 0.719 | 0.661 |
| SP-003 | SFLDIVWSIIDIFKEFCK | 3 | 0.836 | 0.632 |
| SP-004 | QSFLDIVWSIIDIFKEFC | 4 | 0.878 | 0.595 |
| SP-005 | QDLCLRIFSSLVRILDLF | 5 | 0.81 | 0.599 |
| SP-006 | LHGLIDGLEYWIRGYLDW | 6 | 0.764 | 0.625 |
| SP-007 | LIDGLEYWIRGYLDWVVH | 7 | 0.806 | 0.558 |
| SP-008 | NFIDKTFTVLADIILKIL | 8 | 0.767 | 0.581 |
| SP-009 | NFIDKTFTIIADILLRII | 9 | 0.804 | 0.573 |
| SP-010 | VFQWLWDWVKKIFNWLED | 10 | 0.847 | 0.695 |
| SP-011 | LWDWVKKIFNWLEDLFLN | 11 | 0.822 | 0.658 |
| SP-012 | WVKKIFNWLEDLFLNFTG | 12 | 0.759 | 0.626 |
| SP-013 | VVIYGIIGFLDDFLKIFK | 13 | 0.881 | 0.564 |
| SP-014 | FFLRWLYSVSDYLSDFWE | 14 | 0.822 | 0.624 |
| SP-015 | RWLYSVSDYLSDFWESLL | 15 | 0.716 | 0.647 |
| SP-016 | DALWRLWLGFFREHFAAI | 16 | 0.8 | 0.582 |
| SP-017 | SEWTGILDRLLKWVGLLV | 17 | 0.781 | 0.596 |
| SP-018 | TGILDRLLKWVGLLVDIV | 18 | 0.818 | 0.588 |
| SP-019 | VELIDDLYRKLYSWFLHI | 19 | 0.749 | 0.619 |
| SP-020 | IDDLYRKLYSWFLHILTF | 20 | 0.831 | 0.577 |
| SP-021 | MGIFQAFADVFKLLFKEI | 21 | 0.757 | 0.602 |
| SP-022 | AHFGFFWRWFDEIFTLLD | 22 | 0.898 | 0.569 |
| SP-023 | WEALKYWWNLLQYWSKEL | 23 | 0.773 | 0.598 |
| SP-024 | FQFIQKLIDDLLHVIEGL | 24 | 0.751 | 0.674 |
| SP-025 | IQKLIDDLLHVIEGLVLI | 25 | 0.827 | 0.558 |
| SP-026 | FDFIEKFSHVLFDIVGII | 26 | 0.857 | 0.569 |
| SP-027 | IEKFSHVLFDIVGIIMKV | 27 | 0.782 | 0.57 |
| SP-028 | FDFIKSIIIGIVEGLTEF | 28 | 0.804 | 0.561 |
| SP-029 | IDILIRFYHLFESIYKYI | 29 | 0.863 | 0.569 |
| SP-030 | LIRFYHLFESIYKYIMDL | 30 | 0.826 | 0.57 |
| SP-031 | QIGKDISNLLFDIWRVWL | 31 | 0.756 | 0.629 |
| SP-032 | ISNLLFDIWRVWLEKLGE | 32 | 0.734 | 0.638 |
| SP-033 | LLSMFLVMIQWWRDIVRE | 33 | 0.9 | 0.554 |
| SP-034 | ILDTSVQFWYEFIDYIMR | 34 | 0.784 | 0.606 |
| SP-035 | TSVQFWYEFIDYIMRVIE | 35 | 0.764 | 0.593 |
| SP-036 | EILGAFFHKFEKFFDLFM | 36 | 0.754 | 0.641 |
| SP-037 | FGLVKLWVEKIVSFIEQF | 37 | 0.82 | 0.572 |
| SP-038 | VKLWVEKIVSFIEQFLNF | 38 | 0.787 | 0.606 |
| SP-039 | LFYLLSRLWFGLLETFRD | 39 | 0.865 | 0.574 |
| SP-040 | LLSRLWFGLLETFRDILV | 40 | 0.88 | 0.562 |
| SP-041 | RFVQGVDMFLETLWKVWM | 41 | 0.791 | 0.579 |
| SP-042 | QGVDMFLETLWKVWMELL | 42 | 0.833 | 0.573 |
| SP-043 | WSEIFRELFSLLGRIWSS | 43 | 0.74 | 0.747 |
| SP-044 | LALEKYWIEWLEKWQMLL | 44 | 0.857 | 0.533 |
| SP-045 | FFHLWLNILAELLRFGDR | 45 | 0.796 | 0.613 |
| SP-046 | ILDFFVGFIDWLVRYLNK | 46 | 0.866 | 0.665 |
| SP-047 | MDFIRAIILGIIEGITEW | 47 | 0.849 | 0.562 |
| SP-048 | DHWHNLFIKLFKYYYDII | 48 | 0.758 | 0.659 |
| SP-049 | YNLFDLLIYIWNKIFDDY | 49 | 0.817 | 0.605 |
| SP-050 | DIFQDLLKEVIFVLSKIL | 50 | 0.767 | 0.595 |
| SP-051 | LEILVQILRYWIKDVSFF | 51 | 0.892 | 0.548 |
| SP-052 | LHIISYMGDIFKLIKEFW | 52 | 0.851 | 0.594 |
| SP-053 | EVWIEGLRSYLLDWWNFL | 53 | 0.868 | 0.578 |
| SP-054 | LNFIGVLVDSLKEILNEW | 54 | 0.7 | 0.689 |
| SP-055 | GGFWDSIKALWRNLIDFL | 55 | 0.717 | 0.731 |
| SP-056 | NFIYKELEQIIDLFMVLI | 56 | 0.857 | 0.552 |
| SP-057 | FFDSIRILWTKIINGLVD | 57 | 0.763 | 0.595 |
| SP-058 | MLMEQFLDYIGKLLFKIF | 58 | 0.866 | 0.528 |
| SP-059 | EQFLDYIGKLLFKIFMTI | 59 | 0.817 | 0.557 |
| SP-060 | KSLNFVLAAWIRILEEIL | 60 | 0.787 | 0.615 |
| SP-061 | ETFKEFVHVWFKSIFNWI | 61 | 0.788 | 0.642 |
| SP-062 | FDFFKIVGEIFGLFENYF | 62 | 0.81 | 0.57 |
| SP-063 | TIVSRVLGKVLDFWVDFI | 63 | 0.799 | 0.614 |
| SP-064 | TTLMKFVELWFKDVFNWM | 64 | 0.817 | 0.568 |
| SP-065 | QWFDLLKDIFSKFTIFLQ | 65 | 0.798 | 0.621 |
| SP-066 | LLKELLDLLQDMVVMLLS | 66 | 0.837 | 0.565 |
| SP-067 | AIFTFLHKLVEVLSDYLK | 67 | 0.714 | 0.64 |
| SP-068 | AIMKKIIDFYGQWVDSFI | 68 | 0.721 | 0.639 |
| SP-069 | ANINDILMKIIKIFDAIL | 69 | 0.729 | 0.645 |
| SP-070 | AVTWDILLDIIERLLQQL | 70 | 0.795 | 0.567 |
| SP-071 | WDILLDIIERLLQQLQNL | 71 | 0.744 | 0.712 |
| SP-072 | LVLYGLIGFLDDFLKVFR | 72 | 0.863 | 0.531 |
| SP-073 | IDFFDKVSHVFFKIIGYI | 73 | 0.796 | 0.598 |
| SP-074 | IIELIKGIILGIVEGLTE | 74 | 0.804 | 0.557 |
| SP-075 | HEVLDFLDNLMISIFKIL | 75 | 0.808 | 0.577 |
| SP-076 | SIWQLVLEQFDDLLVKIL | 76 | 0.829 | 0.558 |
| SP-077 | DIIDRFVDWITMTFGGFF | 77 | 0.803 | 0.583 |
| SP-078 | YEVYRVFSDILHIFLKLI | 78 | 0.84 | 0.612 |
| SP-079 | VVGLIIKWFDWIYELGKQ | 79 | 0.827 | 0.566 |
| SP-080 | DMLGSLYKILENILIIFR | 80 | 0.774 | 0.589 |
| SP-081 | KKLVEYILDIFLKLSEIL | 81 | 0.712 | 0.635 |
| SP-082 | MTVISLFKNLIEIFEKYL | 82 | 0.769 | 0.585 |
| SP-083 | VDLFSDFVTLFRKLMMIL | 83 | 0.864 | 0.565 |
| SP-084 | QDLKEIISYIFDIIKIII | 84 | 0.802 | 0.623 |
| SP-085 | DLLIDILKYLVRLTEDII | 85 | 0.733 | 0.657 |
| SP-086 | DSIIDLFKDFMLTFRQFF | 86 | 0.715 | 0.632 |
| SP-087 | FWKDIINLIKGFILSIID | 87 | 0.882 | 0.664 |
| SP-088 | MEFFQEYYDIIRLWSTIL | 88 | 0.818 | 0.554 |
| SP-089 | DVLVKAWAMWGDYLENIF | 89 | 0.721 | 0.648 |
| SP-090 | KFLKAVFELWEFIKDFLV | 90 | 0.78 | 0.623 |
| SP-091 | LKEWLDYYNWLSRLIEFF | 91 | 0.773 | 0.588 |
| SP-092 | TEFIKMFKSFVDLLFEVV | 92 | 0.747 | 0.637 |
| SP-093 | VLILKTIRELTSFFIDLL | 93 | 0.876 | 0.526 |
| SP-094 | EILDFFMTLFGSILTDIR | 94 | 0.799 | 0.586 |
| SP-095 | FTTILSDFQSFLERLLDI | 95 | 0.711 | 0.657 |
| SP-096 | TEILKQFLTHFDTIFEVF | 96 | 0.724 | 0.651 |
| SP-097 | ELIDELYQKVYRWFMQIF | 97 | 0.706 | 0.639 |
| SP-098 | LEQLMYLIEEFVKYLIRF | 98 | 0.789 | 0.641 |
| SP-099 | SELMRWIISEIYAVWKIF | 99 | 0.864 | 0.527 |
| SP-100 | IKFLKEFIEFLREYINVI | 100 | 0.702 | 0.735 |
| SP-101 | SWKEIVNLLYELLASLIR | 101 | 0.716 | 0.686 |
| SP-102 | YITAILVLWSRMMKEILD | 102 | 0.806 | 0.604 |
| SP-103 | IAFGIIGFLDDILKIIHR | 103 | 0.816 | 0.576 |
| SP-104 | FWRRHVFIWISFIDSYFE | 104 | 0.881 | 0.51 |
| SP-105 | GKIFKVLYDSFMLLWNEW | 105 | 0.798 | 0.611 |
| SP-106 | GWFGFISDAMELVLKILK | 106 | 0.77 | 0.612 |
| SP-107 | KVIFDVIESFSKVIFGVI | 107 | 0.777 | 0.612 |
| SP-108 | VILDLFKEIIDEVLKGII | 108 | 0.752 | 0.594 |
| SP-109 | FLDALLKILEGWLYTYAK | 109 | 0.764 | 0.626 |
| SP-110 | WSEILREFLNLISLIWSR | 110 | 0.804 | 0.675 |
| SP-111 | IFLYLVDQIYDIIRMIEK | 111 | 0.787 | 0.597 |
| SP-112 | YIYLILKFIQDIYNDIYR | 112 | 0.759 | 0.62 |
| SP-113 | FQFIEKFSEIMFELVRMI | 113 | 0.764 | 0.61 |
| SP-114 | FSFWVWLDNIIDLVKKYI | 114 | 0.896 | 0.515 |
| SP-115 | YTSFVDVFFKIVDYLNKW | 115 | 0.711 | 0.633 |
| SP-116 | FTFWTWLEAILDLIKKHI | 116 | 0.897 | 0.525 |
| SP-117 | MFVETLWKVWTELLDVLG | 117 | 0.859 | 0.617 |
| SP-118 | VLVGMIIKWFDWLYEMGK | 118 | 0.876 | 0.532 |
| SP-119 | GILINVINDFERQWLRIF | 119 | 0.711 | 0.649 |
| SP-120 | GLQNFLEWVVDFIRGIIK | 120 | 0.713 | 0.687 |
| SP-121 | TRIVDWYDLVYGLLDGWW | 121 | 0.831 | 0.577 |
| SP-122 | GLLEIIVILWKNIHRSLE | 122 | 0.76 | 0.596 |
| SP-123 | LIWELIKQILKGISYIHD | 123 | 0.766 | 0.608 |
| SP-124 | YLWLDLRGFWTQIFEEIV | 124 | 0.886 | 0.516 |
| SP-125 | LLKIVSALWEKWLSLLEA | 125 | 0.833 | 0.663 |
| SP-126 | HWWFEGVKWLGRVLELLY | 126 | 0.866 | 0.518 |
| SP-127 | FSELLRLTRTLLGIIDCI | 127 | 0.793 | 0.534 |
| SP-128 | QIIDWYSGLVRRWVIWYE | 128 | 0.807 | 0.555 |
| SP-129 | ECAEKILETWGELLSKWH | 129 | 0.503 | 0.712 |
| SP-130 | IRTLFDEITRILNHLLWL | 130 | 0.809 | 0.571 |
| SP-131 | TLYAFFLTLIREIVKGIE | 131 | 0.767 | 0.581 |
| SP-132 | WKEWWKVFYTIVDYINQI | 132 | 0.797 | 0.727 |
| SP-133 | WKKWWQVFYTVVDYVDQI | 133 | 0.747 | 0.638 |
| SP-134 | VKLGSIYFSFFDDFFRII | 134 | 0.812 | 0.562 |
| SP-135 | KLMVDFFNILNEIVMKLV | 135 | 0.767 | 0.586 |
| SP-136 | YKWFAFFKDVTDFISHLF | 136 | 0.779 | 0.585 |
| SP-137 | LDAIGDLLLDFLLLLNKW | 137 | 0.881 | 0.572 |
| SP-138 | LTLLLLTMVQWWRDIIRE | 138 | 0.884 | 0.517 |
| SP-139 | TLLRAVETLFSLWLEFMR | 139 | 0.793 | 0.623 |
| SP-140 | LRLWEITARYVEGIVHLF | 140 | 0.838 | 0.612 |
| SP-141 | WLDNIIDLVKKYILALWN | 141 | 0.804 | 0.607 |
| SP-142 | WFEALLGYISAVIEYFEK | 142 | 0.758 | 0.643 |
| SP-143 | DFWSKLVYFFAEIIRFLS | 143 | 0.856 | 0.649 |
| SP-144 | AKNIIKLLLELIEVILNV | 144 | 0.777 | 0.625 |
| SP-145 | LEEILEVIISLFKKLNLI | 145 | 0.787 | 0.573 |
| SP-146 | LLVKILKLIINELSNVME | 146 | 0.726 | 0.609 |
| SP-147 | ALLIYFRRDWVDLIGGWL | 147 | 0.868 | 0.517 |
| SP-148 | IQWFEVLDGLLGRYWKAL | 148 | 0.764 | 0.608 |
| SP-149 | ATWQDWFHRWLEILDSSL | 149 | 0.703 | 0.655 |
| SP-150 | LWKSLDSWASLILQWFED | 150 | 0.774 | 0.649 |
| SP-151 | DVTRLLELILDVLAYIIS | 151 | 0.813 | 0.558 |
| SP-152 | DEFLDFAALLFGWFRKFV | 152 | 0.776 | 0.577 |
| SP-153 | DEILGRLLKILFVLFVDL | 153 | 0.869 | 0.53 |
| SP-154 | FRMFIGDLEKIVNLLLSL | 154 | 0.782 | 0.59 |
| SP-155 | ILLKFLESVGGVLLDILR | 155 | 0.81 | 0.589 |
| SP-156 | LIVILDDSFLELLRLFIK | 156 | 0.899 | 0.51 |
| SP-157 | LLFGLLGFLDDYLKVVLR | 157 | 0.852 | 0.55 |
| SP-158 | LFYGFIGFLDDFVSIVKK | 158 | 0.778 | 0.593 |
| SP-159 | IEIMDFVIGVLDSIAKIF | 159 | 0.836 | 0.564 |
| SP-160 | IEQLVEYTKIILDFIISW | 160 | 0.866 | 0.543 |
| SP-161 | KIFIGLLELIEDIFHGTF | 161 | 0.834 | 0.55 |
| SP-162 | FRYLIEAINSFVKVFELW | 162 | 0.801 | 0.622 |
| SP-163 | INLLLKIVFELSKLIDDF | 163 | 0.772 | 0.623 |
| SP-164 | NFIDRVFTVISDLILKLL | 164 | 0.794 | 0.615 |
| SP-165 | SIFELILNQFDDLLVKIL | 165 | 0.815 | 0.569 |
| SP-166 | DIIKDFAKFFTIFFGITF | 166 | 0.847 | 0.528 |
| SP-167 | WRGMLIKILSFIEGYIDF | 167 | 0.843 | 0.549 |
| SP-168 | LKDWLGFWLSLYQRFIEN | 168 | 0.743 | 0.659 |
| SP-169 | NDILKVLSEWLTDLFTII | 169 | 0.787 | 0.692 |
| SP-170 | DIWVNVLVSFITLLREWR | 170 | 0.824 | 0.565 |
| SP-171 | DKIVLFILSILEKIYNLL | 171 | 0.869 | 0.573 |
| SP-172 | EDILFSILYLLKKIANLI | 172 | 0.818 | 0.546 |
| SP-173 | ILADILVKLLDEIKYILK | 173 | 0.724 | 0.612 |
| SP-174 | LLEYIDLVYNILISILKK | 174 | 0.823 | 0.562 |
| SP-175 | MLESLINKLITLIDFIVD | 175 | 0.816 | 0.602 |
| SP-176 | DLIHIILDKIIDTLVHFM | 176 | 0.864 | 0.528 |
| SP-177 | LRLLSNYFESMIDIFVKL | 177 | 0.739 | 0.657 |
| SP-178 | DVLLRVFVEGWTHLVRLL | 178 | 0.799 | 0.594 |
| SP-179 | DVMADQFVTFLEKWFKLF | 179 | 0.704 | 0.626 |
| SP-180 | DWQALWEELKSIFVFWIN | 180 | 0.831 | 0.58 |
| SP-181 | ESIWEFIKSLLDAWSGWV | 181 | 0.773 | 0.726 |
| SP-182 | FWEIFVVWLKQFIDIENS | 182 | 0.862 | 0.544 |
| SP-183 | WFIKKLNTWLDVILDEWK | 183 | 0.721 | 0.613 |
| SP-184 | EALMLIYKLFWDEFSSWL | 184 | 0.892 | 0.501 |
| SP-185 | WDCVQDIWEYIFRTAMKI | 185 | 0.712 | 0.615 |
| SP-186 | EEIAVILRIMEKLWHVFM | 186 | 0.792 | 0.566 |
| SP-187 | EDVSEVLAHIGTCLRKIF | 187 | 0.521 | 0.612 |
| SP-188 | EFWNRWHMSLSFWFRDFV | 188 | 0.782 | 0.593 |
| SP-189 | EGILSFILISLKDFLRVF | 189 | 0.85 | 0.523 |
| SP-190 | IVHLLNELFTKFDRLIGI | 190 | 0.743 | 0.604 |
| SP-191 | FTNFHRQLFCWIDKWIDL | 191 | 0.802 | 0.637 |
| SP-192 | LWEAIETFIGEIIAIFYK | 192 | 0.859 | 0.626 |
| SP-193 | WEKTLSLIGEVIEIWMLV | 193 | 0.888 | 0.501 |
| SP-194 | EILREFMTLISHIWSQIK | 194 | 0.704 | 0.668 |
| SP-195 | QDILSKLYLLLARVFEIW | 195 | 0.831 | 0.581 |
| SP-196 | SFIRKGIEAWFTLLMELI | 196 | 0.823 | 0.582 |
| SP-197 | EMFALIFEYIDRIFSIVR | 197 | 0.771 | 0.603 |
| SP-198 | ENWKDFFLIFWQKYFNVW | 198 | 0.821 | 0.545 |
| SP-199 | LNKYFFEWFQAIKGFVEW | 199 | 0.754 | 0.59 |
| SP-200 | FGLLQSFADFLKYIVKEI | 200 | 0.717 | 0.619 |
| SP-201 | KIMFTFVKELLNYLDGFI | 201 | 0.764 | 0.619 |
| SP-202 | ETLVRLFRFFLGEWYAVL | 202 | 0.838 | 0.578 |
| SP-203 | EVILASWYRIYTKIMDLI | 203 | 0.797 | 0.609 |
| SP-204 | IVLEIIHHFVEILDRYFG | 204 | 0.821 | 0.587 |
| SP-205 | KIVIGLLEFVEDIFHGLY | 205 | 0.809 | 0.563 |
| SP-206 | EYIDNIIEYIINILKLTI | 206 | 0.774 | 0.599 |
| SP-207 | IIIITDLLENLKELIIKI | 207 | 0.835 | 0.536 |
| SP-208 | FAKDIWRIILTAWFAGLAD | 208 | 0.724 | 0.645 |
| SP-209 | LSVAGWLDTIWDAIKRFF | 209 | 0.756 | 0.594 |
| SP-210 | FAKDIWRILVAWTTTLWD | 210 | 0.812 | 0.56 |
| SP-211 | KDIITVIAKYFGFIIEFL | 211 | 0.857 | 0.623 |
| SP-212 | FEFLSRMVDSLVSFLSEF | 212 | 0.706 | 0.658 |
| SP-213 | FEIIHGLLDRIMQLLDVL | 213 | 0.826 | 0.638 |
| SP-214 | MLGLMDELIQMIDALVRI | 214 | 0.778 | 0.581 |
| SP-215 | FFDFFVEDFSRWYIQIIR | 215 | 0.796 | 0.563 |
| SP-216 | QLLLRALDAVATCWEKLF | 216 | 0.714 | 0.598 |
| SP-217 | ILEWFTNYIRSWEEVFKL | 217 | 0.72 | 0.7 |
| SP-218 | ILVSQWVEYLKIFENFFK | 218 | 0.772 | 0.593 |
| SP-219 | FFEKIMSHIKNLLEWLGV | 219 | 0.734 | 0.607 |
| SP-220 | LQMLGDFERAWSLILEFI | 220 | 0.803 | 0.559 |
| SP-221 | ILVRWLSHFFEYLDRFYT | 221 | 0.81 | 0.65 |
| SP-222 | FGDLLGEFWLGLKKIFYI | 222 | 0.866 | 0.52 |
| SP-223 | MYAVIGFLDDFLKVFKQI | 223 | 0.754 | 0.595 |
| SP-224 | VWDEFLVLLSKLMEIVNN | 224 | 0.736 | 0.603 |
| SP-225 | MEKILEIIRVILKLYEQW | 225 | 0.713 | 0.685 |
| SP-226 | DKIFTDIFHYLEVLFRII | 226 | 0.798 | 0.633 |
| SP-227 | FLDNIDFLFMFIVKFLDK | 227 | 0.845 | 0.552 |
| SP-228 | FLEDVVIYISRLLRLWIS | 228 | 0.896 | 0.562 |
| SP-229 | INDLFSEILMSFLKFLDL | 229 | 0.825 | 0.578 |
| SP-230 | LKTFISTVNELFEWVIRL | 230 | 0.754 | 0.615 |
| SP-231 | WLSGIYEFFLRYKNIWVE | 231 | 0.794 | 0.563 |
| SP-232 | LKIYYEIMDFIDFVLSKW | 232 | 0.822 | 0.55 |
| SP-233 | FTYIDDLVEGIVKLIGVI | 233 | 0.783 | 0.624 |
| SP-234 | VLVYFWRDYIEIIVKGIT | 234 | 0.854 | 0.521 |
| SP-235 | FVDIVFGLLEGWWRFADG | 235 | 0.813 | 0.591 |
| SP-236 | QLSILFTDWLEWFDKILA | 236 | 0.868 | 0.593 |
| SP-237 | FWMGVIGFLDDYMKVVLK | 237 | 0.811 | 0.56 |
| SP-238 | GEMASQFVTFLEKWFELF | 238 | 0.703 | 0.627 |
| SP-239 | GFFFGWIGLLEGIIREIK | 239 | 0.83 | 0.603 |
| SP-240 | FFSMVRDLLSWMESIIRQ | 240 | 0.708 | 0.63 |
| SP-241 | GIHALWRIFDDWADIFVV | 241 | 0.836 | 0.549 |
| SP-242 | GRWIDMLISRIFDIMLAF | 242 | 0.867 | 0.513 |
| SP-243 | HEVMEFIEKLFLYIFKTI | 243 | 0.782 | 0.571 |
| SP-244 | HLLKDWTDTFVYILEKLI | 244 | 0.728 | 0.623 |
| SP-245 | HTLLDFWRLVWEFGVKVI | 245 | 0.868 | 0.511 |
| SP-246 | HWSKLLEEIQALIKLLLA | 246 | 0.738 | 0.614 |
| SP-247 | IDDILTILLRLQKGLLGL | 247 | 0.767 | 0.581 |
| SP-248 | IEHLEQLFWRIFRNIVLF | 248 | 0.853 | 0.521 |
| SP-249 | IQVFADVFKMLIKEIITI | 249 | 0.828 | 0.579 |
| SP-250 | VAFGIIGFIDDFIKVILK | 250 | 0.85 | 0.552 |
| SP-251 | IIGWIDHFQNWLEKLHKL | 251 | 0.713 | 0.713 |
| SP-252 | VLYKEVLKTIIDLIENII | 252 | 0.729 | 0.608 |
| SP-253 | IIISLLKQLLKTYGDLID | 253 | 0.73 | 0.646 |
| SP-254 | IIYQVIDELVNYFKKFLF | 254 | 0.796 | 0.582 |
| SP-255 | IKIINQWEALLKEFTSLI | 255 | 0.711 | 0.655 |
| SP-256 | VTIYEMVLKSLLEIFKIL | 256 | 0.866 | 0.519 |
| SP-257 | ILKDLLHWFKTQFFTWFD | 257 | 0.859 | 0.521 |
| SP-258 | TIWLGFIGFMDDYIKVFK | 258 | 0.826 | 0.549 |
| SP-259 | INALFDVWIDVQRRWVYL | 259 | 0.769 | 0.588 |
| SP-260 | IWRRWLYVLWDEVAQLVN | 260 | 0.796 | 0.581 |
| SP-261 | LNDIVIIMKSIIYEWFDI | 261 | 0.897 | 0.524 |
| SP-262 | IRVMFDEITRILNHLLGI | 262 | 0.717 | 0.638 |
| SP-263 | TTWWNILERIDHKLFMWI | 263 | 0.845 | 0.531 |
| SP-264 | LSEWLRIIEFLLQGLKVY | 264 | 0.821 | 0.547 |
| SP-265 | WLLLFSEWLGNLQKIFDE | 265 | 0.804 | 0.652 |
| SP-266 | RVILGAIERFIGVLIEHF | 266 | 0.764 | 0.592 |
| SP-267 | KILDQWFFRITAYIDELL | 267 | 0.748 | 0.601 |
| SP-268 | IYIFFRHWTTIYERLLEI | 268 | 0.872 | 0.583 |
| SP-269 | VFWYFWEDLTGIAKGIIK | 269 | 0.808 | 0.551 |
| SP-270 | KEFFTELFILLKSIFSFV | 270 | 0.877 | 0.515 |
| SP-271 | KELWLNIVNIILTLYEDL | 271 | 0.797 | 0.566 |
| SP-272 | KILNQITLFWMDMFKDII | 272 | 0.823 | 0.606 |
| SP-273 | KLFSKLIDILDDYYVLLV | 273 | 0.773 | 0.585 |
| SP-274 | LDKVMKIWTTFVEQIFGV | 274 | 0.718 | 0.651 |
| SP-275 | LDVLSSIGQLIRDILDFI | 275 | 0.744 | 0.664 |
| SP-276 | LEKIYDIIGYIYQYLKLL | 276 | 0.791 | 0.564 |
| SP-277 | LVFFSKVLQYLIEELLEI | 277 | 0.884 | 0.509 |
| SP-278 | LISFIDYISEIIANIIDR | 278 | 0.749 | 0.646 |
| SP-279 | LITMVLTMIQWWRDVIRE | 279 | 0.837 | 0.548 |
| SP-280 | LVMIVATMIQWWRDVIRE | 280 | 0.813 | 0.555 |
| SP-281 | SLLTLILEQFDDLLVKIL | 281 | 0.852 | 0.54 |
| SP-282 | LLKEILNLYLDLGTWLLK | 282 | 0.827 | 0.542 |
| SP-283 | WEKLMLLIIDLFKFLDQY | 283 | 0.874 | 0.547 |
| SP-284 | LQELMKVLIDWINDVLVG | 284 | 0.753 | 0.595 |
| SP-285 | LWLSWEEGLQHFLKYLLD | 285 | 0.793 | 0.594 |
| SP-286 | LWNDIMQFVEQFFWRLVE | 286 | 0.813 | 0.57 |
| SP-287 | LYEFFQAYKTVWTEIWKI | 287 | 0.771 | 0.605 |
| SP-288 | MLIERLIDAINSWVQLIE | 288 | 0.744 | 0.62 |
| SP-289 | QIWNMVIELLDQVVEVFK | 289 | 0.726 | 0.654 |
| SP-290 | YVVTFVELVWNIIEKLLD | 290 | 0.844 | 0.572 |
| SP-291 | REVHEIWASLIEIALSLW | 291 | 0.776 | 0.576 |
| SP-292 | RVIHQFLELWYRYVVEVL | 292 | 0.797 | 0.611 |
| SP-293 | TDIIDIIDQIVILLTKFI | 293 | 0.889 | 0.578 |
| SP-294 | TLTDWKTWISFWKTLIDW | 294 | 0.848 | 0.523 |
| SP-295 | WFEAVLGYISAVVELFRE | 295 | 0.739 | 0.601 |
| SP-296 | WIESILELIKKHLLSLWN | 296 | 0.811 | 0.587 |
| SP-297 | WLAWIDWIRGLDGQLIKI | 297 | 0.867 | 0.568 |
| SP-298 | YEIIKWTSFLLEYLTEII | 298 | 0.879 | 0.508 |
| SP-299 | YGELNIFWLQILEIIRRW | 299 | 0.857 | 0.543 |
| SP-300 | FKLIWGSIFSFLEQIEKF | 300 | 0.814 | 0.604 |

### Experimental Example 2. Preparation of peptide according to an embodiment of present disclosure

The selected peptides SP-001 to SP-300 were synthesized by ANYGEN Co. Ltd. Each peptide was synthesized by a general solid-phase peptide synthesis (SPPS) method.

The Fmoc protecting group was removed by reaction with 20% piperidine in DMF for 10 minutes (twice), and Fmoc amino acids (6 equiv), HOBT (6 equiv), HBTU (6 equiv), and DIPEA (12 equiv) were used for coupling. At each step, the resin was washed with DMF and methanol (repeated twice).

The synthesized crude peptides were reacted with a mixture of TFA/EDT/thioanisole/TIS/DW (90/2.5/2.5/2.5 volume) for 2 hours to remove the resin and amino acid protecting groups. Ether was added to the resulting mixed solution, which was then centrifuged to recover the peptides, which were then lyophilized.

The crude peptides were dissolved in DW and purified by reversed-phase HPLC using a C18 reversed-phase column. In this case, separation was performed using a water-acetonitrile linear gradient (acetonitrile concentration of 10 to 75% (v/v)) method using 0.1% (v/v) trifluoroacetic acid. Thereafter, the purified fraction was lyophilized.

### Experimental Example 3. Confirmation of liposome leakage efficacy of peptide according to an embodiment of present disclosure

The inventors of the present application attempted to confirm the antiviral efficacy (or lipid membrane destruction efficacy) of the peptides prepared in Experimental Example 2 using a liposome solution in which a fluorescent substance was encapsulated (specifically, by taking advantage of the fact that a fluorescence level to be measured is low in a state where the fluorescent substance is encapsulated in liposomes and high in a state where the fluorescent substance leaks out of the liposomes).

As a detailed experimental method to confirm efficacy, a solution including the peptide prepared in Experimental Example 2 was treated with the liposome solution in which a fluorescent substance was encapsulated, and then a change in the fluorescence level was observed. In this case, the data showing that the fluorescence level has increased indicates that the fluorescent substance has leaked out of the liposome, and such leakage indicates that the liposome (specifically, the lipid membrane) is damaged or destroyed.

The detailed experimental methods and results are shown in the following Experimental Examples 3.1 and 3.2.

### Experimental Example 3.1 Preparation of liposomes in which 6-carboxyfluorescein is encapsulated

2.5 µmol of 1-palmitoyl-2-oleoyl-glycero-3-phosphocholine (POPC) was formed as a film by completely removing the organic solvent in a rotary evaporator for 16 hours, and a fluorescent substance 6-carboxyfluorescein (6CF) was prepared by being dissolved in a buffer solution (10 mM Tris, 150 mM NaCl, pH 7.5) to a concentration of 100 mM. After the film-formed POPC was dissolved in 1 mL of a 100 mM 6CF solution to prepare a POPC solution at a concentration of 2.5 mM, freezing and thawing were repeated seven times through liquid nitrogen and a constant-temperature water bath at 60°C.

The POPC solution was made into liposomes through a mini-extruder. The extruder was equipped with a 1 µm filter membrane and the POPC solution was passed through the extruder 15 times, and then the extruder was equipped with a 30 nm filter and the solution was passed through the extruder 15 times, thereby preparing liposomes with a uniform size (55 nm). The remaining fluorescent substance that was not encapsulated in the liposomes was removed using a PD-10 desalting column.

### Experimental Example 3.2 Liposome leakage assay

Each of the SP-001 to SP-300 peptides dissolved in DMSO at a concentration of 200 µM was diluted in a buffer solution to a concentration of 500 nM, and the liposome solution in which the fluorescent substance was encapsulated was diluted in a buffer solution to a POPC concentration of 20 µM. The diluted peptide, 0.06% Triton X-100 as a positive control (PC), and a buffer solution as a negative control (N.C.) were dispensed in triplicate at 50 µL each into three wells of a 96-well black plate. In this case, the positive control, 0.06% Triton X-100, was a solution containing 0.06% of the surfactant Triton X-100, which is known to destroy lipid membranes.

Thereafter, immediately after 50 µL of the diluted liposome solution was dispensed into each well, a fluorescence value (RFU) was measured at intervals of 1 minute for 20 minutes at an excitation wavelength of 492 nm and an emission wavelength of 517 nm using a plate reader.

After the fluorescence was measured for 20 minutes, 50 µL of 0.06% Triton X-100 was added to all the wells, and then the fluorescence was measured again. In this case, 0.06% Triton X-100 was added to all the wells after 20 minutes and fluorescence was measured again in order to confirm that 1) the increase in fluorescence level over time in each well was due to the destruction of liposomes and 2) there was no significant difference in the total amount of fluorescent substance contained in each well.

The measured fluorescence levels were graphed and are shown in FIGS. 3 to 25.

From the results shown in FIGS. 3 to 25, it can be seen that the fluorescent substance encapsulated in the liposome leaked out of the liposome due to SP-001, SP-003 to SP-005, SP-008 to SP-023, SP-026, SP-029, SP-030, SP-033, SP-035, SP-036, SP-038 to SP-040, SP-043, SP-045 to SP-052, SP-055, SP-058, SP-059, SP-062, SP-064 to SP-073, SP-075, SP-078 to SP-087, SP-090 to SP-093, SP-097 to SP-102, SP-104 to SP-106, SP-109 to SP-112, SP-114 to SP-116, SP-118 to SP-123, SP-126, SP-127, SP-130 to SP-132, SP-136, SP-138 to SP-147, SP-151 to SP-155, SP-162, SP-164, SP-166 to SP-168, SP-170 to SP-174, SP-176, SP-180 to SP-182, SP-184 to SP-186, SP-188, SP-189, SP-191, SP-192, SP-194 to SP-196, SP-198, SP-201, SP-204, SP-206, SP-208 to SP-211, SP-215, SP-218, SP-219, SP-221, SP-224, SP-225, SP-228, SP-229, SP-231, SP-232, SP-236, SP-237, SP-240, SP-242 to SP-246, SP-248 to SP-254, SP-256 to SP-258, SP-260 to SP-270, SP-272, SP-273, SP-275 to SP-288, SP-290, SP-291, SP-293 to SP-296, SP-298 and SP-299. These results show that the antiviral peptide of the present disclosure may cause damage (or destruction) to the lipid bilayer of the virus.

### Experimental Example 4. Liposome rupture efficacy of peptides according to an embodiment of present disclosure

### Experimental Example 4.1 Experiments on liposomes with specific size

The present inventors attempted to confirm the antiviral activity (or lipid membrane destruction activity) of the peptides prepared in Experimental Example 2 using liposomes in which the lipid membrane was labeled with a fluorescent substance.

As a detailed experimental method to confirm efficacy, the present inventors observed changes in fluorescence levels while treating a cover slip to which liposomes with the lipid membrane labeled with a fluorescent substance were attached with a solution containing a peptide prepared in Experimental Example 2 at a constant rate.

In this case, the data showing that the fluorescence level decreased over time indicates that the lipid membrane of the liposome was destroyed and the destroyed lipid membrane was removed during the washing process. In other words, through the results of this experiment, it can be confirmed whether the peptides prepared in Experimental Example 2 may cause the liposome to rupture.

The detailed experimental methods and results are shown in the following Experimental Examples 4.1.1 and 4.1.2.

### Experimental Example 4.1.1 Preparation of rhodamine fluorescently labeled liposomes

2.49 µmol of 1-palmitoyl-2-oleoyl-glycero-3-phosphocholine (POPC), 2.51 nmol of 1,2-distearoyl-sn-glycero-3-phosphoethanolamine-N-[biotinyl(polyethylene glycol)-2000] (DSPE-PEG(2000) Biotin), and 17.58 nmol of 1,2-dioleoyl-sn-glycero-3-phosphoethanolamine-N-(lissamine rhodamine B sulfonyl)(18:1 Liss Rhod PE) were mixed for 16 hours and formed into a film by completely removing the organic solvent in a rotary evaporator for 16 hours. In this case, biotin was used to bind to streptavidin on the plate, and rhodamine was used as a fluorescent substance.

After the film-formed phospholipid solution was dissolved in 1 mL of a buffer solution (10 mM Tris, 150 mM NaCl, pH 7.5) to prepare a phospholipid solution at a concentration of 2.5 mM, freezing and thawing were repeated seven times through liquid nitrogen and a constant-temperature water bath at 60°C. The phospholipid solution was allowed to pass 15 times through a small extruder equipped with a 30 nm filter membrane to prepare liposomes with a uniform size (55 nm).

### Experimental Example 4.1.2 TIRF microscope measurement

A biotin-coated coverslip was attached to a channel-type sticky slide (IBIDI, Cat. 80608), treated with 100 µl of 10 µg/mL Neutravidin diluted in a buffer solution, and then allowed to react for 10 minutes. Unreacted Neutravidin was removed, and after the coverslip was washed five times, the prepared liposomes were diluted to a phospholipid concentration of 0.03 µM. After treatment with 100 µl of diluted liposomes and incubation for 10 minutes, the liposomes were attached to the coverslip. Unattached liposomes were then removed, followed by five washes.

Thereafter, each of the SP-001, SP-003 and SP-101 peptides dissolved in DMSO at a concentration of 200 µM was diluted with a buffer solution to prepare a solution diluted to a concentration of 100 nM. Using a peristaltic pump, the diluted peptide was flowed onto the liposome-attached coverslip at a rate of 150 µL/min, and the changes in the fluorescence signal of single liposomes were measured in a TRITC channel at intervals of 20 seconds for 25 minutes under a total internal reflection fluorescence microscope (TIRF microscope). The measurement was repeated three times, and the changes in the fluorescent signals of 200 to 300 liposomes were measured for each measurement, a fluorescent value at 0 second was converted to 1, and the changes were calculated and graphed.

The observed changes in the fluorescent signal are shown in FIG. 26, and graphs showing the changes in the fluorescent signal are shown in FIGS. 27 to 29. FIG. 27 shows the results for the SP-001 peptide. FIG. 28 shows the results for the SP-003 peptide. FIG. 29 shows the results for the SP-101 peptide.

The experimental results in FIGS. 26 to 29 show that the lipid membrane of the liposomes labeled with a fluorescent substance was destroyed by the SP-001, SP-003 and SP-101 peptides, resulting in a decrease in the fluorescence value. These results show that the antiviral peptide of the present disclosure has antiviral efficacy (or lipid membrane destruction efficacy).

### Experimental Example 4.2 Experiments on liposomes with various sizes

The present inventors have conducted the same experiment as in Experimental Example 4.1 of the present specification by varying the size of liposomes to 55 nm, 200 nm, and 350 nm. In this case, the phospholipid bilayer destruction effect of the peptide on liposomes was greatest for 55 nm liposomes and smallest for 350 nm liposomes (referring Experimental Example 5.2 of PCT Application No. PCT/KR2023/013277).

Therefore, the present inventors performed the same experiment as in Example 4.1 in which liposomes with various sizes were used. The experimental method and results are as follows.

### Experimental Example 4.2.1 Preparation of rhodamine fluorescently labeled liposomes

2.49 µmol of 1-palmitoyl-2-oleoyl-glycero-3-phosphocholine (POPC), 2.51 nmol of 1,2-distearoyl-sn-glycero-3-phosphoethanolamine-N-[biotinyl(polyethylene glycol)-2000] (DSPE-PEG(2000) Biotin), and 17.58 nmol of 1,2-dioleoyl-sn-glycero-3-phosphoethanolamine-N-(lissamine rhodamine B sulfonyl)(18:1 Liss Rhod PE) were mixed for 16 hours and formed into a film by completely removing the organic solvent in a rotary evaporator for 16 hours. After the film-formed phospholipid solution was dissolved in 1 mL of a buffer solution (10 mM Tris, 150 mM NaCl, pH 7.5) to prepare a phospholipid solution at a concentration of 2.5 mM, freezing and thawing were repeated seven times through liquid nitrogen and a constant-temperature water bath at 60°C.

The phospholipid solution was passed through a mini-extruder to prepare liposomes with three sizes. The phospholipid solution was passed 15 times through an extruder equipped with a 30 nm filter membrane to prepare liposomes with a uniform size (55 nm), passed 11 times through an extruder equipped with a 200 nm filter membrane to prepare liposomes with a uniform size (150 nm), and passed 15 times through an extruder equipped with a 400 nm filter membrane to prepare liposomes with a uniform size (250 nm).

### Experimental Example 4.2.2 TIRF microscope measurement

A biotin-coated coverslip was attached to a channel-type sticky slide (IBIDI, Cat. 80608), treated with 100 µl of 10 µg/mL Neutravidin diluted in a buffer solution, and then allowed to react for 10 minutes. Unreacted Neutravidin was removed, and after the coverslip was washed five times, the prepared liposomes were diluted to a phospholipid concentration of 0.03 µM. After treatment with 100 µl of diluted liposomes and incubation for 10 minutes, the liposomes were attached to the coverslip. Unattached liposomes were then removed, followed by five washes.

Thereafter, the SP-049 peptide prepared in Experimental Example 2 at a concentration of 200 µM dissolved in DMSO was diluted with a buffer solution to prepare a peptide diluted to a concentration of 100 nM. Using a peristaltic pump, the diluted peptide was flowed onto the liposome-attached coverslip at a rate of 150 µL/min, and the changes in the fluorescence signal of single liposomes were measured in a TRITC channel at intervals of 20 seconds for 25 minutes under a total internal reflection fluorescence microscope (TIRF microscope). The measurement was repeated three times, and the changes in the fluorescent signals of 200 to 300 liposomes were measured for each measurement, a fluorescent value at 0 second was converted to 1, and the changes were calculated and graphed.

A graph showing the observed changes in fluorescent signal is shown in FIG. 53. FIG. 53 shows the results for the SP-049 peptide.

The experimental results in FIG. 53 show that the lipid membrane of the liposomes labeled with a fluorescent substance was destroyed by the SP-049 peptide, resulting in a decrease in the fluorescence value. These results show that the antiviral peptide of the present disclosure has antiviral efficacy (or lipid membrane-destruction efficacy).

The experimental results in FIG. 53 show that the phospholipid bilayer destruction effect of the peptide is greatest for liposome particles with the smallest diameter (largest curvature), and that the phospholipid bilayer disruption effect of the peptide is smallest for liposome particles with the largest diameter (smallest curvature).

Consequently, the results of this experiment show that the phospholipid bilayer destruction effect of the antiviral peptide of the present disclosure is dependent on the curvature of the lipid membrane. The greater the curvature of a target particle having a phospholipid bilayer (the smaller the diameter of the target particle), the greater the phospholipid bilayer destruction effect of the antiviral peptide of the present disclosure. These results are believed to be due to the phenomenon that the greater the curvature of the phospholipid bilayer of the particle, the greater the stress applied to the lipid membrane, and when the antiviral peptide of the present disclosure is inserted into the phospholipid bilayer structure under conditions in which greater stress is applied, the structure of the lipid membrane is easily destroyed.

These results imply that the antiviral peptide of the present disclosure is very effective in damaging (or destroying) the phospholipid bilayer of a virus (referring to the fact that the size or diameter of a virus is generally about 20 to 200 nm), but do not significantly affect the phospholipid bilayers of other cells (referring to the fact that the size or diameter of animal cells is generally about 10 to 30 µm).

### Experimental Example 5. RNase digestion assay of peptide according to an embodiment of present disclosure

The present inventors attempted to confirm the antiviral efficacy (or lipid membrane destruction efficacy) of the peptides prepared in Experiment 2 using a nuclease that is capable of entering the virus through a pore in the lipid membrane of the virus and degrading RNA.

As an experimental method to confirm efficacy, the present inventors treated dengue virus with the peptide, incubated the treated dengue virus with micrococcal nuclease (NEB, Cat. M0247S), and then confirmed the RNA level of the virus. In this case, data showing a low level of viral RNA means that the lipid membrane of the virus is destroyed and a large amount of RNA is degraded by RNase that enters the virus. In other words, the lower the viral RNA level, the better the antiviral efficacy of the peptide.

The detailed experimental method and results are as follows.

Dengue virus stored at a concentration of 1x10⁷ pfu/ml was diluted in a buffer solution (50 mM Tris-HCl, 5 mM CaCl₂) to a concentration of 4x10⁵ pfu/ml. Thereafter, each of the 50 selected peptides dissolved in DMSO at a concentration of 200 µM was diluted with a buffer solution to prepare a peptide solution at a concentration of 2 µM and 1 µM. The diluted peptide solution, a 2% Triton X-100 solution as a positive control, and a buffer solution as a negative control were each dispensed in triplicate at 50 µL each into three microtubes for each concentration, and the diluted virus solution (or control solution) was mixed at a ratio of 1:1 (v/v) and then incubated at 37°C for 1 hour. After the incubation, each microtube was treated with 14 µg of BSA and 960 gel units of micrococcal nuclease (NEB, Cat. M0247S) to a final volume of 140 µL, and incubated at 37°C for 1 hour. In this case, Micrococcal Nuclease (NEB, Cat. M0247S) was used to degrade the RNA of the virus. In other words, when the lipid membrane of the virus is destroyed, the RNA of the virus will be degraded by micrococcal nuclease (NEB, Cat. M0247S) that enters the virus. In contrast, when the lipid membrane of the virus is not destroyed, micrococcal nuclease (NEB, Cat. M0247S) does not enter the virus, so the RNA of the virus will not be degraded.

Viral RNA from the incubated mixed solution was extracted using a QIAamp Viral RNA Kit (Qiagen; Cat. no. 52906), and each Ct value was confirmed by RT-qPCR. RT-qPCR was performed using a One Step PrimeScript^{™} III RT-qPCR Mix(TaqMan) (Takara; Cat no. #RR600A) and using the forward primer 5'-GAAAGACCAGAGATCCTGCTGTCT-3' (SEQ ID NO: 302), the reverse primer 5'-ACCATTCCATTTTCTGGCGTT-3' (SEQ ID NO: 303), the probe 5'-FAM-AGCATCATTCCAGGCAC-3BHQ1-3' (AGCATCATTCCAGGCAC (SEQ ID NO: 304)), and confirmation was performed by repeating 45 cycles of 42°C for 30 min, 95°C for 5 min, 95°C for 30 sec, and then 60°C for 60 sec.

The results were analyzed by calculating an increase in Ct value (ΔCt) upon peptide treatment compared to the Ct value of the negative control. Each value was obtained from two replicate experiments (in duplicate). In this case, the fact that the ΔCt value is high means that the lipid membrane of the virus is destroyed and a large amount of RNA is degraded by RNase that enters the virus. In other words, the higher the ΔCt value, the better the antiviral efficacy of the peptide.

The experimental results are shown in Table 2 and FIG. 30.

The experimental results showed that SP-116, SP-046, SP-049, SP-110, SP-001, SP-143, SP-141, SP-003, SP-151, SP-114, SP-101, SP-059, SP-126, SP-296, SP-004, SP-020, SP-132, SP-264, SP-087, SP-090 SP-290, SP-283, SP-221, SP-048, SP-091, SP-144, SP-171, SP-246, SP-098, SP-256, SP-228, SP-242, SP-078, SP-254, SP-030, SP-139, SP-029, SP-052, SP-115, SP-106, SP-131, SP-162, SP-123, SP-245, SP-167, SP-099, SP-082, SP-189, SP-112 and SP-005 peptides destroyed the lipid membrane of the virus, and the RNA of the virus was degraded by RNase that entered the virus.

In other words, it is shown that the antiviral peptide of the present disclosure has antiviral efficacy (or lipid membrane destruction efficacy).

**[Table 2]**

| | | 1000 nM | | | 500 nM | | |
|---|---|---|---|---|---|---|---|
| No. | Peptide | Ct value | N.C. | Δ Ct | Ct value | N.C. | Δ Ct |
| 1 | SP-116 | 28.76 | 23.89 | 4.87 | 27.98 | 23.89 | 4.10 |
| 2 | SP-046 | 28.79 | 23.93 | 4.87 | 27.00 | 23.93 | 3.07 |
| 3 | SP-049 | 28.79 | 23.93 | 4.86 | 28.04 | 23.93 | 4.11 |
| 4 | SP-110 | 28.82 | 24.05 | 4.77 | 27.10 | 24.05 | 3.05 |
| 5 | SP-001 | 28.44 | 23.68 | 4.76 | 27.72 | 23.68 | 4.04 |
| 6 | SP-143 | 28.30 | 23.68 | 4.62 | 25.43 | 23.68 | 1.75 |
| 7 | SP-141 | 28.25 | 23.68 | 4.57 | 25.11 | 23.68 | 1.43 |
| 8 | SP-003 | 28.46 | 23.93 | 4.54 | 26.81 | 23.93 | 2.88 |
| 9 | SP-151 | 28.33 | 23.89 | 4.45 | 27.08 | 23.89 | 3.20 |
| 10 | SP-114 | 28.13 | 24.05 | 4.08 | 27.08 | 24.05 | 3.03 |
| 11 | SP-101 | 27.66 | 23.93 | 3.73 | 26.61 | 23.93 | 2.68 |
| 12 | SP-059 | 27.13 | 23.93 | 3.21 | 26.18 | 23.93 | 2.25 |
| 13 | SP-126 | 27.07 | 23.89 | 3.19 | 26.47 | 23.89 | 2.59 |
| 14 | SP-296 | 26.83 | 23.68 | 3.15 | 25.06 | 23.68 | 1.38 |
| 15 | SP-004 | 26.65 | 23.54 | 3.11 | 25.22 | 23.54 | 1.68 |
| 16 | SP-020 | 27.04 | 23.93 | 3.11 | 26.10 | 23.93 | 2.17 |
| 17 | SP-132 | 26.59 | 23.54 | 3.06 | 25.62 | 23.54 | 2.08 |
| 18 | SP-264 | 27.29 | 24.26 | 3.03 | 500 | 24.26 | 0.56 |
| 19 | SP-087 | 27.07 | 24.05 | 3.02 | 24.70 | 24.05 | 0.65 |
| 20 | SP-090 | 26.89 | 24.05 | 2.84 | 25.40 | 24.05 | 1.35 |
| 21 | SP-290 | 26.51 | 23.68 | 2.83 | 25.88 | 23.68 | 2.20 |
| 22 | SP-283 | 26.51 | 23.68 | 2.83 | 25.66 | 23.68 | 1.98 |
| 23 | SP-221 | 26.57 | 23.89 | 2.68 | 24.71 | 23.89 | 0.82 |
| 24 | SP-048 | 26.53 | 23.93 | 2.60 | 25.56 | 23.93 | 1.63 |
| 25 | SP-091 | 26.08 | 23.54 | 2.54 | 25.34 | 23.54 | 1.80 |
| 26 | SP-144 | 25.98 | 23.54 | 2.45 | 25.64 | 23.54 | 2.11 |
| 27 | SP-171 | 26.19 | 23.89 | 2.31 | 24.00 | 23.89 | 0.12 |
| 28 | SP-246 | 25.76 | 23.54 | 2.22 | 25.32 | 23.54 | 1.78 |
| 29 | SP-098 | 26.06 | 24.05 | 2.01 | 25.21 | 24.05 | 1.16 |
| 30 | SP-256 | 26.23 | 24.26 | 1.98 | 24.75 | 24.26 | 0.49 |
| 31 | SP-228 | 25.78 | 23.89 | 1.90 | 24.39 | 23.89 | 0.50 |
| 32 | SP-242 | 26.11 | 24.26 | 1.85 | 25.21 | 24.26 | 0.95 |
| 33 | SP-078 | 25.81 | 24.05 | 1.76 | 24.57 | 24.05 | 0.52 |
| 34 | SP-254 | 25.41 | 23.68 | 1.73 | 24.44 | 23.68 | 0.76 |
| 35 | SP-030 | 25.43 | 23.93 | 1.51 | 25.17 | 23.93 | 1.24 |
| 36 | SP-139 | 25.29 | 23.89 | 1.41 | 24.79 | 23.89 | 0.90 |
| 37 | SP-029 | 24.88 | 23.54 | 1.35 | 24.64 | 23.54 | 1.10 |
| 38 | SP-052 | 24.84 | 23.54 | 1.30 | 24.37 | 23.54 | 0.84 |
| 39 | SP-115 | 25.32 | 24.05 | 1.27 | 24.62 | 24.05 | 0.57 |
| 40 | SP-106 | 25.32 | 24.05 | 1.27 | 24.51 | 24.05 | 0.46 |
| 41 | SP-131 | 24.98 | 23.89 | 1.09 | 24.44 | 23.89 | 0.56 |
| 42 | SP-162 | 24.76 | 23.68 | 1.08 | 25.05 | 23.68 | 1.37 |
| 43 | SP-123 | 24.96 | 23.89 | 1.07 | 23.73 | 23.89 | -0.15 |
| 44 | SP-245 | 24.59 | 23.54 | 1.05 | 24.63 | 23.54 | 1.10 |
| 45 | SP-167 | 24.43 | 23.54 | 0.89 | 24.17 | 23.54 | 0.63 |
| 46 | SP-099 | 24.75 | 24.05 | 0.70 | 24.14 | 24.05 | 0.09 |
| 47 | SP-082 | 24.63 | 24.05 | 0.58 | 23.84 | 24.05 | -0.21 |
| 48 | SP-189 | 24.04 | 23.68 | 0.36 | 24.36 | 23.68 | 0.68 |
| 49 | SP-112 | 23.92 | 23.68 | 0.24 | 24.29 | 23.68 | 0.61 |
| 50 | SP-005 | 24.04 | 23.93 | 0.11 | 24.01 | 23.93 | 0.08 |

### Experimental Example 6. Confirmation of antiviral efficacy of peptide according to an embodiment of present disclosure (dengue virus)

The present inventors attempted to confirm the antiviral efficacy (or lipid membrane destruction efficacy) of the peptides prepared in Experimental Example 2. For this purpose, after the virus and peptide were cultured together, cells were treated with the culture solution, and the RNA levels of the virus cultured with the cells were measured. In this case, the lower the measured RNA level, the better the antiviral efficacy of the peptide.

The detailed experimental methods and results are shown in the following Experimental Examples 6.1 and 6.2.

### Experimental Example 6.1 Evaluation at specific concentrations

Vero cells were inoculated into 96-well plates at a density of 1.5x10⁴ cells per well. The inoculated cells were cultured overnight.

Each of the 30 selected peptides dissolved in DMSO at a concentration of 200 µM was serially diluted 2-fold in MEM medium to prepare a peptide solution diluted to a concentration of 120 nM and 60 nM.

When cells were treated with dengue virus, the dengue virus was diluted to an MOI of 0.03 to prepare a diluted virus solution. The diluted peptide solution and the diluted virus solution were mixed at a ratio of 1:1 (v/v) and then incubated at 37°C for 1 hour to prepare a mixed solution of virus and peptide.

Inoculated cells were treated with 200 µL each of the prepared mixed solution of virus and peptide, and were cultured at 37°C and 5% CO₂ for 1 hour. After the mixed solution was removed, 200 µL of MEM medium containing 10% FBS was further added, and the cells were cultured at 37°C and 5% CO₂ for 2 days.

Viral RNA from the incubated mixed solution was extracted using a QIAamp Viral RNA Kit (Qiagen; Cat. no. 52906), and each Ct value was confirmed by RT-qPCR. RT-qPCR was performed using a One Step PrimeScript^{™} III RT-qPCR Mix(TaqMan) (Takara; Cat no. #RR600A) and using the forward primer 5'-GAAAGACCAGAGATCCTGCTGTCT-3' (SEQ ID NO: 302), the reverse primer 5'-ACCATTCCATTTTCTGGCGTT-3' (SEQ ID NO: 303), the probe 5'-FAM-AGCATCATTCCAGGCAC-3BHQ1-3' (AGCATCATTCCAGGCAC (SEQ ID NO: 304)), and confirmation was performed by repeating 45 cycles of 42°C for 30 min, 95°C for 5 min, 95°C for 30 sec, and then 60°C for 60 sec.

The results were analyzed by calculating an increase in Ct value (ΔCt) upon peptide treatment compared to the Ct value of the negative control. Each value was obtained from two replicate experiments (in duplicate).

The experimental results are shown in Table 3 and FIG. 31.

The experimental results showed that SP-049, SP-143, SP-046, SP-116, SP-114, SP-110, SP-283, SP-151, SP-221, SP-256, SP-003, SP-048, SP-004, SP-101, SP-141, SP-290, SP-098, SP-132, SP-246, SP-090, SP-020, SP-059, SP-001, SP-087, SP-091, SP-144, SP-264, SP-296 and SP-126 peptides attacked the virus (such as destroying the lipid membrane of the virus), thereby reducing the amount of virus that infected the cells compared to the negative control.

In other words, it is shown that the antiviral peptide of the present disclosure has antiviral efficacy (or lipid membrane destruction efficacy).

**[Table 3]**

| | | 60 nM | | | 30 nM | | |
|---|---|---|---|---|---|---|---|
| No. | Peptide | Ct value | N.C. | Δ Ct | Ct value | N.C. | Δ Ct |
| 1 | SP-049 | 34.09 | 20.44 | 13.66 | 26.20 | 20.44 | 5.76 |
| 2 | SP-143 | 26.14 | 20.44 | 5.70 | 22.23 | 20.44 | 1.79 |
| 3 | SP-046 | 24.64 | 20.44 | 4.21 | 22.16 | 20.44 | 1.72 |
| 4 | SP-116 | 24.18 | 20.44 | 3.74 | 22.08 | 20.44 | 1.64 |
| 5 | SP-114 | 23.30 | 20.23 | 2.86 | 21.72 | 20.23 | 1.28 |
| 6 | SP-110 | 23.11 | 20.44 | 2.67 | 22.24 | 20.44 | 1.80 |
| 7 | SP-283 | 23.02 | 20.23 | 2.79 | 22.07 | 20.23 | 1.84 |
| 8 | SP-151 | 22.69 | 20.44 | 2.26 | 21.25 | 20.44 | 0.81 |
| 9 | SP-221 | 22.61 | 20.23 | 2.38 | 21.69 | 20.23 | 1.46 |
| 10 | SP-256 | 22.53 | 20.23 | 2.30 | 21.54 | 20.23 | 1.31 |
| 11 | SP-003 | 22.52 | 20.44 | 2.09 | 21.78 | 20.44 | 1.34 |
| 12 | SP-048 | 22.32 | 20.23 | 2.09 | 21.55 | 20.23 | 1.32 |
| 13 | SP-004 | 22.28 | 20.23 | 2.05 | 21.41 | 20.23 | 1.18 |
| 14 | SP-101 | 22.24 | 20.44 | 1.81 | 21.22 | 20.44 | 0.78 |
| 15 | SP-141 | 22.24 | 20.44 | 1.80 | 21.41 | 20.44 | 1.82 |
| 16 | SP-290 | 22.24 | 20.23 | 2.01 | 22.05 | 20.23 | 0.97 |
| 17 | SP-098 | 22.10 | 20.23 | 1.87 | 21.16 | 20.23 | 0.93 |
| 18 | SP-132 | 21.96 | 20.23 | 1.73 | 20.91 | 20.23 | 0.68 |
| 19 | SP-246 | 21.87 | 20.23 | 1.64 | 21.03 | 20.23 | 0.80 |
| 20 | SP-090 | 21.85 | 20.23 | 1.62 | 21.27 | 20.23 | 1.04 |
| 21 | SP-020 | 21.80 | 20.44 | 1.36 | 22.14 | 20.44 | 1.70 |
| 22 | SP-059 | 21.73 | 20.44 | 1.30 | 21.03 | 20.44 | 0.59 |
| 23 | SP-001 | 21.66 | 20.44 | 1.23 | 21.78 | 20.44 | 1.34 |
| 24 | SP-087 | 21.56 | 20.23 | 1.33 | 20.74 | 20.23 | 0.50 |
| 25 | SP-091 | 21.38 | 20.23 | 1.15 | 20.77 | 20.23 | 0.54 |
| 26 | SP-144 | 21.33 | 20.23 | 1.10 | 19.80 | 20.23 | -0.43 |
| 27 | SP-264 | 21.15 | 20.44 | 0.71 | 21.06 | 20.44 | 0.62 |
| 28 | SP-296 | 21.07 | 20.44 | 0.63 | 20.20 | 20.44 | -0.25 |
| 29 | SP-126 | 20.81 | 20.23 | 0.57 | 20.79 | 20.23 | 0.56 |
| 30 | SP-171 | 20.07 | 20.23 | -0.16 | 20.08 | 20.23 | -0.15 |

### Experimental Example 6.2 Derivation of IC50 value

Vero cells were inoculated into 96-well plates at a density of 1.5x10⁴ cells per well. The inoculated cells were cultured overnight.

Each of the 9 selected peptides dissolved in DMSO at a concentration of 4 mM was serially diluted 2-fold in MEM medium to prepare solutions diluted to 8 concentrations.

The SP-049 peptide was prepared such that the maximum concentration was 125 nM and the minimum concentration was 0.98 nM (concentrations: 125 nM; 62.5 nM; 31.25 nM; 15.62 nM; 7.81 nM; 3.91 nM; 1.95 nM; and 0.98 nM).

The SP-046 and SP-143 peptides were prepared such that the maximum concentration was 250 nM and the minimum concentration was 1.95 nM (concentrations: 250 nM; 125 nM; 62.5 nM; 31.25 nM; 15.62 nM; 7.81 nM; 3.91 nM; and 1.95 nM).

The SP-001, SP-003, SP-004 and SP-101 peptides were prepared such that the maximum concentration was 2000 nM and the minimum concentration was 15.63 nM (concentrations: 2000 nM; 1000 nM; 500 nM; 250 nM; 125 nM; 62.5 nM; 31.25 nM; and 15.63 nM).

The SP-185 and SP-191 peptides were prepared such that the maximum concentration was 10000 nM and the minimum concentration was 78.13 nM (concentrations: 10000 nM; 5000 nM; 2500 nM; 1250 nM; 625 nM; 312.5 nM; 156.25 nM; and 78.13 nM).

When cells were treated with dengue virus, the dengue virus was diluted to an MOI of 0.03 to prepare a diluted virus solution. The diluted peptide solution and the diluted virus solution were mixed at a ratio of 1:1 (v/v) and then incubated at 37°C for 1 hour to prepare a mixed solution of virus and peptide. Inoculated cells were treated with 200 µL each of the prepared mixed solution of virus and peptide, and were cultured at 37°C and 5% CO₂ for 1 hour. After the mixed solution was removed, 200 µL of MEM medium containing 10% FBS was added, and the cells were cultured at 37°C and 5% CO₂ for 2 days.

Viral RNA from the incubated mixed solution was extracted using a QIAamp Viral RNA Kit (Qiagen; Cat. no. 52906), and each Ct value was confirmed by RT-qPCR. RT-qPCR was performed using a One Step PrimeScript^{™} III RT-qPCR Mix(TaqMan) (Takara; Cat no. #RR600A) and using the forward primer 5'-GAAAGACCAGAGATCCTGCTGTCT-3' (SEQ ID NO: 302), the reverse primer 5'-ACCATTCCATTTTCTGGCGTT-3' (SEQ ID NO: 303), the probe 5'-FAM-AGCATCATTCCAGGCAC-3BHQ1-3' (AGCATCATTCCAGGCAC (SEQ ID NO: 304)), and confirmation was performed by repeating 45 cycles of 42°C for 30 min, 95°C for 5 min, 95°C for 30 sec, and then 60°C for 60 sec.

The present inventors calculated the inhibition rate (% inhibition) of each well based on the formula of the standard curve by converting the average Ct value of wells infected with dengue virus without peptide treatment in the same plate into an infection rate of 100%, and using this to create a standard curve. Each value was obtained through three repeated experiments (in triplicate), and IC50 values were derived using GraphPad Prism 5 software. It is possible to determine that the lower the IC50 value, the better the antiviral effect.
The IC50 (nM) value of the SP-049 peptide is 3.85.
The IC50 (nM) value of the SP-001 peptide is 22.60.
The IC50 (nM) value of the SP-046 peptide is 17.68.
The IC50 (nM) value of the SP-143 peptide is 16.09.
The IC50 (nM) value of the SP-003 peptide is 21.99.
The IC50 (nM) value of the SP-004 peptide is 206.6.
The IC50 (nM) value of the SP-101 peptide is 100.1.
The IC50 (nM) value of the SP-185 peptide is 518.9.
The IC50 (nM) value of the SP-191 peptide is 776.2.
The experimental results are shown in Tables 4 to 12 and FIGS. 32 to 40.

The experimental results showed that SP-049, SP-046, SP-143, SP-001, SP-003, SP-004, SP-101, SP-185 and SP-191 peptides attacked the virus (such as destroying the lipid membrane of the virus), thereby reducing the amount of virus that infected the cells compared to the negative control.

In other words, it is shown that the antiviral peptide of the present disclosure has antiviral efficacy (or lipid membrane destruction efficacy).

**[Table 4]**

| | SP-049 | |
|---|---|---|
| Conc. (nM) | Ct Value | % Inhibition |
| 62.50 | 39.68 | 100.00 |
| 31.25 | 41.59 | 100.00 |
| 15.63 | 37.13 | 100.00 |
| 7.81 | 25.38 | 93.03 |
| 3.91 | 23.00 | 45.30 |
| 1.95 | 22.87 | 39.14 |
| 0.98 | 22.56 | 20.16 |
| 0.49 | 22.62 | 24.20 |
| 0 (N.C) | 22.30 | 0.00 |

**[Table 5]**

| | SP-046 | |
|---|---|---|
| Conc. (nM) | Ct Value | % Inhibition |
| 125.00 | 45.00 | 100.00 |
| 62.50 | 42.10 | 100.00 |
| 31.25 | 36.59 | 100.00 |
| 15.62 | 22.75 | 36.10 |
| 7.81 | 22.45 | 15.33 |
| 3.91 | 22.34 | 5.60 |
| 1.95 | 22.32 | 3.30 |
| 0.98 | 22.32 | 3.30 |
| 0 (N.C) | 22.28 | 0.00 |

**[Table 6]**

| | SP-143 | |
|---|---|---|
| Conc. (nM) | Ct Value | % Inhibition |
| 125.00 | 26.73 | 99.42 |
| 62.50 | 23.95 | 94.07 |
| 31.25 | 22.45 | 79.28 |
| 15.63 | 21.47 | 53.07 |
| 7.81 | 21.38 | 49.26 |
| 3.91 | 21.24 | 42.98 |
| 1.95 | 20.26 | -28.80 |
| 0.98 | 21.66 | 59.95 |
| 0 (N.C) | 20.56 | 0.00 |

**[Table 7]**

| | SP-001 | |
|---|---|---|
| Conc. (nM) | Ct Value | % Inhibition |
| 1000.00 | 45.00 | 100.00 |
| 500.00 | 44.60 | 100.00 |
| 250.00 | 38.67 | 100.00 |
| 125.00 | 26.72 | 82.51 |
| 62.50 | 26.44 | 78.78 |
| 31.25 | 25.47 | 57.96 |
| 15.63 | 25.10 | 45.60 |
| 7.81 | 24.23 | -0.47 |
| 0 (N.C) | 24.23 | 0.00 |

**[Table 8]**

| | SP-003 | |
|---|---|---|
| Conc. (nM) | Ct Value | % Inhibition |
| 1000.00 | 45.00 | 100.00 |
| 500.00 | 45.00 | 100.00 |
| 250.00 | 43.56 | 100.00 |
| 125.00 | 34.14 | 99.91 |
| 62.50 | 25.69 | 63.98 |
| 31.25 | 26.01 | 71.23 |
| 15.63 | 24.93 | 38.56 |
| 7.81 | 24.15 | -6.03 |
| 0 (N.C) | 24.23 | 0.00 |

**[Table 9]**

| | SP-004 | |
|---|---|---|
| Conc. (nM) | Ct Value | % Inhibition |
| 1000.00 | 39.11 | 100.00 |
| 500.00 | 32.29 | 99.95 |
| 250.00 | 23.22 | 61.21 |
| 125.00 | 22.51 | 34.78 |
| 62.50 | 22.27 | 22.45 |
| 31.25 | 22.05 | 9.23 |
| 15.63 | 22.01 | 6.56 |
| 7.81 | 21.89 | -1.96 |
| 0 (N.C) | 21.92 | 0.00 |

**[Table 10]**

| | SP-101 | |
|---|---|---|
| Conc. (nM) | Ct Value | % Inhibition |
| 1000.00 | 39.50 | 100.00 |
| 500.00 | 38.40 | 100.00 |
| 250.00 | 26.78 | 97.07 |
| 125.00 | 23.37 | 65.09 |
| 62.50 | 22.28 | 23.20 |
| 31.25 | 21.93 | 0.96 |
| 15.63 | 22.24 | 20.94 |
| 7.81 | 21.74 | -14.11 |
| 0 (N.C) | 21.92 | 0.00 |

**[Table 11]**

| | SP-185 | |
|---|---|---|
| Conc. (nM) | Ct Value | % Inhibition |
| 5000.00 | 45.00 | 100.00 |
| 2500.00 | 40.11 | 100.00 |
| 1250.00 | 23.76 | 89.53 |
| 625.00 | 21.67 | 51.77 |
| 312.50 | 21.22 | 33.04 |
| 156.25 | 21.29 | 36.60 |
| 78.13 | 20.84 | 11.76 |
| 39.06 | 20.57 | -7.43 |
| 0 (N.C) | 20.67 | 0.00 |

**[Table 12]**

| | SP-191 | |
|---|---|---|
| Conc. (nM) | Ct Value | % Inhibition |
| 5000.00 | 34.18 | 99.99 |
| 2500.00 | 26.43 | 98.50 |
| 1250.00 | 22.07 | 64.10 |
| 625.00 | 21.15 | 29.53 |
| 312.50 | 21.53 | 46.78 |
| 156.25 | 20.74 | 5.32 |
| 78.13 | 20.48 | -14.44 |
| 39.06 | 20.41 | -20.14 |
| 0 (N.C) | 20.67 | 0.00 |

### Experimental Example 7. Confirmation of antiviral efficacy of peptide according to an embodiment of present disclosure (SARS-CoV2 virus)

The present inventors attempted to confirm the antiviral efficacy (or lipid membrane destruction efficacy) of the peptides prepared in Experimental Example 2. For this purpose, after the virus and peptide were cultured together, cells were treated with the culture solution and cultured, and then the proportion of infected cells was measured. In this case, the higher the measured proportion of normal cells that are not infected with the virus, the better the antiviral efficacy of the peptide.

The detailed experimental method and results are as follows.

Vero cells were inoculated into 96-well plates at a density of 1.5x10⁴ cells per well. The inoculated cells were cultured overnight.

The 12 selected peptides dissolved in DMSO at a concentration of 4 mM were diluted 1/100 with DPBS, and then serially diluted 2-fold to prepare a peptide solution such that the maximum concentration was 40 µM and the minimum concentration was 0.08 µM (concentrations: 40 µM; 20 µM; 10 µM; 5 µM; 2.5 µM; 1.25 µM; 0.63 µM; 0.31 µM; 0.16 µM; and 0.08 µM). The diluted peptide solution was mixed with 200 TCID50 of SARS-CoV-2 virus at a ratio of 1:1 (v/v) and then incubated at 37°C for 30 minutes to prepare a mixed solution. Four wells inoculated with 40 µL of cells at each concentration were treated with the mixed solution, and cultured at 37°C and 5% CO₂ for 4 days. After the culture, the cells were fixed with 4% paraformaldehyde and stained with a crystal violet solution, and neutralizing activity (%) was calculated by observing a cytopathic effect (CPE). The neutralizing activity was calculated by normalization with a positive control (mock) and a negative control (0.5% DMSO), and a 50% inhibitory concentration (IC50) was calculated.

The IC50 (µM) value of the SP-049 peptide is 1.8.

The IC50 (µM) value of the SP-116 peptide is 4.1.

The IC50 (µM) value of the SP-151 peptide is 5.0.

The IC50 (µM) value of the SP-046 peptide is 7.9.

The IC50 (µM) value of the SP-004 peptide is 8.9.

The IC50 (µM) value of the SP-003 peptide is 10.3.

The IC50 (µM) value of the SP-101 peptide is 11.6.

The IC50 (µM) value of the SP-171 peptide is 18.7.

The IC50 (µM) value of the SP-110 peptide is 19.5.

The IC50 (µM) value of the SP-114 peptide is 19.5.

The IC50 (µM) value of the SP-143 peptide is 19.5.

The IC50 (µM) value of the SP-221 peptide is 19.5.

The experimental results are shown in FIGS. 41 to 52

The experimental results showed that SP-049, SP-116, SP-151, SP-046, SP-004, SP-003, SP-101, SP-171, SP-110, SP-114, SP-143 and SP-221 peptides attacked the virus (such as destroying the lipid membrane of the virus), thereby reducing the level of cells infected by the virus.

In other words, it is shown that the antiviral peptide of the present disclosure has antiviral efficacy (or lipid membrane destruction efficacy).

## Claims

1. An antiviral peptide having an amino acid sequence selected from the group consisting of the following sequences:
SEQ ID NO: 1, SEQ ID NOs: 3 to 5, SEQ ID NOs: 8 to 23, SEQ ID NO: 26, SEQ ID NO: 29, SEQ ID NO: 30, SEQ ID NO: 33, SEQ ID NO: 35, SEQ ID NO: 36, SEQ ID NOs: 38 to 40, SEQ ID NO: 43, SEQ ID NOs: 45 to 52, SEQ ID NO: 55, SEQ ID NO: 58, SEQ ID NO: 59, SEQ ID NO: 62, SEQ ID NOs: 64 to 73, SEQ ID NO: 75, SEQ ID NOs: 78 to 87, SEQ ID NOs: 90 to 93, SEQ ID NOs: 97 to 102, SEQ ID NOs: 104 to 106, SEQ ID NOs: 109 to 112, SEQ ID NOs: 114 to 116, SEQ ID NOs: 118 to 123, SEQ ID NO: 126, SEQ ID NO: 127, SEQ ID NOs: 130 to 132, SEQ ID NO: 136, SEQ ID NOs: 138 to 147, SEQ ID NOs: 151 to 155, SEQ ID NO: 162, SEQ ID NO: 164, SEQ ID NOs: 166 to 168, SEQ ID NOs: 170 to 174, SEQ ID NO: 176, SEQ ID NOs: 180 to 182, SEQ ID NOs: 184 to 186, SEQ ID NO: 188, SEQ ID NO: 189, SEQ ID NO: 191, SEQ ID NO: 192, SEQ ID NOs: 194 to 196, SEQ ID NO: 198, SEQ ID NO: 201, SEQ ID NO: 204, SEQ ID NO: 206, SEQ ID NOs: 208 to 211, SEQ ID NO: 215, SEQ ID NO: 218, SEQ ID NO: 219, SEQ ID NO: 221, SEQ ID NO: 224, SEQ ID NO: 225, SEQ ID NO: 228, SEQ ID NO: 229, SEQ ID NO: 231, SEQ ID NO: 232, SEQ ID NO: 236, SEQ ID NO: 237, SEQ ID NO: 240, SEQ ID NOs: 242 to 246, SEQ ID NOs: 248 to 254, SEQ ID NOs: 256 to 258, SEQ ID NOs: 260 to 270, SEQ ID NO: 272, SEQ ID NO: 273, SEQ ID NOs: 275 to 288, SEQ ID NO: 290, SEQ ID NO: 291, SEQ ID NOs: 293 to 296, SEQ ID NO: 298 and SEQ ID NO: 299.

2. The antiviral peptide of claim 1,
wherein the amino acid sequence of the antiviral peptide is selected from following sequences:
SEQ ID NO: 1, SEQ ID NO: 3, SEQ ID NO: 4, SEQ ID NO: 5, SEQ ID NO: 20, SEQ ID NO: 29, SEQ ID NO: 30, SEQ ID NO: 46, SEQ ID NO: 48, SEQ ID NO: 49, SEQ ID NO: 52, SEQ ID NO: 59, SEQ ID NO: 78, SEQ ID NO: 82, SEQ ID NO: 87, SEQ ID NO: 90 SEQ ID NO: 91, SEQ ID NO: 98, SEQ ID NO: 99, SEQ ID NO: 101, SEQ ID NO: 106, SEQ ID NO: 110, SEQ ID NO: 112, SEQ ID NO: 114, SEQ ID NO: 115, SEQ ID NO: 116, SEQ ID NO: 123, SEQ ID NO: 126, SEQ ID NO: 131, SEQ ID NO: 132, SEQ ID NO: 139, SEQ ID NO: 141, SEQ ID NO: 143, SEQ ID NO: 144, SEQ ID NO: 151, SEQ ID NO: 162, SEQ ID NO: 167, SEQ ID NO: 171, SEQ ID NO: 189, SEQ ID NO: 221, SEQ ID NO: 228, SEQ ID NO: 242, SEQ ID NO: 245, SEQ ID NO: 246, SEQ ID NO: 254, SEQ ID NO: 256, SEQ ID NO: 264, SEQ ID NO: 283, SEQ ID NO: 290 and SEQ ID NO: 296.

3. The antiviral peptide of claim 1,
wherein the amino acid sequence of the antiviral peptide is selected from following sequences:
SEQ ID NO: 1, SEQ ID NO: 3, SEQ ID NO: 4, SEQ ID NO: 20, SEQ ID NO: 46, SEQ ID NO: 48, SEQ ID NO: 49, SEQ ID NO: 59, SEQ ID NO: 87, SEQ ID NO: 90, SEQ ID NO: 91, SEQ ID NO: 98, SEQ ID NO: 101, SEQ ID NO: 110, SEQ ID NO: 114, SEQ ID NO: 116, SEQ ID NO: 126, SEQ ID NO: 132, SEQ ID NO: 141, SEQ ID NO: 143, SEQ ID NO: 144, SEQ ID NO: 151, SEQ ID NO: 221, SEQ ID NO: 246, SEQ ID NO: 256, SEQ ID NO: 264, SEQ ID NO: 283, SEQ ID NO: 290 and SEQ ID NO: 296.

4. The antiviral peptide of claim 1,
wherein the amino acid sequence of the antiviral peptide is selected from following sequences:
SEQ ID NO: 1, SEQ ID NO: 3, SEQ ID NO: 4, SEQ ID NO: 46, SEQ ID NO: 49, SEQ ID NO: 101, SEQ ID NO: 143, SEQ ID NO: 185 and SEQ ID NO: 191.

5. The antiviral peptide of claim 1,
wherein the amino acid sequence of the antiviral peptide is selected from following sequences:
SEQ ID NO: 3, SEQ ID NO: 4, SEQ ID NO: 46, SEQ ID NO: 49, SEQ ID NO: 101, SEQ ID NO: 110, SEQ ID NO: 114, SEQ ID NO: 116, SEQ ID NO: 143, SEQ ID NO: 151, SEQ ID NO: 171 and SEQ ID NO: 221.

6. The antiviral peptide of claim 1,
wherein the antiviral peptide is an amphipathic peptide.

7. The antiviral peptide of claim 1,
wherein the antiviral peptide is capable of causing a damage and/or destruction of a lipid bilayer of a virus.

8. The antiviral peptide of claim 7,
wherein the virus belongs to a family of Bunyaviridae, Coronaviridae, Filoviridae, Flaviviridae, Hepadnaviridae, Herpesviridae, Orthomyxoviridae, Poxviridae, Rhabdoviridae, Retroviridae, Togaviridae, or Herpesviridae.

9. The antiviral peptide of claim 1,
wherein the antiviral peptide is capable of forming an α-helix structure.

10. A pharmaceutical composition for treating a viral disease, comprising a therapeutically effective amount of an antiviral peptide,
wherein the antiviral peptide is the antiviral peptide according to any one of claims 1 to 9, and
wherein the viral disease is caused by an infection of the virus.

11. The pharmaceutical composition of claim 10,
wherein the pharmaceutical composition further comprises a pharmaceutically acceptable carrier and/or pharmaceutically acceptable adjuvant.

12. The pharmaceutical composition of claim 10,
wherein the viral disease is a respiratory disease caused by a viral infection or a coronavirus disease 2019 (COVID-19).

13. A method for treating a viral disease, comprising:
administering a pharmaceutical composition comprising therapeutically ef fective amount of an antiviral peptide to a subject infected with a virus,
wherein, the antiviral peptide is the antiviral peptide according to any one of claims 1 to 9.

14. Use of an antiviral peptide according to any one of claims 1 to 9 for treating a viral disease.

15. Use of an antiviral peptide according to any one of claims 1 to 9 in the manufacture of a medicament for treating a viral disease.

16. A method for screening a candidate group for antiviral peptides, comprising selecting peptides that satisfy the following conditions:
1) the peptide consists of 18 amino acid residues;
2) the peptide does not contain proline residues;
3) the peptide comprises at least six polar amino acid residues;
4) the peptide contains from four to six charged amino acid residues;
5) the peptide has a hydrophobic moment (µH) value of 0.5 to 0.8, as measured by HeliQuest software;
6) the peptide has a hydrophobicity (H) value of 0.7 to 0.9, as measured by HeliQuest software; and
7) the peptide has a net charge ranging from -2 to 0, as measured by HeliQuest software.

17. The method of claim 16,
wherein the polar amino acid residues are aspartic acid (Asp, D), glutamic acid (Glu, E), lysine (Lys, K), arginine (Arg, R), histidine (His, H), serine (Ser, S), threonine (Thr, T), asparagine (Asn, N), glutamine (Gln, Q) and glycine (Gly, G),
wherein the charged amino acid residues are aspartic acid (Asp, D), glutamic acid (Glu, E), lysine (Lys, K) and arginine (Arg, R).

18. The method of claim 16,
wherein the method is **characterized by** selecting a candidate group of antiviral peptides from amino acid sequences disclosed in the Swiss-Prot sequence data.

19. A method for selecting antiviral peptides, comprising:
verifying an antiviral efficacy of peptides selected through a method for screening a candidate group for antiviral peptides according to claim 16.

20. The method of claim 19,
wherein verifying the antiviral efficacy of selected peptides comprises at least one selected from the following:
mixing liposomes with the selected peptide and confirming damage or destruction of the liposomes; or
mixing virus with the selected peptide and confirming damage or destruction of the viral phospholipid bilayer , or confirming a reduction or inactivation of the virus's infectivity toward cells.

21. The method of claim 20,
wherein the size of the liposome ranges from 5nm to 1500nm.

22. The method of claim 21,
wherein the size of the liposome ranges from 20nm to 400nm.
